(19) Europäisches Patentamt
European Patent Office
Office européen des brevets

(11) EP 0 697 412 A1

(12) **EUROPÄISCHE PATENTANMELDUNG**

(43) Veröffentlichungstag: **21.02.1996 Patentblatt 1996/08**

(21) Anmeldenummer: **95111477.6**

(22) Anmeldetag: **21.07.1995**

(51) Int. Cl.[6]: **C07D 417/04**, C07D 263/56, C07D 417/14, C07D 263/58, C07D 491/10, A61K 31/42, A61K 31/425, A61K 31/44, A61K 31/445, A61K 31/495

(84) Benannte Vertragsstaaten:
**AT BE CH DE DK ES FR GB GR IE IT LI LU MC NL PT SE**

(30) Priorität: **03.08.1994 DE 4427475**
**18.04.1995 DE 19514313**

(71) Anmelder: **BAYER AG**
**D-51368 Leverkusen (DE)**

(72) Erfinder:
- **Häbich, Dieter, Dr.** **D-42115 Wuppertal (DE)**
- **Riedl, Bernd, Dr.** **D-42115 Wuppertal (DE)**
- **Ruppelt, Martin, Dr.** **D-42115 Wuppertal (DE)**
- **Stolle, Andreas, Dr.** **D-42115 Wuppertal (DE)**
- **Wild, Hanno, Dr.** **Orange, Connecticut 06477 (US)**
- **Endermann, Rainer, Dr.** **D-42113 Wuppertal (DE)**
- **Bremm, Klaus Dieter, Dr.** **D-45661 Recklinghausen (DE)**
- **Kroll, Hein-Peter, Dr.** **D-42115 Wuppertal (DE)**
- **Labischinski, Harald, Prof. Dr.** **D-42109 Wuppertal (DE)**
- **Schaller, Klaus, Dr.** **D-42109 Wuppertal (DE)**
- **Werling, Klaus, Dr.** **D-42113 Wuppertal (DE)**

(54) **Benzoxazolyl- und Benzothiazolyloxazolidinone**

(57) Die Erfindung betrifft Benzoxazolyl und Benzothiazolyloxazolidinone der allgemeinen Formel (I),

der allgemeinen Formel (I),

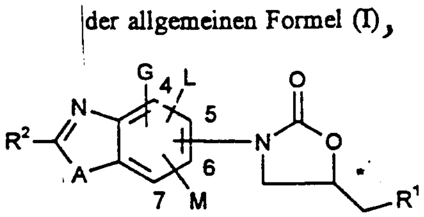

(I)

in welcher

A für ein Sauerstoffatom oder für einen Rest der Formel $-S(O)_a$ steht, worin

a eine Zahl 0 oder 2 bedeutet,

$R^1$ für Azido oder für eine Gruppe der Formel $O-SO_2R^3$ oder $-NR^4R^5$ steht,

Verfahren zu ihrer Herstellung und ihre Verwendung als Arzneimittel, insbesondere als antibakterielle Arzneimittel.

EP 0 697 412 A1

Printed by Rank Xerox (UK) Business Services
2.9.9/3.4

## Beschreibung

Die vorliegende Erfindung betrifft Benzoxazolyl und Benzothiazolyloxazolidinone, Verfahren zu ihrer Herstellung und ihre Verwendung als Arzneimittel, insbesondere als antibakterielle Arzneimittel.

Aus den Publikationen US 5 254 577, US 4 705 799, EP 311 090, US 4 801 600, US 4 921 869, US 4 965 268, EP 312 000 und C.H. Park et al., J. Med. Chem. 35, 1156(1992) sind N-Aryloxazolidinone mit antibakterieller Wirkung bekannt.

Ferner werden in der PCT 93 08 179 A Oxazolidinonderivate mit einer Monoaminoxidase inhibitorischer Wirkung beschrieben.

Die vorliegende Erfindung betrifft Benzoxazolyl- und Benzothiazolyloxazolidinone der allgemeinen Formel (I)

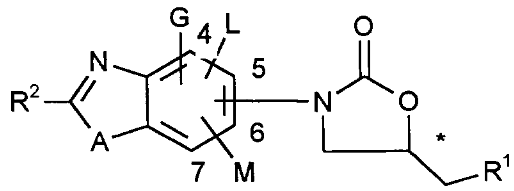

(I)

in welcher

A für ein Sauerstoffatom oder für einen Rest der Formel - $S(O)_a$ steht,
worin

a eine Zahl 0 oder 2 bedeutet,

$R^1$ für Azido oder für eine Gruppe der Formel $O-SO_2R^3$ oder - $NR^4R^5$ steht,
worin

$R^3$ geradkettiges oder verzweigtes Alkyl mit bis zu 4 Kohlenstoffatomen oder Phenyl bedeutet, das gegebenenfalls durch geradkettiges oder verzweigtes Alkyl mit bis zu 4 Kohlenstoffatomen substituiert ist,

$R^4$ und $R^5$ gleich oder verschieden sind und
Cycloalkyl mit 3 bis 6 Kohlenstoffatomen, Wasserstoff, Phenyl oder geradkettiges oder verzweigtes Alkyl mit bis zu 8 Kohlenstoffatomen oder eine Aminoschutzgruppe bedeuten,
oder

$R^4$ oder $R^5$ eine Gruppe der Formel $-CO-R^6$ bedeutet,
worin

$R^6$ Cycloalkyl mit 3 bis 6 Kohlenstoffatomen, geradkettiges oder verzweigtes Alkyl oder Alkoxy mit jeweils bis zu 8 Kohlenstoffatomen, Phenyl oder Wasserstoff bedeutet,

G, L und M gleich oder verschieden sind und
für Wasserstoff, Carboxy, Halogen, Cyano, Formyl, Trifluormethyl, Nitro, geradkettiges oder verzweigtes Alkoxy, Alkoxycarbonyl, Alkylthio oder Acyl mit jeweils bis zu 6 Kohlenstoffatomen oder für geradkettiges oder verzweigtes Alkyl mit bis zu 6 Kohlenstoffatomen stehen, das seinerseits durch Hydroxy, durch geradkettiges oder verzweigtes Alkoxy oder Acyl mit bis zu 5 Kohlenstoffatomen oder durch eine Gruppe der Formel - $NR^7R^8$ substituiert sein kann,
worin

$R^7$ und $R^8$ gleich oder verschieden sind und
Wasserstoff, geradkettiges oder verzweigtes Alkyl mit bis zu 4 Kohlenstoffatomen oder Phenyl bedeuten,
oder gemeinsam mit dem Stickstoffatom einen 5- bis 6-gliedrigen, gesättigten Heterocyclus mit gegebenenfalls einem weiteren Heteroatom aus der Serie N, S und/oder O bilden, der seinerseits gegebenenfalls, auch an einem weiteren Stickstoffatom, durch geradkettiges oder verzweigtes Alkyl oder Acyl mit bis zu 3 Kohlenstoffatomen substituiert sein kann,

und/oder
gegebenenfalls für eine Gruppe der Formel $-NR^{7'}R^{8'}$ stehen,
worin

$R^{7'}$ und $R^{8'}$ gleich oder verschieden sind und die oben angegebene Bedeutung von $R^7$ und $R^8$ haben und mit dieser gleich oder verschieden sind,

und/oder
gegebenenfalls für ($C_2$-$C_8$)-Alkenylphenyl, Phenyl oder durch einen 5- oder 6-gliedrigen gesättigten oder ungesättigten Heterocyclus mit bis zu 3 Heteroatomen aus der Reihe S, N und/oder O stehen, die ihrerseits gegebenenfalls durch eine Gruppe der Formel -CO-$NR^9R^{10}$, $NR^{11}R^{12}$, $NR^{13}$-$SO_2$-$R^{14}$, $R^{15}R^{16}N$-$SO_2$- oder $R^{17}$-$S(O)_b$- substituiert sind, worin

b eine Zahl 0, 1 oder 2 bedeutet,
$R^9$, $R^{10}$, $R^{13}$, $R^{15}$ und $R^{16}$ gleich oder verschieden sind und Wasserstoff, geradkettiges oder verzweigtes Alkyl mit bis zu 6 Kohlenstoffatomen oder Phenyl bedeuten,
$R^{11}$ und $R^{12}$ gleich oder verschieden sind und die oben angegebene Bedeutung von $R^7$ und $R^8$ haben und mit dieser gleich oder verschieden sind,
$R^{14}$ und $R^{17}$ gleich oder verschieden sind und die oben angegebene Bedeutung von $R^3$ haben und mit dieser gleich oder verschieden sind,

und/oder ihrerseits gegebenenfalls bis zu 2-fach gleich oder verschieden durch Carboxy, Halogen, Cyano, Formyl, Trifluormethyl, Nitro, Phenyl, geradkettiges oder verzweigtes Alkoxy, Alkoxycarbonyl, Alkylthio oder Acyl mit jeweils bis zu 6 Kohlenstoffatomen oder durch geradkettiges oder verzweigtes Alkyl mit bis zu 6 Kohlenstoffatomen substituiert sind, das seinerseits durch Hydroxy, durch geradkettiges oder verzweigtes Alkoxy oder Acyl mit bis zu 5 Kohlenstoffatomen oder durch eine Gruppe der Formel -$NR^{18}R^{19}$ substituiert sein kann, worin

$R^{18}$ und $R^{19}$ die oben angegebene Bedeutung von $R^7$ und $R^8$ haben und mit dieser gleich oder verschieden sind,

$R^2$ für Wasserstoff, Formyl, Carboxy,
für geradkettiges oder verzweigtes Alkoxycarbonyl mit bis zu 6 Kohlenstoffatomen steht, oder
für geradkettiges oder verzweigtes Alkyl oder Alkenyl mit jeweils bis zu 8 Kohlenstoffatomen steht, die gegebenenfalls durch Hydroxy, Halogen oder durch geradkettiges oder verzweigtes Alkoxy, Acyl, Alkylthio oder Alkoxycarbonyl mit jeweils bis zu 6 Kohlenstoffatomen oder Phenyl substituiert sind, das seinerseits durch Halogen substituiert sein kann, oder
für Aryl mit 6 bis 10 Kohlenstoffatomen steht, das gegebenenfalls durch Carboxy, Halogen, Cyano, Formyl, Trifluormethyl, Nitro, geradkettiges oder verzweigtes Alkoxy, Alkoxycarbonyl, Alkylthio oder Acyl mit jeweils bis zu 6 Kohlenstoffatomen oder durch geradkettiges oder verzweigtes Alkyl mit bis zu 6 Kohlenstoffatomen substituiert ist,
oder
für einen Rest der Formel -$NR^{20}R^{21}$, -$OR^{22}$ oder -$S(O)_c$-$R^{23}$ steht,
worin

$R^{20}$ Cycloalkyl mit 3 bis 6 Kohlenstoffatomen, Phenyl, geradkettiges oder verzweigtes Acyl mit bis zu 6 Kohlenstoffatomen oder geradkettiges oder verzweigtes Alkyl mit bis zu 8 Kohlenstoffatomen bedeutet, das gegebenenfalls durch Hydroxy, geradkettiges oder verzweigtes Alkoxy oder Hydroxy substituiertes Alkoxy mit jeweils bis zu 6 Kohlenstoffatomen, durch einen 5- bis 6-gliedrigen aromatischen Heterocyclus mit bis zu 3 Heteroatomen aus der Reihe S, N und/oder O, oder durch Phenyl substituiert ist, das seinerseits durch Hydroxy, Trifluormethyl, Halogen, Nitro oder durch geradkettiges oder verzweigtes Alkoxy mit bis zu 4 Kohlenstoffatomen substituiert sein kann, oder
Alkyl gegebenenfalls durch einen Rest der Formel -$NR^{24}R^{25}$ oder

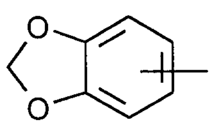

substituiert ist,

worin

$R^{24}$ und $R^{25}$ gleich oder verschieden sind und Wasserstoff oder geradkettiges oder verzweigtes Alkyl mit bis zu 4 Kohlenstoffatomen bedeuten, oder

$R^{20}$ einen Rest der Formel

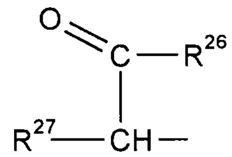

bedeutet worin

$R^{26}$ Hydroxy, geradkettiges oder verzweigtes Alkoxy mit bis zu 4 Kohlenstoffatomen, oder einen Rest der Formel $-NR^{28}R^{29}$ bedeutet, worin

$R^{28}$ und $R^{29}$ Wasserstoff oder geradkettiges oder verzweigtes Alkyl mit bis zu 5 Kohlenstoffatomen, Cycloalkyl mit 3 bis 6 Kohlenstoffatomen oder Phenyl bedeuten,

$R^{27}$ Wasserstoff oder geradkettiges oder verzweigtes Alkyl mit bis zu 7 Kohlenstoffatomen bedeutet, das gegebenenfalls durch Indolyl, Hydroxy, Mercaptyl, Imidazolyl, Methylthio, Amino, Phenyl, Hydroxy substituiertes Phenyl oder durch einen Rest der Formel $-CO-NH_2$, $-CO_2H$ oder

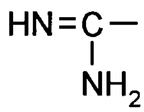

substituiert ist, oder

$R^{20}$ einen Rest der Formel

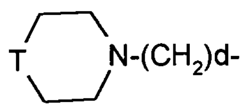

bedeutet

worin

d eine Zahl 0, 1, 2, 3, 4, 5 oder 6 bedeutet

T ein Sauerstoffatom oder eine Gruppe der Formel $CH_2$ oder $-NR^{30}$ bedeutet,

worin

$R^{30}$ Wasserstoff, Phenyl oder geradkettiges oder verzweigtes Alkyl mit bis zu 6 Kohlenstoffatomen bedeutet, das gegebenenfalls durch Hydroxy substituiert ist,

und

$R^{21}$ die oben angegebene Bedeutung von $R^{20}$ hat und mit dieser gleich oder verschieden ist, oder Wasserstoff bedeutet,

$R^{22}$ geradkettiges oder verzweigtes Alkyl mit bis zu 8 Kohlenstoffatomen bedeutet, das gegebenenfalls durch geradkettiges oder verzweigtes Alkoxy, oder Hydroxy oder Alkoxy substituiertes Alkoxy mit jeweils bis zu 6 Kohlenstoffatomen, Cycloalkyl mit 3 bis 6 Kohlenstoffatomen oder einen 6-gliedrigen aromatischen, gegebenenfalls benzokondensierten Heterocyclus mit bis zu 3 Stickstoffatomen substituiert ist,

der seinerseits bis zu 2fach gleich oder verschieden durch Nitro, Trifluormethyl, Halogen, Cyano, Hydroxy oder durch geradkettiges oder verzweigtes Alkyl, Alkoxy oder Acyl mit jeweils bis zu 5 Kohlenstoffatomen substituiert sein kann,

oder

$R^{22}$ einen Rest der Formel

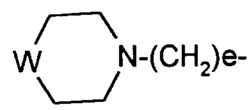

bedeutet,
worin

e die oben angegebene Bedeutung von d hat und mit dieser gleich oder verschieden ist,

W die oben angegebene Bedeutung von T hat und mit dieser gleich oder verschieden ist,

oder

$R^{22}$ Phenyl oder Pyridyl bedeutet,

c eine Zahl 0, 1 oder 2 bedeutet,

$R^{23}$ geradkettiges oder verzweigtes Alkyl oder Alkenyl mit bis zu 16 Kohlenstoffatomen bedeutet, das gegebenenfalls durch geradkettiges oder verzweigtes Alkoxycarbonyl mit bis zu 6 Kohlenstoffatomen oder Phenyl oder durch einen 5- bis 7-gliedrigen, aromatischen Heterocyclus mit bis zu 3 Heteroatomen aus der Reihe S, N oder O substituiert ist, oder
Aryl mit 6 bis 10 Kohlenstoffatomen oder einen 5- bis 7-gliedrigen aromatischen Heterocyclus mit bis zu 3 Heteroatomen aus der Reihe S, N oder O bedeutet,
und wobei die oben aufgeführten Cyclen gegebenenfalls bis zu 2fach gleich oder verschieden durch Carboxy, Halogen, Cyano, Formyl, Trifluormethyl, Nitro, geradkettiges oder verzweigtes Alkoxy, Alkoxycarbonyl, Alkylthio oder Acyl mit jeweils bis zu 6 Kohlenstoffatomen oder durch geradkettiges oder verzweigtes Alkyl mit bis zu 6 Kohlenstoffatomen substituiert sind

oder

$R^2$ für Morpholinyl oder für einen Rest der Formel

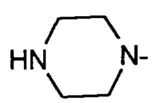 , 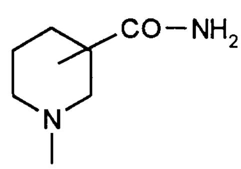 , 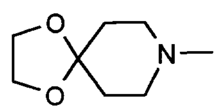

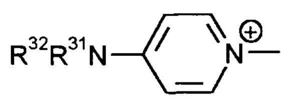 , 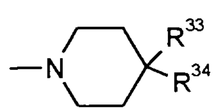 , 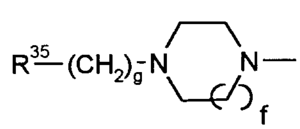

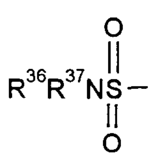 oder 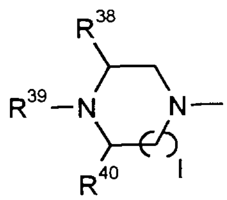

steht
worin

$R^{31}$ und $R^{32}$ die oben angegebene Bedeutung von $R^{24}$ und $R^{25}$ haben und mit dieser gleich oder verschieden sind,

$R^{33}$ und $R^{34}$ gemeinsam einen Rest der Formel =O bilden
oder

$R^{33}$ und $R^{34}$ gleich oder verschieden sind und Wasserstoff, Hydroxy oder geradkettiges oder verzweigtes Alkyl mit bis zu 6 Kohlenstoffatomen bedeuten, das gegebenenfalls durch eine Gruppe der Formel $-NR^{41}R^{42}$ substituiert ist,
worin

$R^{41}$ und $R^{42}$ die oben angegebene Bedeutung von $R^{24}$ und $R^{25}$ haben und mit dieser gleich oder verschieden sind

f eine Zahl 0 oder 1 bedeutet,

g eine Zahl 0, 1, 2, 3, 4, 5 oder 6 bedeutet,

$R^{35}$ Aryl mit 6 bis 10 Kohlenstoffatomen oder einen 5-bis 6-gliedrigen aromatischen, gegebenenfalls auch benzokondensierten Heterocyckus mit bis zu 3 Heteroatomen aus der Reihe S, N und oder O bedeutet, wobei alle Ringsysteme bis zu 3fach, gleich oder verschieden durch Nitro, Cyano, Hydroxy, Phenyl, Halogen, Trifluormethyl oder durch geradkettiges oder verzweigtes Alkyl, Alkoxy oder Acyl mit jeweils bis zu 5 Kohlenstoffatomen substituiert sein können, oder

$R^{35}$ Morpholinyl, Hydroxy, geradkettiges oder verzweigtes Alkoxy mit bis zu 6 Kohlenstoffatomen oder einen Rest der Formel

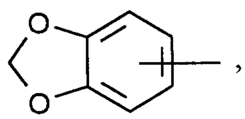

$-NR^{43}R^{44}$ oder $-CO-R^{45}$ bedeutet,
worin

$R^{43}$ und $R^{44}$ gleich oder verschieden sind und die oben angegebene Bedeutung von $R^{24}$ und $R^{25}$ haben,

$R^{45}$ Morpholinyl, Hydroxy oder geradkettiges oder verzweigtes Alkoxy mit bis zu 6 Kohlenstoffatomen bedeutet,

$R^{36}$ und $R^{37}$ gleich oder verschieden sind und Wasserstoff, Phenyl oder geradkettiges oder verzweigtes Alkyl mit bis zu 6 Kohlenstoffatomen oder Benzyl bedeutet,

$R^{38}$, $R^{39}$ und $R^{40}$ gleich oder verschieden sind und die oben angegebene Bedeutung von $R^{30}$ haben und mit dieser gleich oder verschieden sind,

l eine Zahl 1 oder 2 bedeutet,

und deren Salze.

Physiologisch unbedenkliche Salze der Benzoxazolyl- und Benzothiazolyloxazolidinone können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Als Salze können Salze mit üblichen Basen genannt werden, wie beispielsweise Alkalimetallsalze (z.B. Natrium- oder Kaliumsalze), Erdalkalisalze (z.B. Calcium-oder Magnesiumsalze) oder Ammoniumsalze, abgeleitet von Ammoniak oder organischen Aminen wie beispielsweise Diethylamin, Triethylamin, Ethyldiisopropylamin, Prokain, Dibenzylamin, N-Methylmorpholin, Dihydroabiethylamin, 1-Ephenamin oder Methyl-piperidin oder Pyridiniumsalze.

Als Salze können außerdem Reaktionsprodukte mit $C_1$-$C_4$-Alkylhalogenide, insbesondere $C_1$-$C_4$-Alkyljodide fungieren.

Heteroyclus steht im allgemeinen für einen 5- bis 6-gliedrigen, gesättigten oder ungesättigten Ring, der als Heteroatome bis zu 3 Sauerstoff-, Schwefel- und/oder Stickstoffatome enthalten kann. Bevorzugt werden genannt: Thienyl, Furyl, Pyrrolyl, Pyrazolyl, Pyridyl, Pyrimidyl, Pyrazinyl, Pyridazinyl, Thiazolyl, Oxazolyl, Imidazolyl, Pyrrolidinyl, Piperidinyl oder Piperazinyl.

Dazu gehören auch über N-gebundene, 5- bis 6-gliedrige gesättigte Heterocyclen, die außerdem als Heteroatome bis zu 2 Sauerstoff-, Schwefel- und/oder Stickstoffatome enthalten können, wie beispielsweise Piperidyl, Morpholinyl oder Piperazin oder Pyrrolidinyl. Besonders bevorzugt sind Piperidyl und Pyrrolidinyl. Hydroxyschutzgruppe im Rahmen der oben angegebenen Definition steht im allgemeinen für eine Schutzgruppe aus der Reihe: Trimethylsilyl, Triisopropylsilyl, tert.Butyl-dimethylsilyl, Benzyl, Benzyloxycarbonyl, 2-Nitrobenzyl, 4-Nitrobenzyl, tert. Butyloxycarbonyl, Allyloxycarbonyl, 4-Methoxybenzyl, 4-Methoxybenzyloxycarbonyl, Tetrahydropyranyl, Formyl, Acetyl, Trichloracetyl, 2,2,2-Trichlorethoxycarbonyl, Methoxyethoxymethyl, [2-(Trimethylsilyl)ethoxy]methyl, Benzoyl, 4-Methylbenzoyl, 4-Nitrobenzoyl, 4-Fluorbenzoyl, 4-Chlorbenzoyl oder 4-Methoxybenzoyl. Bevorzugt sind Acetyl, tert. Butyldimethylsilyl oder Tetrahydropyranyl.

Aminoschutzgruppe im Rahmen der Erfindung sind die üblichen in der Peptid-Chemie verwendeten Aminoschutzgruppen.

Hierzu gehören bevorzugt: Benzyloxycarbonyl, 2,4-Dimethoxybenzyloxycarbonyl, 4-Methoxybenzyloxycarbonyl, Methoxycarbonyl, Ethoxycarbonyl, tert.Butoxycarbonyl, Allyloxycarbonyl, Phthaloyl, 2,2,2-Trichlorethoxycarbonyl, Fluorenyl-9-methoxycarbonyl, Formyl, Acetyl, 2-Chloracetyl, 2,2,2-Trifluoracetyl, 2,2,2-Trichloracetyl, Benzoyl, 4-Chlorbenzoyl, 4-Brombenzoyl, 4-Nitrobenzoyl, Phthalimido, Isovaleroyl oder Benzyloxymethylen, 4-Nitrobenzyl, 2,4-Dinitrobenzyl, 4-Nitrophenyl, 4-Methoxyphenyl oder Triphenylmethyl.

Die erfindungsgemäßen Verbindungen können in stereoisomeren Formen, die sich entweder wie Bild und Spiegelbild (Enantiomere), oder die sich nicht wie Bild und Spiegelbild (Diastereomere) verhalten, existieren. Die Erfindung betrifft sowohl die Enantiomeren oder Diastereomeren oder deren jeweiligen Mischungen. Die Racemformen lassen sich ebenso wie die Diastereomeren in bekannter Weise in die stereoisomer einheitlichen Bestandteile trennen.

Bevorzugt sind Verbindungen der allgemeinen Formel (I),
in welcher

| | |
|---|---|
| A | für ein Sauerstoffatom oder für einen Rest der Formel - $S(O)_a$ steht, worin |
| a | eine Zahl 0 oder 2 bedeutet, |
| $R^1$ | für Azido oder für eine Gruppe der Formel $-OSO_2R^3$ oder $-NR^4R^5$ steht, worin |
| $R^3$ | geradkettiges oder verzweigtes Alkyl mit bis zu 3 Kohlenstoffatomen, Phenyl oder Toluolyl bedeutet, |
| $R^4$ und $R^5$ | gleich oder verschieden sind und Cyclopropyl, Cycylopentyl, Cyclohexyl, Wasserstoff, Phenyl oder geradkettiges oder verzweigtes Alkyl oder Alkoxy mit jeweils bis zu 6 Kohlenstoffatomen, tert. Butoxycarbonyl oder Benzyloxycarbonyl bedeuten, oder |
| $R^4$ oder $R^5$ | eine Gruppe der Formel $-CO-R^6$ bedeutet, worin |
| $R^6$ | Cyclopropyl, Cyclobutyl, Cyclopentyl, Cyclohexyl oder geradkettiges oder verzweigtes Alkyl oder Alkoxy mit jeweils bis zu 6 Kohlenstoffatomen, Phenyl oder Wasserstoff bedeutet, |
| G, L und M | gleich oder verschieden sind und für Wasserstoff, Carboxy, Fluor, Chlor, Brom, Jod, Cyano, Trifluormethyl, Formyl, Nitro, geradkettiges oder verzweigtes Alkoxy, Alkoxycarbonyl, Alkylthio oder Acyl mit jeweils bis zu 3 Kohlenstoffatomen oder für geradkettiges oder verzweigtes Alkyl mit bis zu 4 Kohlenstoffatomen stehen, das seinerseits durch Hydroxy, durch geradkettiges oder verzweigtes Alkoxy oder Acyl mit bis zu 4 Kohlenstoffatomen oder durch eine Gruppe der Formel $-NR^7R^8$ substituiert sein kann, worin |
| $R^7$ und $R^8$ | gleich oder verschieden sind und Wasserstoff, geradkettiges oder verzweigtes Alkyl mit bis zu 3 Kohlenstoffatomen oder Phenyl bedeuten, oder gemeinsam mit dem Stickstoffatom einen Morpholinyl-, Pyrrolidinyl-, Piperazinyl- oder Piperidylring bilden, die gegebenenfalls, auch über die freie N-Funktion, durch Methyl, Ethyl oder Acetyl substituiert sind, |

und/oder
gegebenenfalls für eine Gruppe der Formel $-NR^{7'}R^{8'}$ stehen,
worin

| | |
|---|---|
| $R^{7'}$ und $R^{8'}$ | die oben angegebene Bedeutung von $R^7$ und $R^8$ haben und mit dieser gleich oder verschieden sind, |

und/oder
gegebenenfalls für $(C_2$-$C_4)$-Alkenylphenyl, Phenyl, Pyridyl oder Thienyl stehen, die ihrerseits gegebenenfalls durch eine Gruppe der Formel $-CO-NR^9R^{10}$, $-NR^{11}R^{12}$, $-NR^{13}-SO_2-R^{14}$, $R^{15}R^{16}N-SO_2-$ oder $-R^{17}-S(O)_b$-substituiert sind,
worin

b — eine Zahl 0, 1 oder 2 bedeutet,

$R^9$, $R^{10}$, $R^{13}$, $R^{15}$ und $R^{16}$ — gleich oder verschieden sind und Wasserstoff, geradkettiges oder verzweigtes Alkyl mit bis zu 4 Kohlenstoffatomen oder Phenyl bedeuten,

$R^{11}$ und $R^{12}$ — gleich oder verschieden sind und die oben angegebene Bedeutung von $R^7$ und $R^8$ haben und mit dieser gleich oder verschieden sind,

$R^{14}$ und $R^{17}$ — gleich oder verschieden sind und die oben angegebene Bedeutung von $R^3$ haben und mit dieser gleich oder verschieden sind,

und/oder ihrerseits gegebenenfalls bis zu 2-fach gleich oder verschieden durch Carboxy, Fluor, Chlor, Brom, Jod, Cyano, Trifluormethyl, Formyl, Nitro, Phenyl geradkettiges oder verzweigtes Alkoxy, Alkoxycarbonyl, Alkylthio oder Acyl mit jeweils bis zu 4 Kohlenstoffatomen oder durch geradkettiges oder verzweigtes Alkyl mit bis zu 4 Kohlenstoffatomen substituiert sind, das seinerseits durch Hydroxy, durch geradkettiges oder verzweigtes Alkoxy oder Acyl mit bis zu 4 Kohlenstoffatomen oder durch eine Gruppe der Formel $-NR^{18}R^{19}$ gegebenenfalls substituiert sein kann,

worin

$R^{18}$ und $R^{19}$ — die oben angegebene Bedeutung von $R^7$ und $R^8$ haben und mit dieser gleich oder verschieden sind,

$R^2$ — für Wasserstoff, Formyl, Carboxy,

für geradkettiges oder verzweigtes Alkoxycarbonyl mit bis zu 5 Kohlenstoffatomen steht, oder

für geradkettiges oder verzweigtes Alkyl oder Alkenyl mit jeweils bis zu 6 Kohlenstoffatomen steht, die gegebenenfalls durch Hydroxy, Fluor, Chlor, Brom oder durch geradkettiges oder verzweigtes Alkoxy, Acyl, Alkylthio oder Alkoxycarbonyl mit jeweils bis zu 6 Kohlenstoffatomen oder durch Phenyl substituiert sind, das seinerseits durch Fluor, Chlor oder Brom substituiert sein kann,

für Phenyl steht, das gegebenenfalls durch Carboxy, Fluor, Chlor, Brom, Jod, Cyano, Trifluormethyl, Formyl, Nitro, geradkettiges oder verzweigtes Alkoxy, Alkoxycarbonyl, Alkylthio oder Acyl mit jeweils bis zu 4 Kohlenstoffatomen oder durch geradkettiges oder verzweigtes Alkyl mit bis zu 4 Kohlenstoffatomen substituiert ist,

oder

für einen Rest der Formel $-NR^{20}R^{21}$, $-OR^{22}$ oder $-S(O)_c-R^{23}$ steht,

worin

$R^{20}$ — Cyclopropyl, Cyclopentyl, Cyclohexyl, Phenyl, geradkettiges oder verzweigtes Acyl mit bis zu 4 Kohlenstoffatomen oder geradkettiges oder verzweigtes Alkyl mit bis zu 6 Kohlenstoffatomen bedeutet, das gegebenenfalls durch Hydroxy, geradkettiges oder verzweigtes Alkoxy oder Hydroxy substituiertes Alkoxy mit jeweils bis zu 5 Kohlenstoffatomen, Pyridyl, Pyrazinyl, Pyrimidyl, oder durch Phenyl substituiert ist, das seinerseits durch Hydroxy, Trifluormethyl, Fluor, Chlor, Brom, Nitro oder durch geradkettiges oder verzweigtes Alkoxy mit bis zu 3 Kohlenstoffatomen substituiert sein kann, oder Alkyl gegebenenfalls durch einen Rest der Formel $-NR^{24}R^{25}$ oder

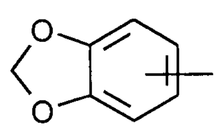

substituiert ist,

worin

$R^{24}$ und $R^{25}$ — gleich oder verschieden sind und Wasserstoff oder geradkettiges oder verzweigtes Alkyl mit bis zu 3 Kohlenstoffatomen bedeuten, oder

$R^{20}$ einen Rest der Formel

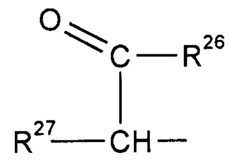

bedeutet
worin

$R^{26}$ Hydroxy, geradkettiges oder verzweigtes Alkoxy mit bis zu 3 Kohlenstoffatomen oder einen Rest der Formel $-NR^{28}R^{29}$ bedeutet,
worin

$R^{28}$ und $R^{29}$ gleich oder verschieden sind und Wasserstoff oder geradkettiges oder verzweigtes Alkyl mit bis zu 3 Kohlenstoffatomen Cyclopropyl, Cyclopentyl Cyclohexyl oder Phenyl bedeuten

$R^{27}$ Wasserstoff oder geradkettiges oder verzweigtes Alkyl mit bis zu 5 Kohlenstoffatomen bedeutet, das gegebenenfalls durch Phenyl substituiert ist,
oder

$R^{20}$ einen Rest der Formel

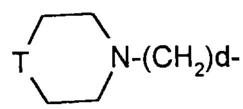

bedeutet
worin

d eine Zahl 0, 1, 2 oder 3 bedeutet

T ein Sauerstoffatom oder eine Gruppe der Formel $-CH_2$ oder $-NR^{30}$ bedeutet,
worin

$R^{30}$ Wasserstoff, Phenyl oder geradkettiges oder verzweigtes Alkyl mit bis zu 4 Kohlenstoffatomen bedeutet, das gegebenenfalls durch Hydroxy substituiert ist,
und

$R^{21}$ die oben angegebene Bedeutung von $R^{20}$ hat und mit dieser gleich oder verschieden ist, oder Wasserstoff bedeutet,

$R^{22}$ geradkettiges oder verzweigtes Alkyl mit bis zu 6 Kohlenstoffatomen bedeutet, das gegebenenfalls durch geradkettiges oder verzweigtes Alkoxy oder Hydroxy oder Alkoxy substituiertes Alkoxy mit jeweils bis zu 5 Kohlenstoffatomen, Cyclopropyl, Cyclopentyl, Cyclohexyl, Pyridyl, Pyrimidyl, Pyrazinyl oder Chinolyl substituiert ist,
die ihrerseits durch Nitro, Trifluormethyl, Fluor, Chlor, Brom, Cyano, Hydroxy oder durch geradkettiges oder verzweigtes Alkyl, Alkoxy oder Acyl mit jeweils bis zu 4 Kohlenstoffatomen substituiert sein können,
oder

$R^{22}$ einen Rest der Formel

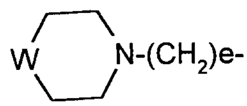

bedeutet,
worin

e die oben angegebene Bedeutung von d hat und mit dieser gleich oder verschieden ist,

W die oben angegebene Bedeutung von T hat und mit dieser gleich oder verschieden ist,

oder

$R^{22}$ Phenyl oder Pyridyl bedeutet,

c eine Zahl 0, 1 oder 2 bedeutet,

$R^{23}$ geradkettiges oder verzweigtes Alkyl oder Alkenyl mit jeweils bis zu 14 Kohlenstoffatomen bedeutet, das gegebenenfalls durch geradkettiges oder verzweigtes Alkoxycarbonyl mit bis zu 4 Kohlenstoffatomen oder durch Phenyl, Thienyl, Furyl, Pyrryl, Imidazolyl, Pyridyl, Pyrazinyl, Pyrimidyl oder Pyridazinyl substituiert ist, oder
Phenyl, Thienyl, Furyl, Pyrryl, Imidazolyl, Pyridin, Pyrazinyl, Pyrimidyl oder Pyridazinyl bedeutet,
und wobei die oben aufgeführten Cyclen gegebenenfalls bis zu 2-fach gleich oder verschieden durch Carboxy, Fluor, Chlor, Brom, Jod, Cyano, Trifluormethyl, Formyl, Nitro, geradkettiges oder verzweigtes Alkoxy, Alkoxycarbonyl, Alkylthio oder Acyl mit jeweils bis zu 4 Kohlenstoffatomen oder durch geradkettiges oder verzweigtes Alkyl mit bis zu 4 Kohlenstoffatomen substituiert sind

oder

$R^2$ für Morpholinyl oder für einen Rest der Formel

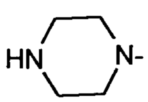 , 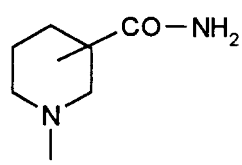 , 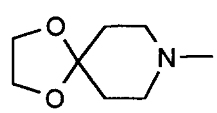

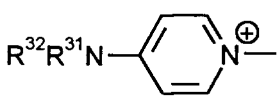 , 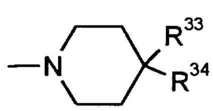 , 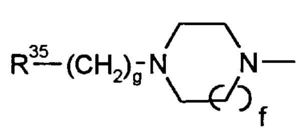

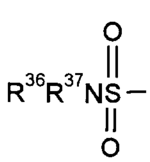 oder 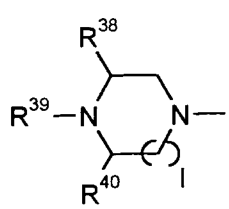

steht
worin

$R^{31}$ und $R^{32}$ die oben angegebene Bedeutung von $R^{24}$ und $R^{25}$ haben und mit dieser gleich oder verschieden sind,

$R^{33}$ und $R^{34}$ gemeinsam einen Rest der Formel =O bilden

oder

| | |
|---|---|
| $R^{33}$ und $R^{34}$ | gleich oder verschieden sind und Wasserstoff, Hydroxy oder geradkettiges oder verzweigtes Alkyl mit bis zu 4 Kohlenstoffatomen bedeuten, das gegebenenfalls durch eine Gruppe der Formel $-NR^{41}R^{42}$ substituiert ist, worin |
| $R^{41}$ und $R^{42}$ | die oben angegebene Bedeutung von $R^{24}$ und $R^{25}$ haben und mit dieser gleich oder verschieden sind |
| f | eine Zahl 0 oder 1 bedeutet, |
| g | eine Zahl 0, 1, 2, 3 oder 4 bedeutet, |
| $R^{35}$ | Phenyl, Pyridyl, Pyrimidyl, Pyrazinyl oder Chinolyl bedeutet, die ihrerseits bis zu 2-fach, gleich oder verschieden durch Nitro, Cyano, Hydroxy, Phenyl, Fluor, Chlor, Brom, Trifluormethyl oder durch geradkettiges oder verzweigtes Alkyl, Alkoxy oder Acyl mit jeweils bis zu 4 Kohlenstoffatomen substituiert sein können, oder |
| $R^{35}$ | Morpholinyl, Hydroxy, geradkettiges oder verzweigtes Alkoxy mit bis zu 4 Kohlenstoffatomen oder einen Rest der Formel 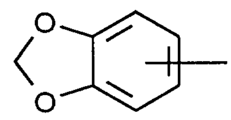 , $-NR^{43}R^{44}$ oder $-CO-R^{45}$ bedeutet, worin |
| $R^{43}$ und $R^{44}$ | gleich oder verschieden sind und die oben angegebene Bedeutung von $R^{24}$ und $R^{25}$ haben, |
| $R^{45}$ | Morpholinyl, Hydroxy oder geradkettiges oder verzweigtes Alkoxy mit bis zu 5 Kohlenstoffatomen bedeutet, |
| $R^{36}$ und $R^{37}$ | gleich oder verschieden sind und Wasserstoff, Phenyl oder geradkettiges oder verzweigtes Alkyl mit bis zu 4 Kohlenstoffatomen oder Benzyl bedeutet, |
| $R^{38}$, $R^{39}$ und $R^{40}$ | gleich oder verschieden sind und die oben angegebene Bedeutung von $R^{30}$ haben und mit dieser gleich oder verschieden sind, |
| l | eine Zahl 1 oder 2 bedeutet, |

und deren Salze.

Besonders bevorzugt sind Verbindungen der allgemeinen Formel (I), in welcher

| | |
|---|---|
| A | für ein Sauerstoffatom oder für einen Rest der Formel $-S(O)_a$ steht, worin |
| a | eine Zahl 0 oder 2 bedeutet, |
| $R^1$ | für Azido oder für eine Gruppe der Formel $-OSO_2R^3$ oder $-NR^4R^5$ steht, worin |
| $R^3$ | Methyl, Ethyl, Phenyl oder Toluolyl bedeutet, |
| $R^4$ und $R^5$ | gleich oder verschieden sind und Cyclopropyl, Cyclopentyl, Cyclohexyl, Wasserstoff, Phenyl oder geradkettiges oder verzweigtes Alkyl mit bis zu 5 Kohlenstoffatomen bedeuten, oder |
| $R^4$ oder $R^5$ | eine Gruppe der Formel $-CO-R^6$ bedeutet, worin |
| $R^6$ | Cyclopropyl, Cyclopentyl, Cyclohexyl oder geradkettiges oder verzweigtes Alkyl oder Alkoxy mit jeweils bis zu 4 Kohlenstoffatomen, Wasserstoff oder Phenyl bedeutet, |
| G, L und M | gleich oder verschieden sind und für Wasserstoff, Carboxy, Fluor, Chlor, Brom, Jod, Cyano, Formyl, Nitro, oder für geradkettiges oder verzweigtes Alkyl mit bis zu 3 Kohlenstoffatomen stehen, und/oder |

für eine Gruppe der Formel $-NR^{7'}R^{8'}$ stehen,
worin

$R^{7'}$ und $R^{8'}$ gleich oder verschieden sind und Wasserstoff oder Methyl bedeuten,

$R^2$ für Wasserstoff, Formyl, Carboxy, geradkettiges oder verzweigtes Alkoxycarbonyl mit bis zu 3 Kohlenstoffatomen oder für geradkettiges oder verzweigtes Alkyl oder Alkenyl mit jeweils bis zu 4 Kohlenstoffatomen steht, die gegebenenfalls durch Hydroxy, Fluor, Chlor, Brom oder durch geradkettiges oder verzweigtes Alkoxy, Acyl, Alkylthio oder Alkoxycarbonyl mit jeweils bis zu 3 Kohlenstoffatomen oder durch Phenyl substituiert sind, das seinerseits durch Chlor substituiert sein kann,
für Phenyl steht, das gegebenenfalls durch Carboxy, Fluor, Chlor, Brom, Jod, Cyano, Formyl, Trifluormethyl, Nitro, geradkettiges oder verzweigtes Alkoxy, Alkoxycarbonyl oder Acyl mit jeweils bis zu 3 Kohlenstoffatomen oder durch geradkettiges oder verzweigtes Alkyl mit bis zu 3 Kohlenstoffatomen substituiert ist,
oder
für einen Rest der Formel $-NR^{20}R^{21}$, $-OR^{22}$ oder $-S(O)_c-R^{23}$ steht,
worin

$R^{20}$ Cyclopropyl, Cyclopentyl, Cyclohexyl, Phenyl, geradkettiges oder verzweigtes Acyl mit bis zu 3 Kohlenstoffatomen, oder geradkettiges oder verzweigtes Alkyl mit bis zu 4 Kohlenstoffatomen bedeutet, das gegebenenfalls durch Hydroxy, geradkettiges oder verzweigtes Alkoxy oder Hydroxy substituiertes Alkoxy mit jeweils bis zu 3 Kohlenstoffatomen, Pyridyl, Pyrimidyl oder Pyrazinyl, oder durch Phenyl substituiert ist, das seinerseits durch Hydroxy, Trifluormethyl, Fluor, Chlor, Brom, Nitro, Methoxy oder Ethoxy substituiert sein kann, oder
Alkyl gegebenenfalls durch einen Rest der Formel $-NR^{24}R^{25}$ oder

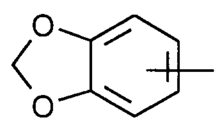

substituiert ist,
worin

$R^{24}$ und $R^{25}$ gleich oder verschieden sind und Wasserstoff, Methyl oder Ethyl bedeuten, oder

$R^{20}$ einen Rest der Formel

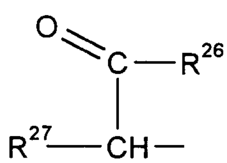

bedeutet
worin

$R^{26}$ Hydroxy, Methoxy, Ethoxy oder einen Rest der Formel $-NR^{28}R^{29}$ bedeutet, worin $R^{28}$ und $R^{29}$ gleich oder verschieden sind und Wasserstoff, Methyl, Ethyl, Cyclopropyl oder Phenyl bedeuten,

$R^{27}$ Wasserstoff oder geradkettiges oder verzweigtes Alkyl mit bis zu 3 Kohlenstoffatomen bedeutet, das gegebenenfalls durch Phenyl substituiert ist, oder

$R^{20}$ einen Rest der Formel

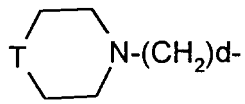

bedeutet
worin

d eine Zahl 0, 1 oder 2 bedeutet

T ein Sauerstoffatom oder eine Gruppe der Formel $-CH_2$ oder $-NR^{30}$ bedeutet,
worin

$R^{30}$ Wasserstoff oder geradkettiges oder verzweigtes Alkyl mit bis zu 3 Kohlenstoffatomen bedeutet, das gegebenenfalls durch Hydroxy substituiert ist,

und

$R^{21}$ die oben angegebene Bedeutung von $R^{20}$ hat und mit dieser gleich oder verschieden ist, oder Wasserstoff bedeutet,

$R^{22}$ geradkettiges oder verzweigtes Alkyl mit bis zu 4 Kohlenstoffatomen bedeutet, das gegebenenfalls durch geradkettiges oder verzweigtes Alkoxy oder Hydroxy oder Alkoxy substituiertes Alkoxy mit jeweils bis zu 4 Kohlenstoffatomen, Cyclopropyl, Cyclopentyl, Cyclohexyl, Pyridyl, Pyrazinyl oder Pyrimidyl substituiert ist, die ihrerseits durch Nitro, Trifluormethyl, Fluor, Chlor, Brom, Cyano, Hydroxy oder durch geradkettiges oder verzweigtes Alkyl, Alkoxy oder Acyl mit jeweils bis zu 3 Kohlenstoffatomen substituiert sein können,

oder

$R^{22}$ einen Rest der Formel

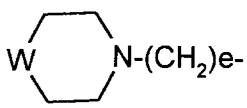

bedeutet,
worin

e die oben angegebene Bedeutung von d hat und mit dieser gleich oder verschieden ist,

W die oben angegebene Bedeutung von T hat und mit dieser gleich oder verschieden ist,

oder

$R^{22}$ Phenyl oder Pyridyl bedeutet,

c eine Zahl 0, 1 oder 2 bedeutet,

$R^{23}$ geradkettiges oder verzweigtes Alkyl oder Alkenyl mit bis zu 13 Kohlenstoffatomen bedeutet, das gegebenenfalls durch geradkettiges oder verzweigtes Alkoxycarbonyl mit bis zu 3 Kohlenstoffatomen oder Phenyl, durch Thienyl, Pyridyl, Pyrazinyl oder Pyrimidyl substituiert ist, oder
Phenyl, Thienyl, Pyridyl, Pyrazinyl oder Pyrimidyl bedeutet, und wobei die oben aufgeführten Cyclen gegebenenfalls durch Carboxy, Fluor, Chlor, Brom, Jod, Cyano, Formyl, Trifluormethyl, Nitro, geradkettiges oder verzweigtes Alkoxy, Alkoxycarbonyl oder Acyl mit jeweils bis zu 4 Kohlenstoffatomen oder durch geradkettiges oder verzweigtes Alkyl mit bis zu 4 Kohlenstoffatomen substituiert sind

oder

$R^2$ für Morpholinyl oder für einen Rest der Formel

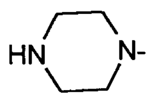 , 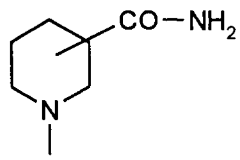 , 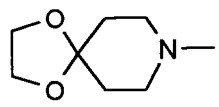

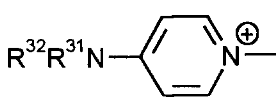 , 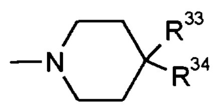 , 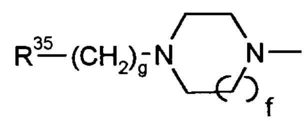

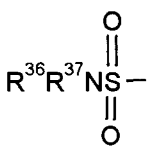 oder 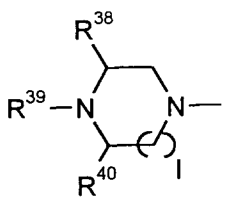

steht
worin

$R^{31}$ und $R^{32}$ die oben angegebene Bedeutung von $R^{24}$ und $R^{25}$ haben und mit dieser gleich oder verschieden sind,

$R^{33}$ und $R^{34}$ gemeinsam einen Rest der Formel =O bilden
oder

$R^{33}$ und $R^{34}$ gleich oder verschieden sind und Wasserstoff, Hydroxy oder geradkettiges oder verzweigtes Alkyl mit bis zu 3 Kohlenstoffatomen bedeuten, das gegebenenfalls durch eine Gruppe der Formel $-NR^{41}R^{42}$ substituiert ist,
worin

$R^{41}$ und $R^{42}$ die oben angegebene Bedeutung von $R^{24}$ und $R^{25}$ haben und mit dieser gleich oder verschieden sind

f eine Zahl 0 oder 1 bedeutet,

g eine Zahl 0, 1, 2 oder 3 bedeutet,

$R^{35}$ Phenyl, Pyridyl, Pyrazinyl oder Pyrimidyl bedeutet, die ihrerseits durch Nitro, Cyano, Hydroxy, Phenyl, Fluor, Chlor, Brom, Trifluormethyl oder durch geradkettiges oder verzweigtes Alkyl, Alkoxy oder Acyl mit jeweils bis zu 3 Kohlenstoffatomen substituiert sein können, oder

$R^{35}$ Morpholinyl, Hydroxy, geradkettiges oder verzweigtes Alkoxy mit bis zu 3 Kohlenstoffatomen oder einen Rest der Formel

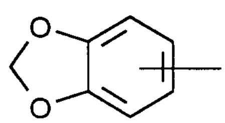 ,

$-NR^{43}R^{44}$ oder $-CO-R^{45}$ bedeutet,
worin

$R^{43}$ und $R^{44}$ gleich oder verschieden sind und die oben angegebene Bedeutung von $R^{24}$ und $R^{25}$ haben,

$R^{45}$ Morpholinyl, Hydroxy oder geradkettiges oder verzweigtes Alkoxy mit bis zu 4 Kohlenstoffatomen bedeutet,

$R^{36}$ und $R^{37}$ gleich oder verschieden sind und Wasserstoff, Phenyl oder geradkettiges oder verzweigtes Alkyl mit bis zu 3 Kohlenstoffatomen oder Benzyl bedeutet,

| | |
|---|---|
| $R^{38}$, $R^{39}$ und $R^{40}$ | gleich oder verschieden sind und die oben angegebene Bedeutung von $R^{30}$ haben und mit dieser gleich oder verschieden sind, |
| l | eine Zahl 1 oder 2 bedeutet, |

und deren Salze.

Ganz besonders bevorzugt sind Verbindungen der allgemeinen Formel (I),
in welcher
G, L und M für Wasserstoff stehen und der Oxazolidinonrest in den Positionen 5 oder 6 an den Phenylring angebunden ist.

Außerdem wurden Verfahren zur Herstellung der erfindungsgemäßen Verbindungen der allgemeinen Formel (I) gefunden, dadurch gekennzeichnet, daß man
[A] zunächst Verbindungen der allgemeinen Formel (II)

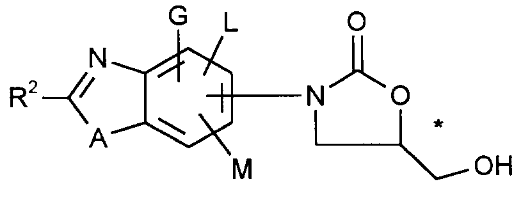 (II)

in welcher
A, G, L, M und $R^2$ die oben angegebene Bedeutung haben,
durch Umsetzung mit $(C_1\text{-}C_4)$-Alkyl- oder Phenylsulfonsäurechloriden in inerten Lösemitteln und in Anwesenheit einer Base in die entsprechenden Verbindungen der allgemeinen Formel (Ia)

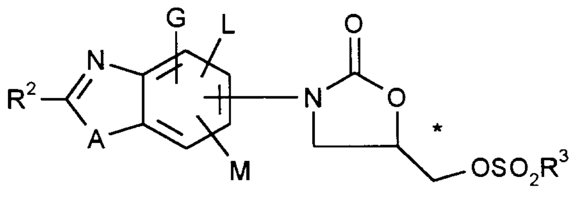 (Ia)

in welcher
A, G, L, M, $R^2$ und $R^3$ die oben angegebene Bedeutung haben,
überführt,
anschließend mit Natriumazid in inerten Lösemitteln die Azide der allgemeinen Formel (Ib)

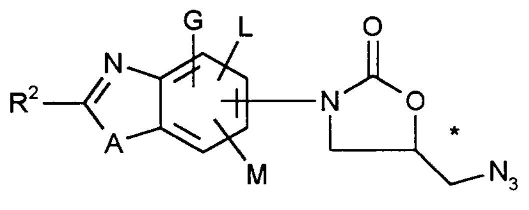 (Ib)

in welcher
A, G, L, M und $R^2$ die oben angegebene Bedeutung haben,
herstellt,
in einem weiteren Schritt durch Umsetzung mit $(C_1\text{-}C_4\text{-}O)_3\text{-}P$ oder $Ph_3P$, vorzugsweise $(CH_3O)_3P$ in inerten Lösemitteln

und mit Säuren in die Amine der allgemeinen Formel (Ic)

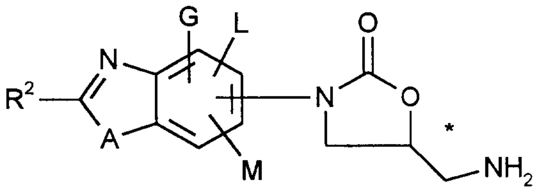

(Ic)

in welcher
A, G, L, M und $R^2$ die oben angegebene Bedeutung haben,
überführt,
und durch Umsetzung mit Acetanhydrid, Acetylchlorid oder anderen Acylierungsmitteln der allgemeinen Formel (III)

$$Y\text{-}CO\text{-}R^6 \qquad (III)$$

in welcher
$R^6$ die oben angegebene Bedeutung hat
und

Y für Halogen, vorzugsweise für Chlor oder für den Rest $-OCOR^6$ steht,

in inerten Lösemitteln die Verbindungen der allgemeinen Formel (Id)

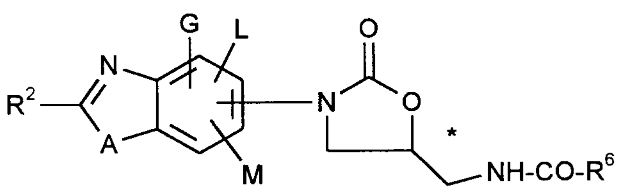

(Id)

in welcher
A, G, L, M, $R^2$ und $R^6$ die oben angegebene Bedeutung haben
herstellt,
und im Fall der S-Oxide, ausgehend von den entsprechenden S-Alkyl-Verbindungen mit m-Chlorperbenzoesäure oxidiert [B],
und im Fall von Substitutionsreaktionen, ausgehend von den Sulfonylverbindungen unter Substitution Nucleophile einführt [C],
und gegebenenfalls eine Alkylhalogenierung nach üblichen Methoden durchführt [D],
und gegebenenfalls ausgehend von den Verbindungen mit $R^2$ = 2-Phenylvinyl zunächst zu den entsprechenden Formylderivaten oxidiert und in einem zweiten Schritt nach bekannten Methoden reduziert [E],
und im Fall $R^4$, $R^5$, $R^7$, $R^{7'}$, $R^8$, $R^{8'}$, $R^9$, $R^{10}$, $R^{11}$, $R^{12}$, $R^{13}$, $R^{15}$, $R^{16}$, $R^{18}$, $R^{19}$, $R^{21}$, $R^{24}$, $R^{25}$, $R^{31}$, $R^{32}$, $R^{41}$, $R^{42}$, $R^{43}$ und/oder $R^{44} \neq H$ gegebenenfalls eine Alkylierung nach üblichen Methoden durchführt,
und gegebenenfalls weitere Substituenten oder bereits vorhandene funktionelle Gruppen nach üblichen Methoden, wie beispielsweise Redoxreaktionen, Substitutionsreaktionen und/oder Verseifungen oder Ein- und Abbau von Schutzgruppen, einführt bzw. derivatisiert.

Die erfindungsgemäßen Verfahren können durch folgende Formelschemata beispielhaft erläutert werden:

[A]

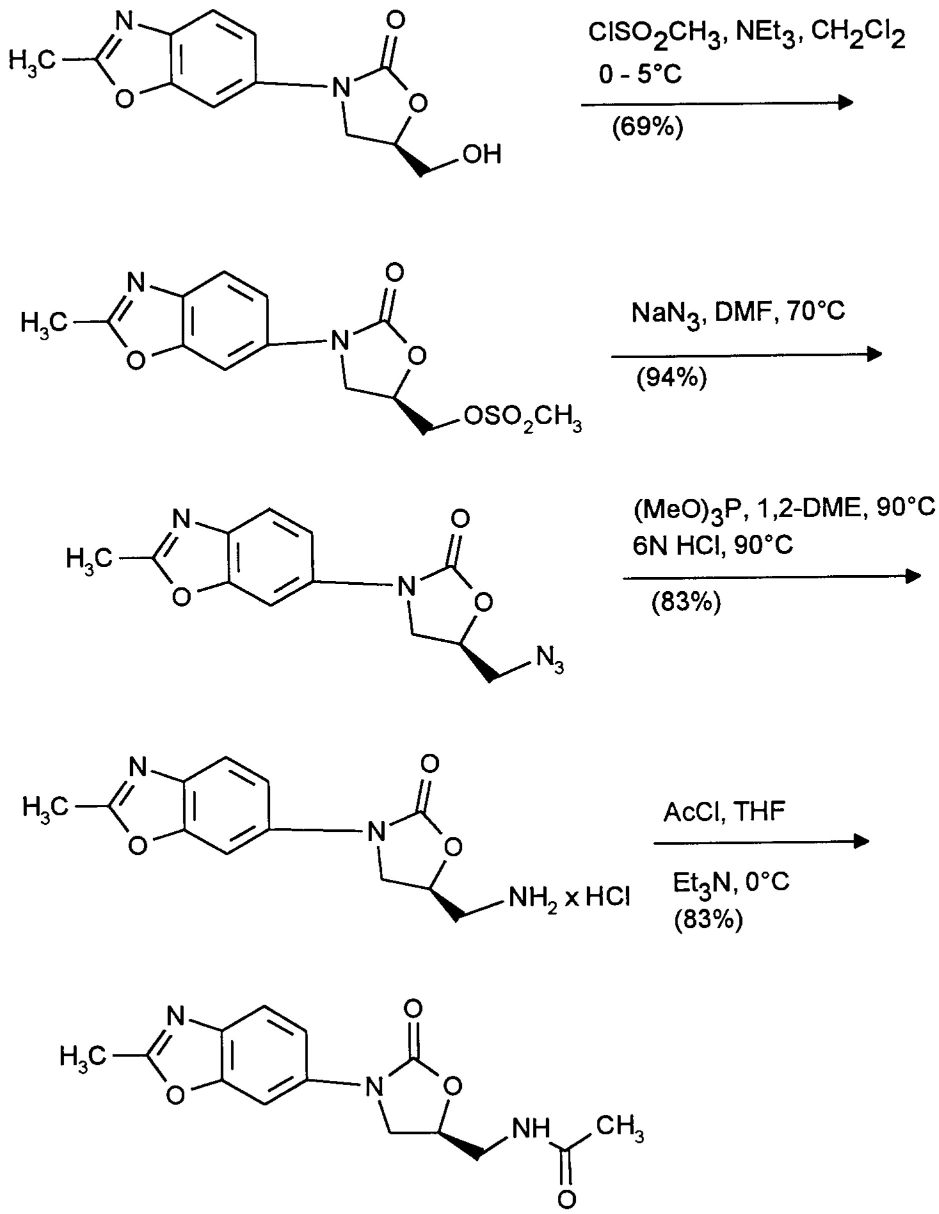

[B]

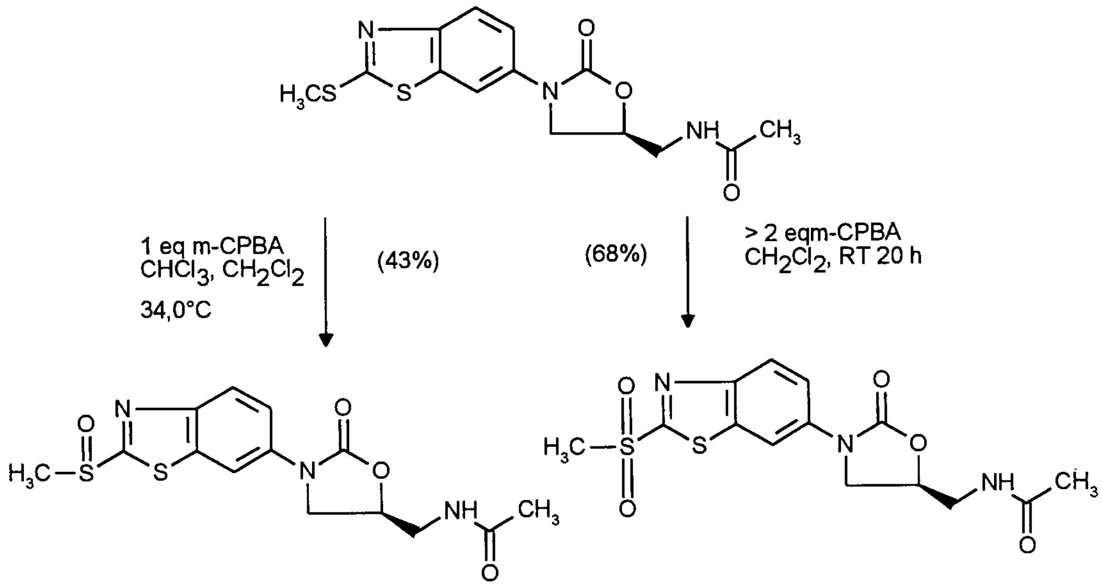

1 eq m-CPBA
$CHCl_3$, $CH_2Cl_2$
34,0°C
(43%)
(68%)
> 2 eqm-CPBA
$CH_2Cl_2$, RT 20 h


[C]

a)

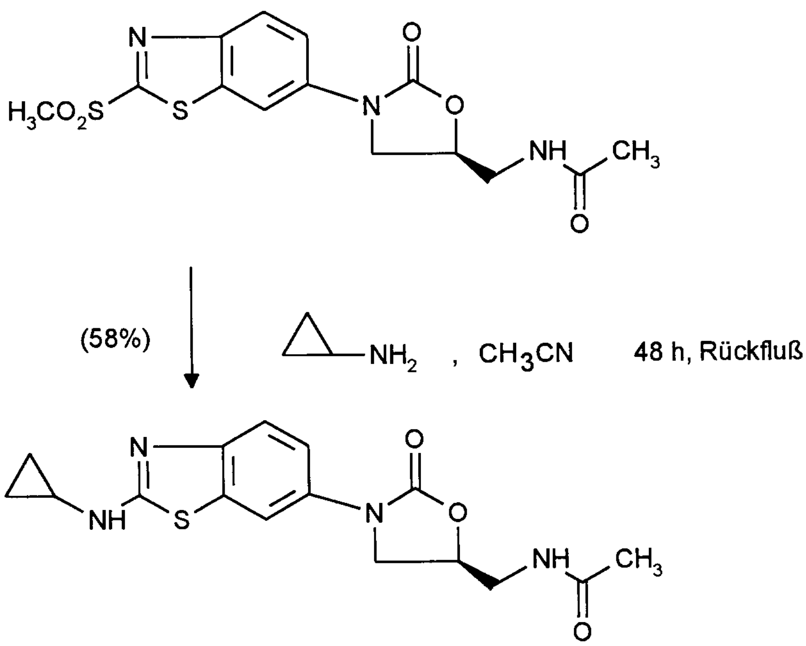

(58%)
, $CH_3CN$
48 h, Rückfluß

b)

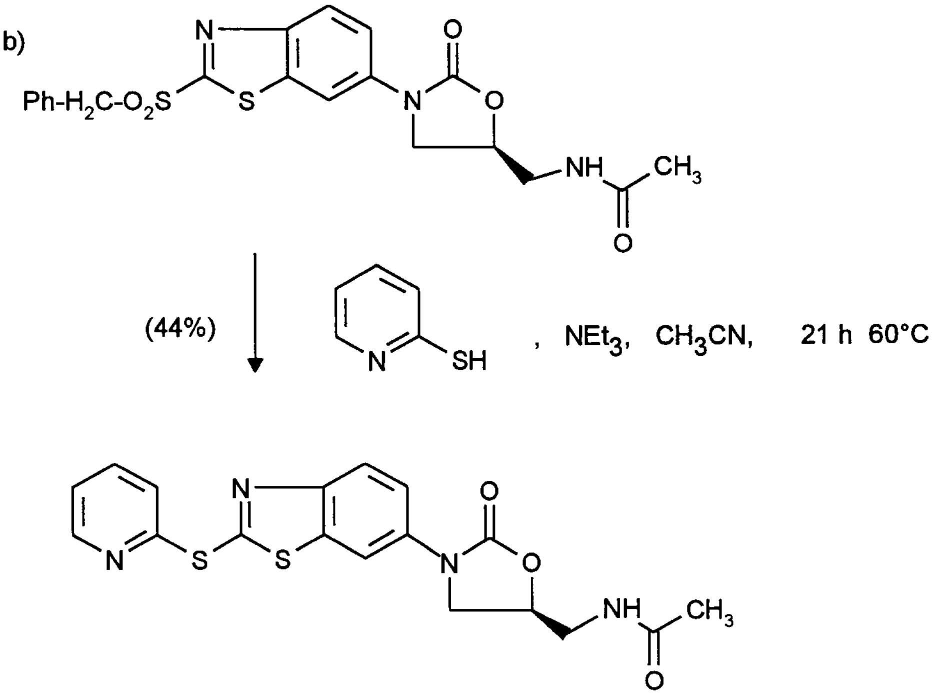


[D]

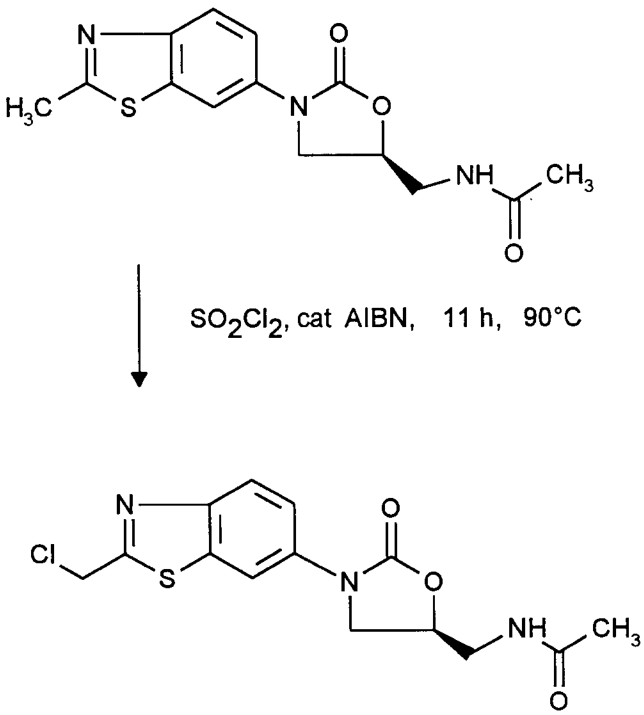

[E]

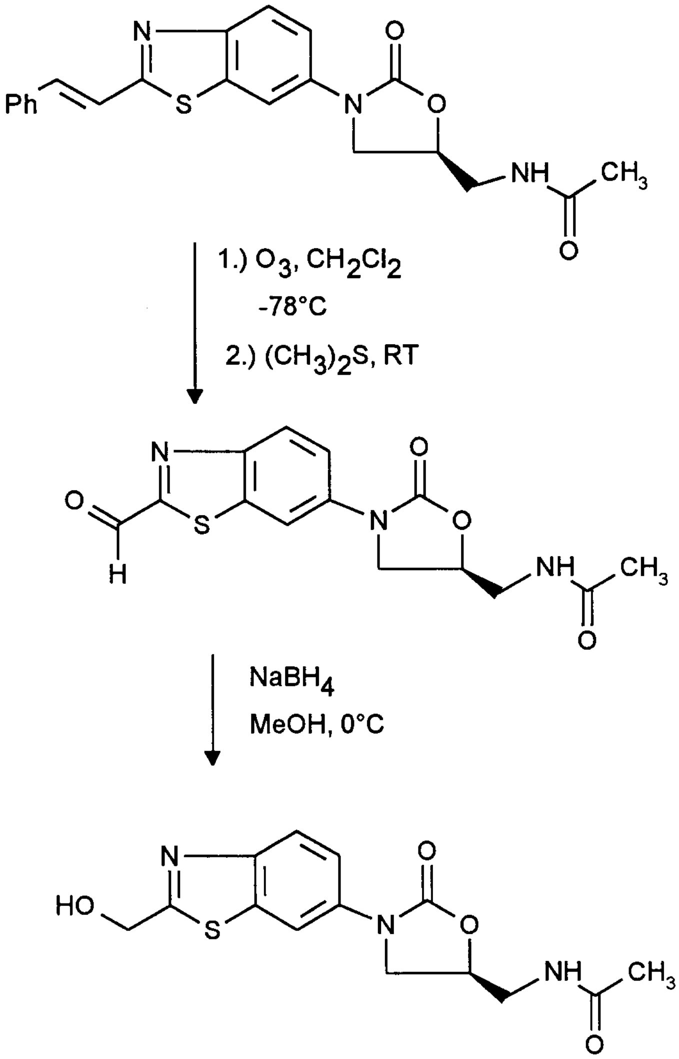


Als Lösemittel eignen sich in Abhängigkeit von den einzelnen Verfahrensschritten die üblichen Lösemittel, die sich unter den Reaktionsbedingungen nicht verändern. Hierzu gehören bevorzugt Alkohole wie Methanol, Ethanol, Propanol oder Isopropanol, oder Ether wie Diethylether, Dioxan, 1,2-Dimethoxyethan, Tetrahydrofuran, Glykoldimethylether oder tert.Butylmethylether, oder Ketone wie Aceton oder Butanon, oder Amide wie Dimethylformamid oder Hexamethyl-phosphorsäuretriamid, oder Kohlenwasserstoffe wie Hexan, Benzol, Dichlorbenzol, Xylol oder Toluol, oder Dimethylsulfoxid, Acetonitril, Essigester, oder Halogenkohlenwasserstoffe wie Methylenchlorid, Chloroform oder Tetrachlorkohlenstoff, oder Pyridin, Picolin oder N-Methylpiperidin. Ebenso können Gemische der genannten Lösemittel verwendet werden.

Als Basen eignen sich in Abhängigkeit von den einzelnen Verfahrensschritten die üblichen anorganischen oder organischen Basen. Hierzu gehören bevorzugt Alkalihydroxide wie beispielsweise Natrium- oder Kaliumhydroxid, oder Alkalicarbonate wie Natrium- oder Kaliumcarbonat, oder Alkalialkoholate wie beispielsweise Natrium- oder Kaliummethanolat, oder Natrium- oder Kaliumethanolat, oder organische Amine wie Ethyldiisopropylamin, Triethylamin, Picolin, Pyridine oder N-Methylpiperidin, oder Amide wie Natriumamid oder Lithiumdiisopropylamid, oder Lithium-N-silylalkylamide, wie beispielsweise Lithium-N-(bis)triphenysilylamid oder Lithiumalkyle wie n-Butyllithium.

Die Base wird in einer Menge von 1 mol bis 10 mol, bevorzugt von 1 mol bis 3 mol bezogen auf 1mol der Verbindungen der allgemeinen Formel (II) eingesetzt.

Alle Umsetzungen werden im allgemeinen bei normalem, erhöhtem oder bei erniedrigtem Druck durchgeführt (z.B. 0,5 bis 5 bar). Im allgemeinen arbeitet man bei Normaldruck.

Die Oxidation [B] erfolgt im allgemeinen in einem der oben aufgeführten Lösemittel, vorzugsweise in Methylenchlorid mit Oxidationsmitteln wie beispielsweise Metachlorperbenzoesäure, Wasserstoffperoxid oder Peressigsäure, vorzugsweise mit Metachlorperbenzoesäure in einem Temperaturbereich von 0°C bis 80°C bevorzugt von 0°C bis 40°C.

Die Substitution [C] erfolgt im allgemeinen in einem der oben aufgeführten Lösemitteln, vorzugsweise Acetonitril und in Anwesenheit einer der oben aufgeführten $C_1$-$C_4$-Alkylaminbasen, vorzugsweise Triethylamin in einem Temperaturbereich von +20°C bis 80°C, vorzugsweise bei 60°C bis 80°C und Normaldruck. Im Fall $R^2 = OR^{22}$ wird im allgemeinen die Umsetzung des Sulfons mit dem entsprechenden Alkoholat in Anwesenheit von Hydriden, vorzugsweise Kaliumhydrid, in einem der oben aufgeführten Lösemittel, vorzugsweise Dimethylformamid durchgeführt.

Die Alkylhalogenierung [D] erfolgt im allgemeinen mit Halogenierungsmitteln wie beispielsweise N-Brom-succinimid oder Sulfurylchlorid, vorzugsweise Sulfurylchlorid, in Anwesenheit eines Katalysators, wie beispielsweise Dibenzoylperoxid oder Azobisisobutyronitril, vorzugsweise Azobisisobutyronitril, in einem Temperaturbereich von +60°C bis +130°C, vorzugsweise bei +70°C bis +90°C und Normaldruck.

Die Oxidation zu den Formylderivaten [E] erfolgt im allgemeinen mit $O_3$ und anschließend $(CH_3)_2S$ in einem der oben aufgeführten Halogenkohlenwasserstoffen, vorzugsweise Dichlormethan und Methanol in einem Temperaturbereich von -78°C bis +40°C und Normaldruck.

Die Reduktionen erfolgen im allgemeinen mit Hydriden in inerten Lösemitteln oder mit Boranen, Diboranen oder ihren Komplexverbindungen.

Bevorzugt werden die Reduktionen mit Hydriden, wie komplexen Borhydriden oder Aluminiumhydriden sowie Boranen durchgeführt. Besonders bevorzugt werden hierbei Natriumborhydrid, Lithiumborhydrid, Natriumcyanoborhydrid, Lithiumaluminiumhydrid, Natrium-bis-(2-methoxyethoxy)aluminiumhydrid oder Boran-Tetrahydrofuran eingesetzt.

Die Reduktion erfolgt im allgemeinen in einem Temperaturbereich von - 50°C bis zum jeweiligen Siedepunkt des Lösemittels, bevorzugt von -20°C bis +90°C.

Die Reduktionen können im allgemeinen durch Wasserstoff in Wasser oder in inerten organischen Lösemitteln wie Alkoholen, Ethern oder Halogenkohlenwasserstoffen, oder deren Gemischen, mit Katalysatoren wie Raney-Nickel, Palladium, Palladium auf Tierkohle oder Platin, oder mit Hydriden oder Boranen in inerten Lösemitteln, gegebenenfalls in Anwesenheit eines Katalysators durchgeführt werden.

Bevorzugt wird die Reaktion mit Hydriden, wie komplexen Borhydriden oder Aluminiumhydriden durchgeführt. Besonders bevorzugt werden hierbei Natriumborhydrid, Lithiumaluminiumhydrid oder Natriumcyanoborhydrid eingesetzt.

Als Lösemittel eignen sich hierbei alle inerten organischen Lösemittel, die sich unter den Reaktionsbedingungen nicht verändern. Hierzu gehören bevorzugt Alkohole wie Methenol, Ethanol, Propanol oder Isopropanol, oder Ether wie Diethylether, Dioxan, Tetrahydrofuran, Glykoldimethylether, oder Diethylenglykoldimethylether oder Amide wie Hexamethylphosphorsäuretriamid oder Dimethylformamid, oder Essigsäure. Ebenso ist es möglich, Gemische der genannten Lösemittel zu verwenden. Besonders bevorzugt ist Methanol.

Die Abspaltung der Hydroxyschutzgruppen erfolgt im allgemeinen nach üblicher Methode, beispielsweise durch hydrogenolytische Spaltung der Benzylether in den oben aufgeführten inerten Lösemitteln in Anwesenheit eines Katalysators mit Wasserstoff-Gas.

Die Abspaltung der Aminoschutzgruppe erfolgt im allgemeinen, ebenfalls nach üblichen Methoden, abspaltet und zwar vorzugsweise Boc mit Salzsäure in Dioxan, Fmoc mit Piperidin und Z mit HBr/HOAc oder durch Hydrogenolyse.

Die oben aufgeführten anderen Derivatisierungsreaktionen erfolgen im allgemeinen nach denen in Compendium of Organic Synthetic Methods, T.T. Harrison und S. Harrison, Wiley Interscience, publizierten Methoden.

Bevorzugt werden Redoxreaktionen, reduktive Aminierung, Umesterung und die Halogenisierung von Methylgruppen mit N-Bromsuccinimid (NBS) oder N-Chlorsuccinimid (NCS) aufgeführt, die im folgenden beispielhaft erläutert werden.

Als Lösemittel für die Alkylierung eignen sich übliche organische Lösemittel, die sich unter den Reaktionsbedingungen nicht verändern. Hierzu gehören bevorzugt Ether wie Diethylether, Dioxan, Tetrahydrofuran, Glykoldimethylether, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, oder Triethylamin, Pyridin, Dimethylsulfoxid, Dimethylformamid, Acetonitril, Aceton oder Nitromethan. Ebenso ist es möglich, Gemische der genannten Lösemittel zu verwenden. Bevorzugt sind Dichlormethan, Dimethylsulfoxid und Dimethylformamid.

Die Alkylierung wird in den oben aufgeführten Lösemitteln bei Temperaturen von 0°C bis +150°C, vorzugsweise bei Raumtemperaturen bis +100°C, bei Normaldruck durchgeführt.

Die Amidierung und die Sulfoamidierung erfolgen im allgemeinen in inerten Lösemitteln in Anwesenheit einer Base und eines Dehydratisierungsmittels.

Als Lösemittel eignen sich hierbei inerte organische Lösemittel, die sich unter den Reaktionsbedingungen nicht verändern. Hierzu gehören Halogenkohlenwasserstoffe wie Dichlormethan, Trichlormethan, Tetrachlormethan, 1,2-Dichlorethan, Trichlorethan, Tetrachlorethan, 1,2-Dichlorethan oder Trichlorethylen, Kohlenwassserstoffe wie Benzol, Xylol, Toluol, Hexan, Cyclohexan, oder Erdölfraktionen, Nitromethan, Dimethylformamid, Acetonitril oder Tetrahydro-

furan. Ebenso ist es möglich, Gemische der Lösemittel einzusetzen. Besonders bevorzugt sind Dichlormethan und Tetrahydrofuran.

Als Basen für die Amidierung und die Sulfoamidierung eignen sich die üblichen basischen Verbindungen. Hierzu gehören vorzugsweise Alkali- und Erdalkalihydroxide wie Lithiumhydroxid, Natriumhydroxid, Kaliumhydroxid oder Bariumhydroxid, Alkalihydride wie Natriumhydrid, Alkali- oder Erdalkalicarbonate wie Natriumcarbonat, Kaliumcarbonat, oder Alkalialkoholate wie beispielsweise Natriummethanolat oder -ethanolat, Kaliummethanolat oder -ethanolat oder Kalium-tert.buylat, oder organische Amine wie Benzyltrimethylammoniumhydroxid, Tetrabutylammoniumhydroxid, Pyridin, Triethylamin oder N-Methylpiperidin.

Die Amidierung und die Sulfoamidierung werden im allgemeinen in einem Temperaturbereich von 0°C bis 150°C, bevorzugt bei 25°C bis 40°C, durchgeführt.

Die Amidierung und die Sulfoamidierung werden im allgemeinen bei Normaldruck durchgeführt. Es ist aber auch möglich, das Verfahren bei Unterdruck oder bei Überdruck durchzuführen (z.B. in einem Bereich von 0,5 bis 5 bar).

Bei der Durchführung der Amidierung und der Sulfoamidierung wird die Base im allgemeinen in einer Menge von 1 bis 3 Mol, bevorzugt von 1 bis 1,5 Mol, bezogen auf 1 Mol der jeweiligen Carbonsäure, eingesetzt.

Als Dehydratisierungsreagenzien eignen sich Carbodiimide wie beispielsweise Diisopropylcarbodiimid, Dicyclohexylcarbodiimid oder N-(3-Dimethylaminopropyl)-N'-ethylcarbodiimid-Hydrochlorid oder Carbonylverbindungen wie Carbonyldiimidazol oder 1,2-Oxazoliumverbindungen wie 2-Ethyl-5-phenyl-1,2-oxazolium-3-sulfonat oder Propanphosphorsäureanhydrid oder Isobutylchloroformat oder Benzotriazolyloxy-tris-(dimethylamino)phosphoniumhexyfluorophosphat oder Phosphonsäurediphenylesteramid oder Methansulfonsäurechlorid, gegebenenfalls in Anwesenheit von Basen wie Triethylamin oder N-Ethylmorpholin oder N-Methylpiperidin oder 4-Dimethylaminopyridin.

Als Basen eignen sich für die Verseifung die üblichen anorganischen Basen. Hierzu gehören bevorzugt Alkalihydroxide oder Erdalkalihydroxide wie beispielsweise Natriumhydroxid, Kaliumhydroxid oder Bariumhydroxid, oder Alkalicarbonate wie Natrium- oder Kaliumcarbonat oder Natriumhydrogencarbonat. Besonders bevorzugt werden Natriumhydroxid oder Kaliumhydroxid eingesetzt.

Als Lösemittel eignen sich für die Verseifung Wasser oder die für eine Verseifung üblichen organischen Lösemittel. Hierzu gehören bevorzugt Alkohole wie Methanol, Ethanol, Propanol, Isopropanol oder Butanol, oder Ether wie Tetrahydrofuran oder Dioxan, oder Dimethylformamid oder Dimethylsulfoxid. Besonders bevorzugt werden Alkohole wie Methanol, Ethanol, Propanol oder Isopropanol verwendet. Ebenso ist es möglich, Gemische der genannten Lösemittel einzusetzen.

Die Verseifung wird im allgemeinen in einem Temperaturbereich von 0°C bis +100°C, bevorzugt von +20°C bis +80°C durchgeführt.

Im allgemeinen wird die Verseifung bei Normaldruck durchgeführt. Es ist aber auch möglich, bei Unterdruck oder bei Überdruck zu arbeiten (z.B. von 0,5 bis 5 bar).

Bei der Durchführung der Verseifung wird die Base im allgemeinen in einer Menge von 1 bis 3 Mol, bevorzugt von 1 bis 1,5 Mol bezogen auf 1 Mol des Esters eingesetzt. Besonders bevorzugt verwendet man molare Mengen der Reaktanden.

Die Veresterung erfolgt im allgemeinen mit den entsprechenden Alkoholen in Anwesenheit von Säuren, vorzugsweise Schwefelsäure, in einem Temperaturbereich von 0°C bis 150°C, vorzugsweise von 50°C bis 100°C und Normaldruck.

Die Verbindungen der allgemeinen Formel (II) werden partiell vom Bedeutungsumfang der PCT 93 081 79 umfaßt, sind aber als konkrete Beispiele größtenteils neu und können beispielsweise hergestellt werden, indem man

[F] Verbindungen der allgemeinen Formeln (IV) oder (V)

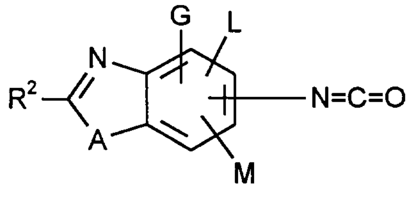 (IV) oder 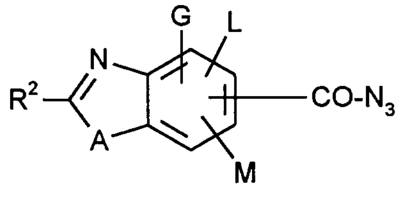 (V)

in welchen

A, G, L, M und $R^2$ die oben angegebene Bedeutung haben,

mit Lithiumbromid/$(C_4H_9)_3$ P(O) und Epoxiden der allgemeinen Formel (VI)

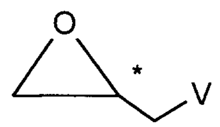

(VI)

in welcher

V für $C_1$-$C_6$-Acyloxy steht,

in inerten Lösemitteln, gegebenenfalls in Anwesenheit einer Base umsetzt,
und durch eine typische Esterverseifung oder durch eine typische Umesterung die Hydroxyfunktion freisetzt,
oder
[G] Verbindungen der allgemeinen Formel (VII)

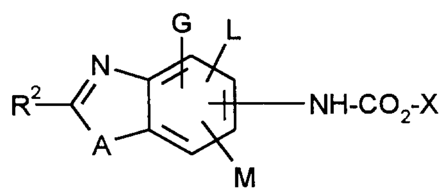

(VII)

in welcher

A die oben angegebene Bedeutung hat

und

X für eine typische Schutzgruppe, vorzugsweise Benzyl steht,

in inerten Lösemitteln und in Anwesenheit einer Base, beispielsweise Lithiumalkylen oder Lithium-N-alkyl- oder Lithium-N-silylalkylamiden, vorzugsweise N-Butyllithium, mit Epoxiden der allgemeinen Formel (VI) umsetzt,
oder
[H] zunächst Verbindungen der allgemeinen Formel (V) durch Abspaltung von Stickstoff in Alkoholen in die Verbindungen der allgemeinen Formel (VIIa)

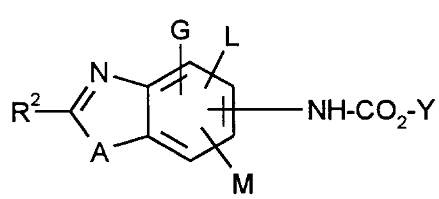

(VIIa)

in welcher
A, G, L, M und $R^2$ die oben angegebene Bedeutung haben
und

Y für geradkettiges oder verzweigtes $C_2$-$C_6$-Alkyl, vorzugsweise n-Butyl steht,

überführt,
und in einem zweiten Schritt wie unter [G] beschrieben in inerten Lösemitteln und in Anwesenheit einer Base, vorzugsweise Lithium-N-alkyl-oder N-Silylalkylamiden oder n-Butyllithium und Epoxiden der allgemeinen Formel (VI) umsetzt, oder

[I] Verbindungen der allgemeinen Formel (VIII)

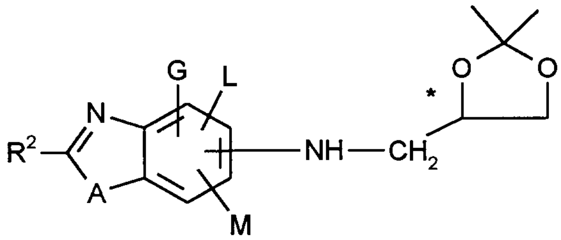

(VIII)

in welcher

A, G, L, M und $R^2$ die oben angegebene Bedeutung haben,

entweder direkt mit Säuren und Kohlensäurediethylester

umsetzt,

oder zunächst durch Umsetzung der Verbindungen der allgemeinen Formel (VIII) mit Säuren die Verbindungen der allgemeinen Formel (IX)

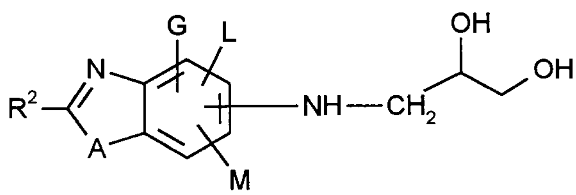

(IX)

in welcher

A, G, L, M und $R^2$ die oben angegebene Bedeutung haben

herstellt,

und anschließend in Anwesenheit eines Hilfsmittels in inerten Lösemitteln cyclisiert.

Die erfindungsgemäßen Verfahren können durch folgende Formelschemata beispielhaft erläutert werden:

[F]

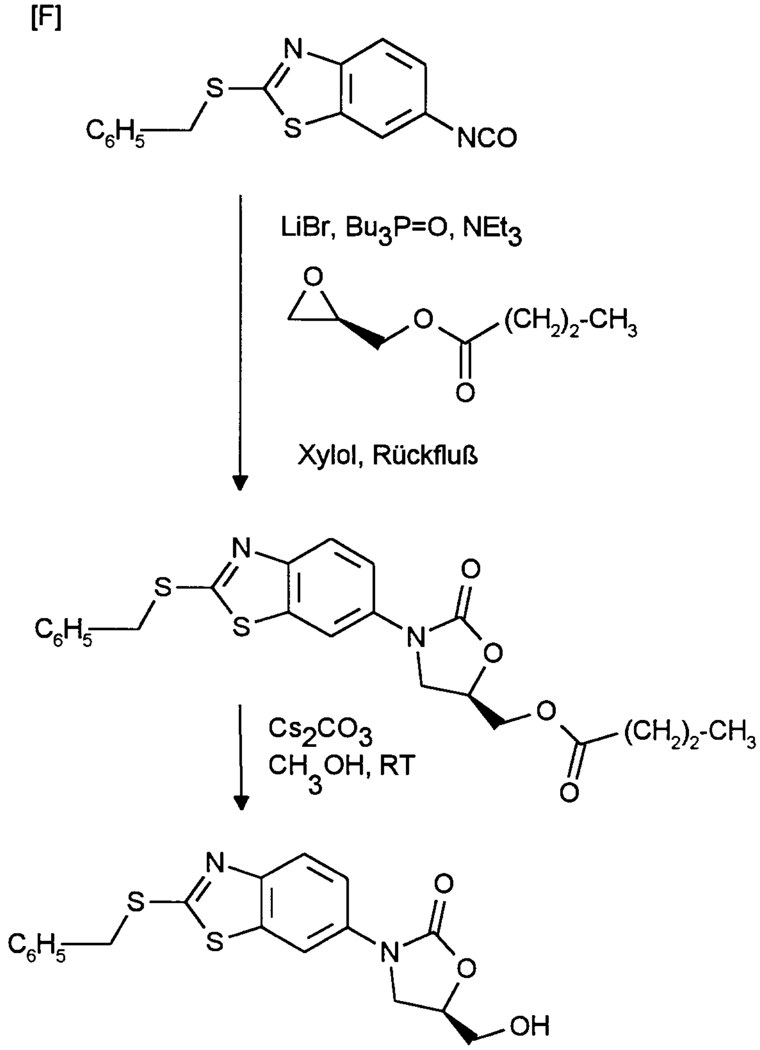


[G]

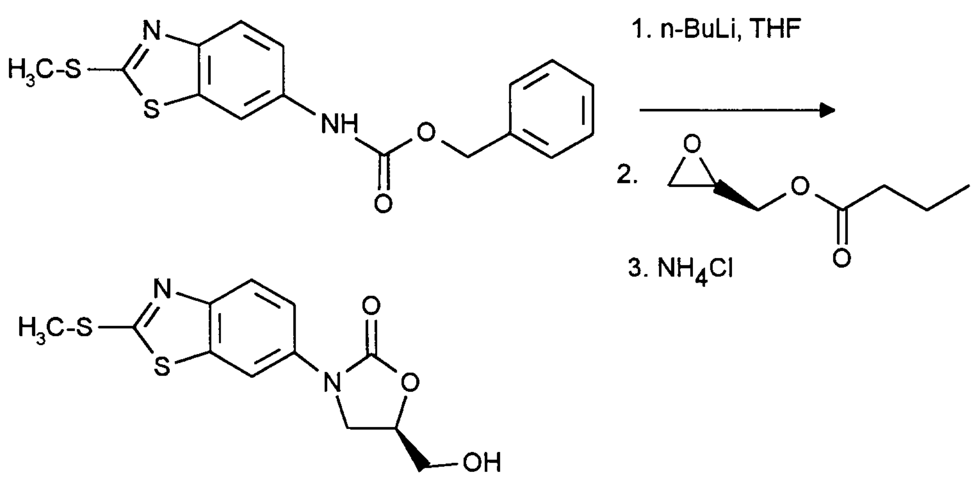

[H]

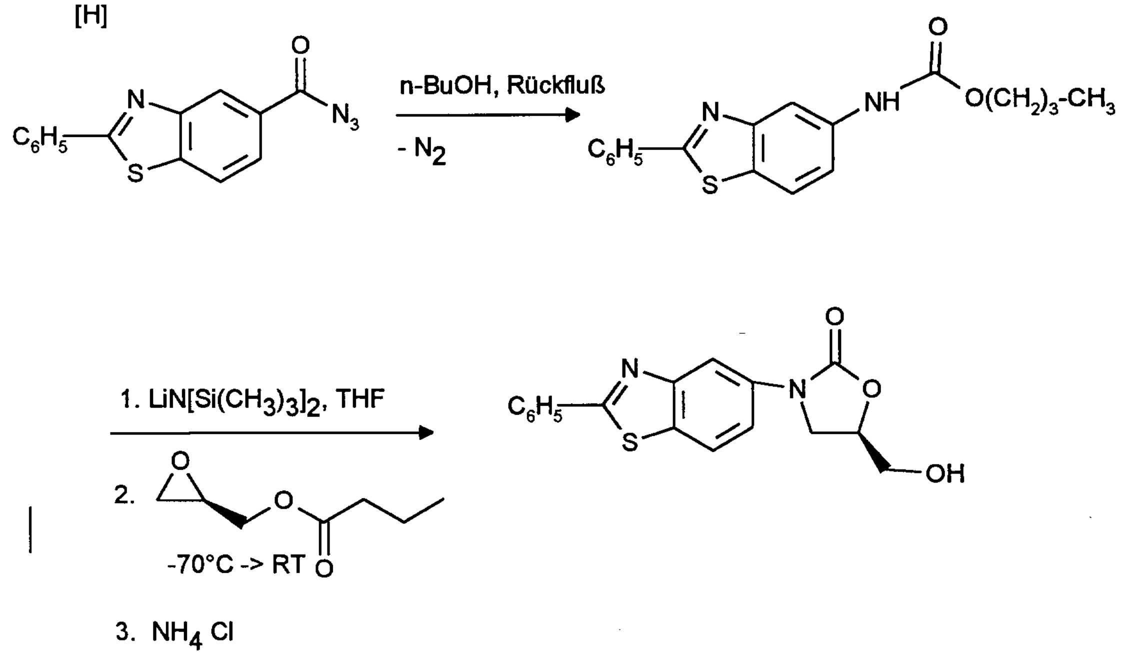


[I]

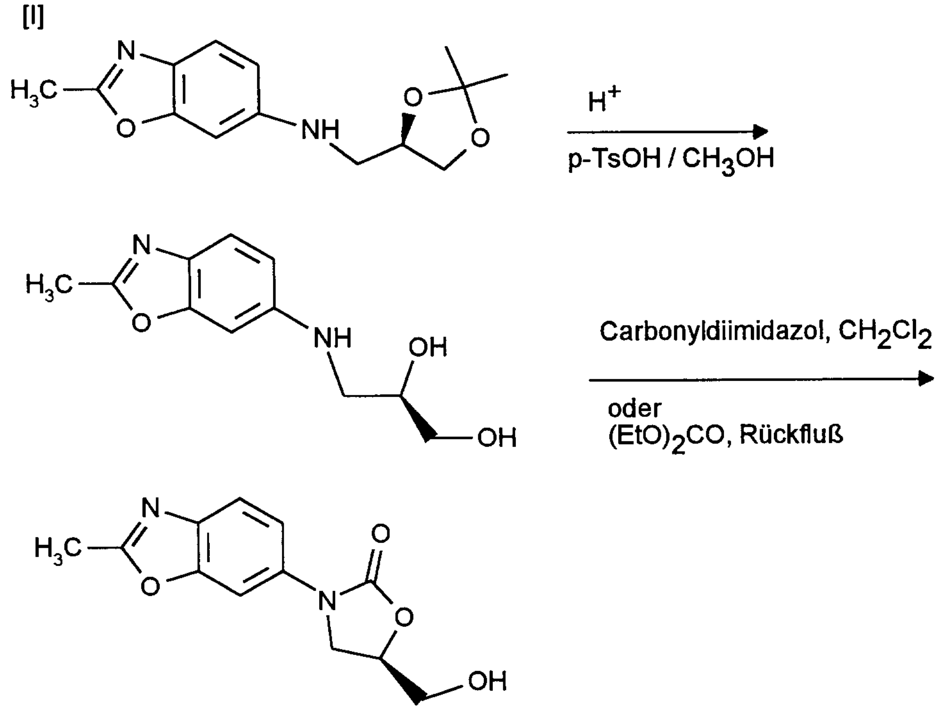


Als Lösemittel eignen sich in Abhängigkeit von den einzelnen Verfahrensschritten die üblichen Lösemittel, die sich unter den Reaktionsbedingungen nicht verändern. Hierzu gehören bevorzugt Alkohole wie Methanol, Ethanol, Propanol oder Isopropanol, oder Ether wie Diethylether, Dioxan, 1,2-Dimethoxyethan, Tetrahydrofuran, Glykoldimethylether oder tert.Butylmethylether, oder Ketone wie Aceton oder Butanon, oder Amide wie Dimethylformamid oder Hexamethyl-phosphorsäuretriamid, oder Kohlenwasserstoffe wie Hexan, Benzol, Dichlorbenzol, Xylol oder Toluol, oder Dimethylsulfoxid, Acetonitril, Essigester, oder Halogenkohlenwasserstoffe wie Methylenchlorid, Chloroform oder Tetrachlorkohlenstoff, oder Pyridin, Picolin oder N-Methylpiperidin. Ebenso können Gemische der genannten Lösemittel verwendet werden.

Als Basen eignen sich in Abhängigkeit von den einzelnen Verfahrensschritten die üblichen anorganischen oder organischen Basen. Hierzu gehören bevorzugt Alkalihydroxide wie beispielsweise Natrium- oder Kaliumhydroxid, oder Alkalicarbonate wie Natrium- oder Kaliumcarbonat, oder Alkalialkoholate wie beispielsweise Natrium- oder Kaliummethanolat, oder Natrium- oder Kaliumethanolat, oder organische Amine wie Ethyldiisopropylamin, Triethylamin, Picolin, Pyridine oder N-Methylpiperidin, oder Amide wie Natriumamid oder Lithiumdiisopropylamid, oder Lithium-N-silylalkylamide, wie beispielsweise Lithium-N-(bis)triphenysilylamid oder Lithiumalkyle wie n-Butyllithium.

Die Base wird in einer Menge von 1 mol bis 10 mol, bevorzugt von 1 mol bis 3 mol bezogen auf 1mol der Verbindungen der allgemeinen Formeln (IV), (V), (VI) und (VIIa) eingesetzt.

Alle Umsetzungen werden im allgemeinen bei normalem, erhöhtem oder bei erniedrigtem Druck durchgeführt (z.B. 0,5 bis 5 bar). Im allgemeinen arbeitet man bei Normaldruck.

Das Verfahren [F] erfolgt bevorzugt in Xylol oder Dichlorbenzol, gegebenenfalls in Anwesenheit von Triethylamin, unter Rückfluß.

Die basenkatalysierte Umesterung wird mit einem der oben aufgeführten Alkohole, vorzugsweise Methanol, in einem Temperaturbereich von -10°C bis +40°C, vorzugsweise bei Raumtemperatur durchgeführt.

Als Basen eignen sich im allgemeinen Natriumhydrogencarbonat, Natriummethanolat, Hydrazinhydrat, Kaliumcarbonat oder Caesiumcarbonat. Bevorzugt ist Caesiumcarbonat.

Das Verfahren [G] erfolgt in einem der oben aufgeführten Ether mit Lithiumalkylverbindungen oder Lithium-N-silylamiden, wie beispielsweise n-Butyllithium, Lithiumdiisopropylamid oder Lithium-bistrimethylsilylamid, vorzugsweise in Tetrahydrofuran mit Lithium-bistrimethylsilylamid oder n-Butyllithium, in einem Temperaturbereich von -100°C bis +20°C, vorzugsweise von -75°C bis -40°C.

Für das Verfahren [H] eignen sich für den 1. Schritt vorzugsweise die oben aufgeführten Alkohole, im Falle der anschließenden Cyclisierung Tetrahydrofuran.

Als Basen für die Cyclisierung eignen sich vorzugsweise die oben aufgeführten Lithium-N-silylalkylverbindungen oder n-Butyllithium. Besonders bevorzugt ist n-Butyllithium.

Der erste Reaktionsschritt wird bei der Siedetemperatur des entsprechenden Alkohols, die Cyclisierung in einem Temperaturbereich von -70°C bis Raumtemperatur durchgeführt.

Die Cyclisierung [I] wird in Anwesenheit eines Hilfsmittels und/oder Anwesenheit einer Säure durchgeführt.

Als Säuren eignen sich im allgemeinen anorganische Säuren wie beispielsweise Salzsäure oder Schwefelsäure, oder organische Carbonsäuren mit 1-6 C-Atomen, gegebenenfalls substituiert durch Fluor, Chlor und/oder Brom, wie beispielsweise Essigsäure, Trifluoressigsäure, Trichloressigsäure oder Propionsäure, oder Sulfonsäuren mit $C_1$-$C_4$-Alkylresten oder Arylresten wie beispielsweise Methansulfonsäure, Ethansulfonsäure, Benzolsulfonsäure oder Toluolsulfonsäure. Besonders bevorzugt ist Salzsäure.

Die Säure wird in einer Menge von 1 mol bis 10 mol, bevorzugt von 1 mol bis 2 mol, bezogen auf 1 mol der Verbindungen der allgemeinen Formel (VIII) eingesetzt.

Als Hilfsmittel eignen sich die üblichen Reagenzien wie Phosgen, Carbonyldiimidazol oder Kohlensäurediethylester oder Chlorameisensäuretrichlormethylester. Bevorzugt sind Carbonyldiimidazol, Kohlensäurediethylester oder Chlorameisensäuretrichlormethylester.

Als Lösemittel eignen sich die oben aufgeführten Halogenkohlenwasserstoffe. Bevorzugt ist Methylenchlorid.

Die Cyclisierungen erfolgen im allgemeinen in einem Temperaturbereich von -20°C bis 100°C, vorzugsweise bei -20°C bis Raumtemperatur.

Die Verbindungen der allgemeinen Formel (VI) sind bekannt oder können nach üblichen Methoden hergestellt werden.

Die Verbindungen der allgemeinen Formeln (VIII) und (IX) sind teilweise bekannt oder neu und können dann nach bekannten Methoden oder wie oben beschrieben hergestellt werden.

Die Verbindungen der allgemeinen Formel (IV) sind teilweise bekannt oder neu und können dann beispielsweise hergestellt werden, indem man die entsprechenden Amine mit Chlorameisensäuretrichlorethylester in einem der oben aufgeführten Lösemittel, vorzugsweise Xylol bei Rückflußtemperatur umsetzt.

Die Verbindungen der allgemeinen Formel (V) sind teilweise bekannt oder neu und können dann beispielsweise hergestellt werden, indem man ausgehend von den entsprechenen Carbonsäuren entweder mit Chlorameisensäureisobutylester / Aceton, Natriumazid/Wasser oder mit Diphenylphosphorylazid / Tetrahydrofuran oder mit Xylol oder Methylenchlorid in Gegenwart einer der oben angegebenen Basen, vorzugsweise Triethylamin, bei - 10°C bis Raumtemperatur umsetzt.

Die Verbindungen der allgemeinen Formeln (VII) und (VIIa) sind teilweise bekannt oder neu und können entweder durch Abspaltung von Stickstoff aus den entsprechenden Carbonsäureaziden und Umsetzung mit den entsprechenden Alkoholen oder durch Umsetzung der entsprechenden Amine mit Chlorameisensäureestern, vorzugsweise Chlorameisensäurebenzylester in einem der oben aufgeführten Lösemittel, vorzugsweise Tetrahydrofuran oder Dioxan, in einem Temperaturbereich von -10°C bis 200°C, vorzugsweise von 0°C bis 150°C, hergestellt werden.

Die Verbindungen der allgemeinen Formel (III) sind bekannt oder können nach üblichen Methoden hergestellt werden.

Die Verbindungen der allgemeinen Formel (Ia), (Ib), (Ic) und (Id) sind neu und können wie oben beschrieben hergestellt werden.

Die MHK-Werte wurden mit Hilfe der Mikrodilutionsmethode in BH-Medium bestimmt. Jede Prüfsubstanz wurde im Nährmedium gelöst. In der Mikrotiterplatte wurde durch serielle Verdünnung eine Konzentrationsreihe der Prüfsubstanzen angelegt. Zur Inokulation wurden Übernachtkulturen der Erreger verwandt, die zuvor im Nährmedium 1:250 verdünnt wurden. Zu 100 µl der verdünnten, wirkstoffhaltigen Nährlösungen wurden je 100 µl Inokulationslösung gegeben.

Die Mikrotiterplatten wurden bei 37°C bebrütet und nach ca. 20 Stunden abgelesen. Der MHK-Wert (µg/ml) gibt die niedrigste Wirkstoffkonzentration an, bei der kein Wachstum zu erkennen war.

Die minimalen Hemmkonzentrationen (MHK) wurden per Reihenverdünnungsverfahren auf Iso-Sensitest Agar (Oxoid) bestimmt. Für jede Prüfungssubstanz wurde eine Reihe von Agarplatten hergestellt, die bei jeweils doppelter Verdünnung abfallende Konzentrationen des Wirkstoffes enthielten. Die Agarplatten wurden mit einem Multipoint-Inokulator (Denley) beimpft. Zum Beimpfen wurden Übernachtkulturen der Erreger verwandt, die zuvor so verdünnt wurden, daß jeder Impfpunkt ca. $10^4$ koloniebildende Partikel enthielt. Die beimpften Agarplatten wurden bei 37°C bebrütet, und das Keimwachstum wurde nach ca. 20 Stunden abgelesen. Der MHK-Wert (µg/ml) gibt die niedrigste Wirkstoffkonzentration an, bei der mit bloßem Auge kein Wachstum zu erkennen war.

**MHK-Werte (µg/ml):**

| Bsp.-Nr. | Staph. 133 | Staph. 48N | Staph 25701 | Staph. 9TV | E. coli Neumann | Klebs. 57 USA | Psdm. Bonn |
|---|---|---|---|---|---|---|---|
| 24 | 2 | 1 | 0,25 | 0,25 | > 64 | > 64 | > 64 |
| 25 | 2 | 1 | 1 | 1 | > 64 | > 64 | > 64 |
| 26 | 2 | 1 | 1 | 1 | > 64 | > 64 | > 64 |
| 32 | 1 | 2 | 1 | 0,5 | > 64 | > 64 | > 64 |
| 33 | 4 | 2 | 2 | 4 | > 64 | > 64 | > 64 |
| 36 | 4 | 4 | 4 | 2 | > 64 | > 64 | > 64 |
| 37 | 2 | 2 | 1 | 1 | > 64 | > 64 | > 64 |

Die erfindungsgemäßen Verbindungen der allgemeinen Formeln (I), (Ia), (Ib), (Ic), und (Id) weisen bei geringer Toxizität ein breites antibakterielles Spektrum, speziell gegen gram-positive Bakterien sowie Mycobacterien, Corynebacterien, Haemophilus Influenzae und Anaerobae Keime auf. Diese Eigenschaften ermöglichen ihre Verwendung als chemotherapeutische Wirkstoffe in der Human-und Tiermedizin.

Die erfindungsgemäßen Verbindungen sind gegen ein breites Spektrum von Mikroorganismen wirksam. Mit ihrer Hilfe können gram-positive Bakterien und bakterienähnliche Mikroorganismen, wie Mycoplasmen, bekämpft sowie die durch diese Erreger hervorgerufenen Erkrankungen verhindert, gebessert und/oder geheilt werden.

Besonders wirksam sind die erfindungsgemäßen Verbindungen gegen Bakterien und bakterienähnliche Mikroorganismen. Sie sind daher besonders gut zur Prophylaxe und Chemotherapie von lokalen und systemischen Infektionen in der Human- und Tiermedizin geeignet, die durch solche Erreger hervorgerufen werden.

Zur vorliegenden Erfindung gehören pharmazeutische Zubereitungen, die neben nicht-toxischen, inerten pharmazeutisch geeigneten Trägerstoffen eine oder mehrere erfindungsgemäße Verbindungen enthalten oder die aus einem oder mehreren erfindungsgemäßen Wirkstoffen bestehen, sowie Verfahren zur Herstellung dieser Zubereitungen.

Der oder die Wirkstoffe können gegebenenfalls in einem oder mehreren der oben angegebenen Trägerstoffe auch in mikroverkapselter Form vorliegen.

Die therapeutisch wirksamen Verbindungen sollen in den oben aufgeführten pharmazeutischen Zubereitungen vorzugsweise in einer Konzentration von etwa 0,1 bis 99,5 vorzugsweise von etwa 0,5 bis 95 Gew.-%, der Gesamtmischung vorhanden sein.

Die oben aufgeführten pharmazeutischen Zubereitungen können außer den erfindungsgemäßen Verbindungen auch weitere pharmazeutische Wirkstoffe enthalten.

Im allgemeinen hat es sich sowohl in der Human- als auch in der Veterinärmedizin als vorteilhaft erwiesen, den oder die erfindungsgemäßen Wirkstoffe in Gesamtmengen von etwa 0,5 bis etwa 500, vorzugsweise 5 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die erfindungsgemäßen Wirkstoffe vorzugsweise in Mengen von etwa 1 bis etwa 80, insbesondere 3 bis 30mg/kg Körpergewicht.

Die neuen Verbindungen können in den üblichen Konzentrationen und Zubereitungen zusammen mit dem Futter bzw. Lactamaseinhibitoren z.B. mit Penicillinen, die besonders penicillinasefest sind und Clavulansäure kombiniert werden. Eine solche Kombination wäre z.B. die mit Oxacillin oder Dicloxacillin.

Die erfindungsgemäßen Verbindungen können zum Zweck der Erweiterung des Wirkungsspektrums und um eine Wirkungssteigerung zu erreichen auch mit anderen Antibiotika kombiniert werden.

## Anhang zum experimentellen Teil

Liste der verwendeten Laufmittelgemische zur Chromatographie:

| | |
|---|---|
| I | Dichormethan : Methanol |
| II | Toluol : Ethylacetat |
| III | Acetonitril : Wasser |
| IV | Ethylacetat |
| V | Petrolether : Ethylacetat |

## Abkürzungen:

| | |
|---|---|
| Z | Benzyloxycarbonyl |
| Boc | tert.Butyloxycarbonyl |
| DMF | Dimethylformamid |
| Ph | Phenyl |
| Me | Methyl |
| THF | Tetrahydrofuran |
| CDI | Carbonylimidazol |
| DCE | Dichlorethan |

**Ausgangsverbindungen**

**Beispiel I**

2-Benzylthio-6-isocyanato-benzo[4,5-d]thiazol Hydrochlorid

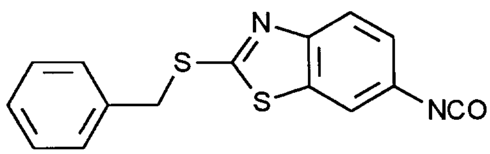

x HCl

Zu einer gerührten Suspension von 26,05 g (0,096 mol) 6-Amino-2-benzylthiobenzo[4,5-d]-thiazol in 250 ml 1,2-Dichlorethan tropft man in der Siedehitze 23,0 ml (0,19 mol) Chlorameisensäuretrichlorethylester. Nach der Zugabe wird 0,5 h im Rückfluß gekocht, wobei eine klare Lösung entsteht, dann darf sich das Gemisch auf Raumtemperatur abkühlen. Das Reaktionsgemisch wird am Rotationsverdampfer eingeengt und das zurückbleibende Öl im Hochvakuum getrocknet. Man erhält 21,5 g (67%) der Titelverbindung als wachsartigen Feststoff.
Schmp.: 54°C
$R_f$ = ca. 0,4 (Toluol : Ethylacetat 4:1)
MS (DCI, $NH_3$) m/z = 299 $(M+H)^+$
$^1$H-NMR (250 MHz, $D_6$-DMSO): δ = 4,65 (s, 2H, $CH_2$); 7,3 (m, 5H Ph); 7,5 (m, 2H); 7,85 (d, J = 9 Hz, 1H).

Wie für Beispiel I beschrieben, erhält man aus den entsprechenden heteroaromatischen Aminen durch Umsetzung mit Chlorameisensäuretrichlormethylester die Hydrochloride folgender Isocyanate:

**Tabelle I:**

D-N=C=O x HCl

| Bsp.-Nr. | D | Ausbeute (% d.Th.) | Schmp. (°C) | MS m/z = $(M)^+$ |
|---|---|---|---|---|
| II | 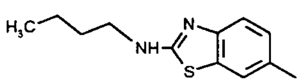 | 47 | 211 | 247 |

**Beispiel III**

6-Benzyloxycarbonylamino-2-methylthio-benzo[4,5-d]thiazol

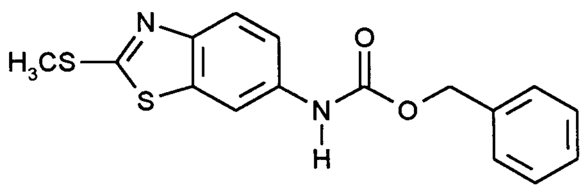

Zu einer auf 0°C gekühlten, gerührten Lösung von 17,92 g (91,30 mmol) 6-Amino-2-methylthio-benzo[4,5-d]thiazol in 240 ml Wasser und 60 ml THF werden innerhalb von 30 min 14,80 ml (100,40 mmol) Chlorameisensäurebenzylester getropft, wobei pH = 10 durch gleichzeitige Zugabe einer 4 H NaOH Lösung gehalten wird. Man rührt noch 0,5 h bei 0°C nach, dampft das THF im Vakuum ab, trennt den entstandenen Niederschlag durch Filtration verrührt gut mit 150 ml Pentan und trocknet im Hochvakuum. Man erhält 28,96 g (96%) der Titelverbindung als Kristalle.
Schmp.: 111°C
$R_f$ = 0,71 (Toluol : Ethylacetat 1:4)
MS (DCI, $NH_3$) m/z = 331 $(M+H)^+$
$^1$H-NMR (200 MHz, $D_6$-DMSO): δ = 2,76 (s, 3H, $CH_3S$); 5,18 (s, 2H, $CH_2$); 7,4 (m, 6H, Ph, H-5); 7,77 (d, J = 10 Hz, 1H, H-4); 8,18 (d, J = 1,5 Hz, 1H, H-7); 10,03 (bs, 1H, NH).

Wie für Beispiel III beschrieben erhält man aus den entsprechenden Heteroaromaten durch Umsetzung mit Chlorameisensäurebenzylester die in der Tabelle II aufgeführten Verbindungen:

**Tabelle II:**

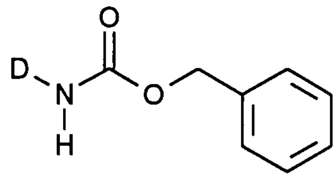

| Bsp.-Nr. | D | Ausbeute [% d.Th.] | Schmp.: [°C] | $R_f$ / Laufmittel (Verhältnis) |
|---|---|---|---|---|
| IV | 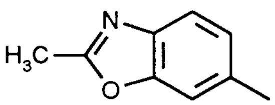 | 99 | 74 | 0,53 II (1:4) |
| V | 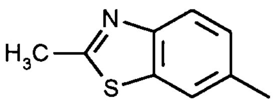 | 98 | 108 | 0,55 II (2:3) |
| VI | 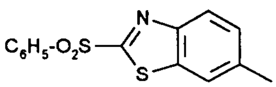 | 96 | 146 | 0,73 II (1:4) |
| VII | 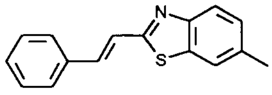 | 83 | 164 | 0,65 I (9:1) |

| Bsp.-Nr. | D | Ausbeute [% d.Th.] | Schmp.: [°C] | $R_f$ / Laufmittel (Verhältnis) |
|---|---|---|---|---|
| VIII | 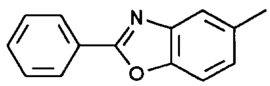 | 100 | - | 0,70 II (1:1) |
| IX | 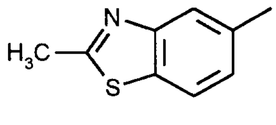 | 95 | - | 0,53 II (1:1) |
| X | 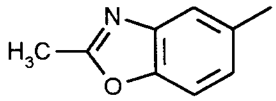 | 94 | - | 0,50 II (1:1) |
| XI | 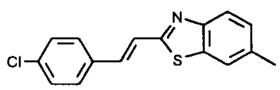 | 90 | 190 | 0,84 II (1:2) |

**Beispiel XII**

(5R)-3-(2-Benzylthio-benzo[4,5-d]thiazol-6-yl)-5-butyryloxy-methyl-oxazolidin-2-on

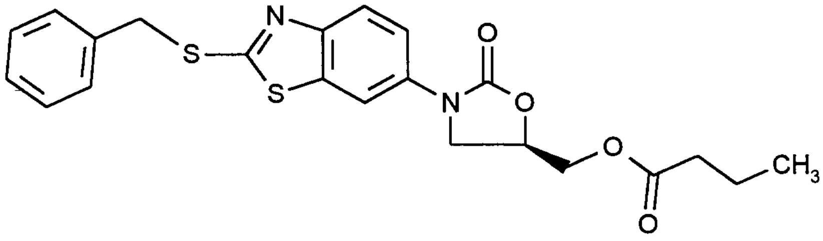

Eine Suspension von 333 mg (3,84 mmol) Lithiumbromid und 838 mg (3,84 mmol) Tributylphosphinoxid in 200 ml Xylol wird 1 h am Wasserabscheider gekocht. Dazu wird in der Siedehitze ein Gemisch von 8,0 ml (57,6 mmol) Triethylamin und 10,0 ml (70,40 mmol) (R)-Glycidylbutyrat getropft. Gleichzeitig wird innerhalb von 20 min eine Lösung von 21,0 g (63 mmol) der Verbindung aus Beispiel I in 200 ml Xylol zugetropft. Nach beendeter Zugabe wird noch 5 min unter Rückfluß nachgerührt. Man läßt auf Raumtemperatur abkühlen und dampft das Lösemittel im Vakuum ab. Nach Chromatographie des Rückstands an 0,5 kg Kieselgel (Toluol : Ethylacetat 4:1) erhält man 16,2 g (57%) der Titelverbindung als helle Kristalle.
Schmp.: 109°C
$R_f$ = 0,32 (Toluol : Ethylacetat 4:1)

MS(FAB) m/z = 443 $(M+H)^+$
$^1$H-NMR (200 MHz, $D_6$-DMSO): δ = 0,82 (t, J = 7 Hz, 3H, $\underline{CH_3}CH_2$); 1,5 (m, 2H, $CH_3\underline{CH_2}CH_2CO$); 2,30 (t, J = 7 Hz, 2H, $CH_3CH_2\underline{CH_2}CO$); 3,89 (dd, J = 7 Hz, 10 Hz, 1H, H-4 trans); 4,23 (dd, J = 9 Hz, 10 Hz, 1H, H-4 cis); 4,33 (m, 2H, $CH_2O$); 4,62 (s, 2H, $Ph\underline{CH_2}S$); 4,97 (m, 1H, H-5); 7,35 (m, 3H, Ph); 7,5 (m, 2H, Ph); 7,75 (dd, J = 1,5, 10 Hz, 1H, Benothiazol-H-5); 7,90 (d, J = 10 Hz, 1H, Benzothiazol-H-4); 8,15 (d, J = 1,5 Hz, 1H, Benzothiazol-H-7).

**Beispiel XIII**

(5R)-3-(2-Methylthio-benzo[4,5-d]thiazol-6-yl)-5-hydroxymethyl-oxazolidin-2-on

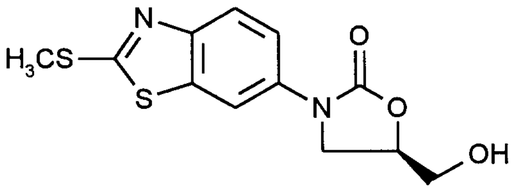

Eine auf -78°C gekühlte, gerührte Lösung von 28,90 g (87,64 mmol) 6-Benzyloxycarbonylamino-2-methylthio-benzo[4,5-d]thiazol (Beispiel III) und 1 mg 1,10-Phenanthrolinhydrat in 280 ml wasserfreiem THF wird bis zum Farbumschlag langsam mit 35,00 ml (87,64 mmol) einer 2,5 M Lösung von n-Butyllithium in n-Hexan vesetzt. Danach tropft man 12,40 ml (87,64 mmol) (R)-Glycidylbutyrat zu und erlaubt der Reaktionsmischung sich innerhalb von 16 h auf Raumtemperatur zu erwärmen. Danach werden innerhalb von 15 min 200 ml gesättigte wässrige $NH_4Cl$-Lösung zugetropft. Die Wasserphase wird mit 3 x 100 ml Ethylacetat extrahiert, die organischen Phasen werden vereinigt, mit 2 x 100 ml NaCl-Lösung gewaschen und über $MgSO_4$ getrocknet. Nach Abdampfen des Lösemittels im Vakuum, Verreiben des Rückstands mit Ether erhält man 17,25 g (67%) der Titelverbindung als farblose Kristalle.
Schmp.: 156°C
$R_f$= 0,24 (Toluol : Ethylacetat 1:4)
MS (DCI, $NH_3$) m/z = 297 $(M+H)^+$
$^1$H-NMR (200 MHz, $D_6$-DMSO) δ = 2,78 (s, 3H, $SCH_3$); 3,6 - 3,8 (m, 2H, $CH_2O$); 3,90 (dd, J = 7, 10 Hz, 1H, H-4 trans); 4,15 (dd, J = 10, 10 Hz, 1H, H-4 cis); 4,72 (m, 1H, H-5); 5,25 (t, J = 6 Hz, 1H, OH); 7,74 (dd, J = 1,5, 10 Hz, 1H, Benzothiazol H-5); 7,87 (d, J = 10 Hz, 1H, Benzothiazol H-4); 8,18 (d, J = 1,5 Hz, 1H, Benzothiazol H-7).

**Beispiel XIV**

(5R)-3-(2-Benzylthio-benzo[4,5-d]thiazol-6-yl)-5-hydroxymethyl-oxazolidin-2-on

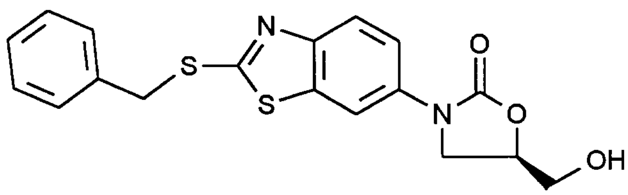

Eine Lösung von 16,15 g (36,49 mmol) der Verbindung aus Beispiel XII in 150 ml wasserfreiem Methanol wird mit 12 mg (0,367 mmol) Caesiumcarbonat versetzt und 18 h bei Raumtemperatur gerührt. Das Lösemittel wird im Vakuum abgedampft und der Rückstand wird mit 30 ml Ether verrührt. Der Niederschlag wird durch Filtration abgetrennt, mit 25 ml Wasser und 5 ml Ether gewaschen und im Hochvakuum getrocknet. Man erhält 13,01 g (96%) der Titelverbindung als helle Kristalle.
Schmp.: 145°C
$R_f$ = 0,06 (Toluol : Ethylacetat 7:3)
MS (DCI, $NH_3$) m/z = 373 $(M+H)^+$

$^1$H-NMR (200 MHz, $D_6$-DMSO) δ = 3,50 - 3,75 (m, 2H, $CH_2O$); 3,89 (dd, J = 7, 10 Hz, 1H, H-4 trans); 4,13 (dd, J = 10, 10 Hz, 1H, H-4 cis); 4,63 (s, 2H, $CH_2$); 4,70 (m, 1H, H-5); 5,25 (t, J = 6 Hz, 1H, OH); 7,30 (m, 3H, Ph); 7,50 (m, 2H,Ph); 7,77 (dd, J = 1,5, 10 Hz, 1H, Benzothiazol H-5); 7,89 (d, J = 10 Hz, 1H, Benzothiazol H-4); 8,18 (d, J = 1,5 Hz, 1H, Benzothiazol H-7).

In Analogie zu den Vorschriften der Beispiele XI - XIV werden die in Tabelle II aufgeführten Verbindungen hergestellt:

**Tabelle II:**

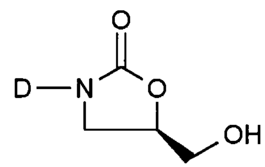

| Bsp.-Nr. | D | analog Herstellungs-methode (Reagenz) | Ausbeute [% d.Th.] | Schmp. [°C] | $R_f$ / Laufmittel (Verhältnis) | $[\alpha]_D^{20}$ (DMSO) | MS (FAB) m/z $(M+H)^+$ |
|---|---|---|---|---|---|---|---|
| XV | 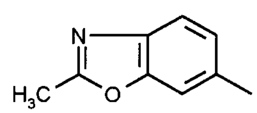 | XIII (n-BuLi) | 80 | 188 | 0,13, II (1:4) | | 249 |
| XVI | 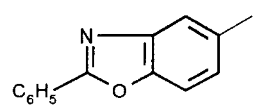 | XIII (n-BuLi) | 78 | 208 | 0,10, II (1:1) | -44.1 (c=1) | 310 [a)] |
| XVII | 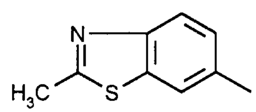 | XIII (n-BuLi) | 71 | 164 | 0,12, II (1:4) | | 265 |
| XVIII | 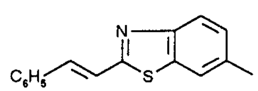 | XIII (n-BuLi) | 76 | 188 | 0,52, I (9:1) | | 352 [a)] |

| Bsp.-Nr. | D | analog Herstellungs-methode (Reagenz) | Ausbeute [% d.Th.] | Schmp. [°C] | $R_f$ / Laufmittel (Verhältnis) | $[\alpha]_D^{20}$ (DMSO) | MS (FAB) m/z $(M+H)^+$ |
|---|---|---|---|---|---|---|---|
| XIX | 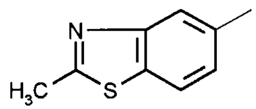 | XIII (n-BuLi) | 57 | | 0,05, II (1:1) | | - |
| XX | 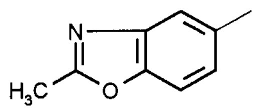 | XIII (n-Buli) | 68 | | 0,47, II (1:1) | -52.3 (c=1) | 248 [a)] |
| XXI | 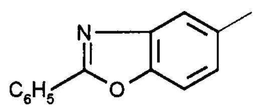 | XIII (n-BuLi) | 78 | 208 | 0,10, II (1:1) | -44,2 (c=1) | 310 [a)] |
| XXII | 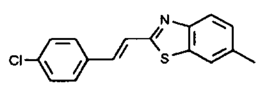 | XIII (n-BuLi) | 72 | 203 | 0,18, II (1:2) | - | 387[b)] |

a) MS (EI) m/z = $M^+$
b) MS(DCI/$NH_3$) m/z = $(M+H)^+$

**Beispiel XXIII**

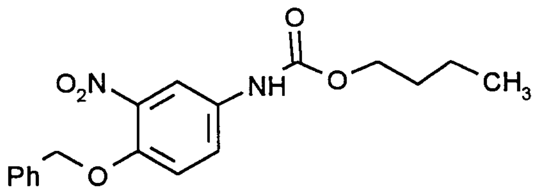

4-Benzyloxy-1-Butyloxycarbonylamino-3-nitrobenzol

Zu einer Lösung aus 24,3 g 3-Nitro-4-Benzyloxybenzoesäure (J. Med. Chem. 1967 (10) 462), 8,7 g (86,4 mmol) Triethylamin in 200 ml Aceton werden bei 0°C 12,4 ml (93,6 mol) Chlorameisensäurebutylester in 80 ml Aceton gegeben. Man rührt weiter 1 Stunde bei -5°C, tropft dann 7,0 g (108 mmol) Natriumazid in 100 ml Wasser zu und rührt 2 Stunden bei 0°C. Anschließend gibt man den Ansatz auf 800 ml Eiswasser, saugt den ausgefallenen Niederschlag ab und trägt diesen anschließend in 300 ml siedendes n-Butanol ein.
Nach beendeter Zugabe wird weitere 10 Minuten unter Rückfluß gerührt, abgekühlt und der ausgefallene Niederschlag abfiltriert.
Ausbeute: 23,0 g (75 %)
Schmelzpunkt: 121 - 122°C
$^1$H-NMR (250 MHz, $D_6$-DMSO): δ = 9,8 (bs, 1H), 8,12 (d, 1H), 7,62 (dd, 1H), 7,20-7,45 (m, 6H), 5,25 (s, 2H), 4,10 (t, 2H), 1,70 (m, 2H), 1,40 (m, 2H), 0,48 (t, 3H).

In Analogie zur Vorschrift des Beispiels XXIII werden die in der Tabelle III aufgeführten Verbindungen hergestellt:

## Tabelle III

| Bsp.-Nr. | Verbindung | Ausbeute (% d.Th.) | Schmp. (°C) | $R_f$ (Laufmittel, Verhältnis) | MS (DCl, $NH_3$) m/z $(M+H)^+$ |
|---|---|---|---|---|---|
| XXIV | 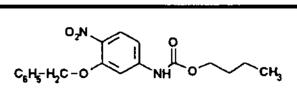 | 63 | 133 | 0,51 (VII, 95:5) | 345 |

**Beispiel XXV**

(5R)-3-(4-Benzyloxy-3-nitrophenyl)-5-(hydroxymethyl)-oxazolidin-2-on

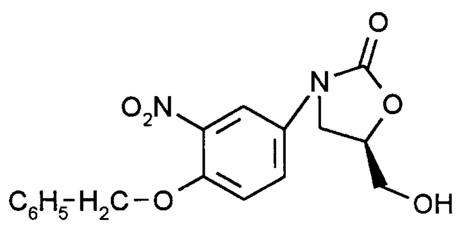

23,0 g (66,7 mmol) der Verbindung aus Beispiel XXIII werden in 200 ml THF gelöst und auf 0°C gekühlt. Nun werden langsam ca. 68 ml 1,0 M LiHMDS-Lösung in THF zugetropft. Anschließend werden 9,5 ml (68 mmol) (R)-Glycidylbutyrat zugetropft. Man läßt auf Raumtemperatur kommen, versetzt mit gesättigter Ammoniumchloridlösung und zieht im Vakuum das THF ab. Der entstandene Niederschlag wird abgesaugt, mit Wasser und Ether gewaschen und im Hochvakuum getrocknet.
Ausbeute: 20,85 g (91 %)
Schmelzpunkt: 128 - 130°C
$R_f$ (II, 1:1) = 0,21
MS (FAB): m/z = 345 $M^{\oplus}$
In Analogie zur Vorschrift des Beispiels XXV werden die in der Tabelle IV aufgeführten Verbindungen hergestellt:

**Tabelle IV**

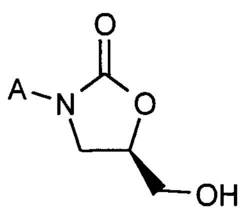

| Bsp.-Nr. | A | Ausbeute (% d.Th.) | Schmp. (°C) | $R_f$ (Laufmittel, Verhältnis | (α)20$_D$ (DMSO) | MS (FAB) m/z ($M^+$+H) |
|---|---|---|---|---|---|---|
| XXVI | 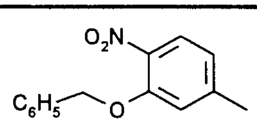 | 73 | 137-139 | 0,28 (II, 1:1) | -38,1 (c=0,985) | 345 |

**Beispiel XXVII**

(5R)-3-(4-Benzyloxy-3-nitrophenyl)-5-(methylsulfonyloxymethyl)-oxazolidin-2-on

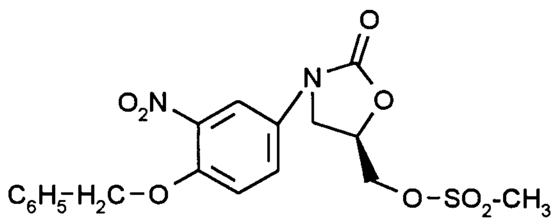

Eine auf 0°C gekühlte Lösung von 71,5 g (208 mmol) der Verbindung aus Beispiel XXV und 35 ml (250 mmol) Triethylamin in 650 ml wasserfreiem THF wird langsam mit 23,6 ml (230 mmol) Methansulfonsäurechlorid versetzt. Der entstandene Niederschlag wird abgesaugt, mit Wasser und Toluol gewaschen und im Hochvakuum getrocknet.
Ausbeute: 65,8 g (75 %)
Schmelzpunkt: 149 - 150°C
$R_f$ (VII, 5:1) = 0,36
MS (FAB): m/z = 423 $M^{\oplus}$
$^1$H-NMR ([$D_6$]DMSO): δ = 8,12 (d, J = 1 Hz, 1H Ph); 7,75 (dd, J = 6 Hz, J = 1 Hz, 1H, Ph); 7,35-7,55 (m, 6H, Ph); 5,30 (s, 2H, $CH_2$); 4,40-4,60 (m, 2H, $CH_2O$); 4,22 (t, J = 9 Hz, 1H, 4-H); 3,85 (dd, J = 9 Hz, J = 5 Hz, 1H, 4-H); 3,25 (s, 3H, $SO_2CH_3$).

In Analogie zur Vorschrift des Beispiels XXVII werden die in der Tabelle V aufgeführten Verbindungen hergestellt:

## Tabelle V

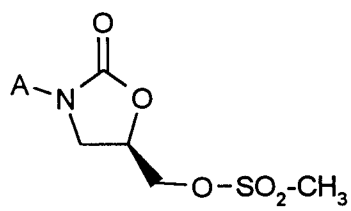

| Bsp.-Nr. | A | Ausbeute (% d.Th.) | Schmp. (°C) | $R_f$ (Laufmittel, Verhältnis | $(\alpha)20_D$ (DMSO) | MS (FAB) m/z ($M^+$+H) |
|---|---|---|---|---|---|---|
| XXVIII | 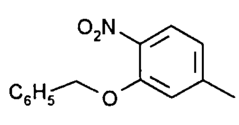 | 92 | 140-142 | 0,34 (VII, 5:1) | -48,8 (c=1,01) | 423 |

**Beispiel XXIX**

(5R)-3-(4-Benzyloxy-3-nitrophenyl)-5-(azidomethyl)-oxazolidin-2-on

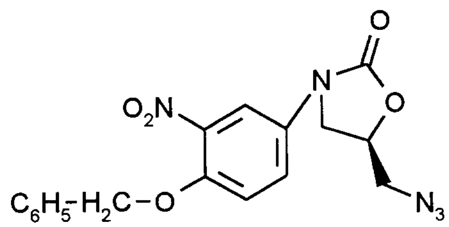

Eine Lösung von 25,7 g (60,8 mmol) der Verbindung aus Beispiel XXVII in 200 ml wasserfreiem DMF wird mit 4,4 ml (66,9 mmol) Natriumazid versetzt und 12 Stunden bei 70°C gerührt. Man läßt auf Raumtemperatur abkühlen und rührt 200 ml Eiswasser ein. Der entstandene Niederschlag wird abfiltriert, mit Wasser und Petrolether gewaschen und im Vakuum getrocknet.
Ausbeute: 21,4 g (95 %)
Schmelzpunkt: 158 - 160°C
$R_f$ (VII, 5:1) = 0,48
MS (EI): m/z = 370 $M^{\oplus}$
$^1$H-NMR ([$D_6$]DMSO): δ = 8,05 (d, 1H, J = 8 Hz, Ph); 7,25-7,50 (m, 7H, Ph); 5,30 (s, 2H, $CH_2$); 4,85-5,05 (m, 1H, 5-H); 4,23 (t, J = 9 Hz, 1H, 4-H); 3,55-3,90 (m, 3H, 4-H, $CH_2N_3$).

In Analogie zur Vorschrift des Beispiels XXIX werden die in der Tabelle VII aufgeführten Verbindungen hergestellt:

**Tabelle VII**

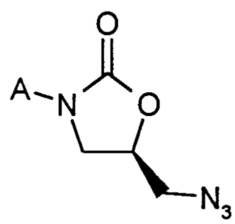

| Bsp.-Nr. | A | Ausbeute (% d.Th.) | Schmp. (°C) | $R_f$ (Laufmittel, Verhältnis | (α)20$_D$ (DMSO) | MS (FAB) m/z ($M^+$+H) |
|---|---|---|---|---|---|---|
| XXX | 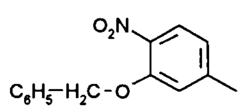 | 92 | 138-140 | 0,26 (VII, 5:1) | -119,4 (c=1,1) | 370 |

**Beispiel XXXI**

(5S)-3-(4-Benzyloxy-3-nitrophenyl)-5-(aminomethyl)-oxazolidin-2-on

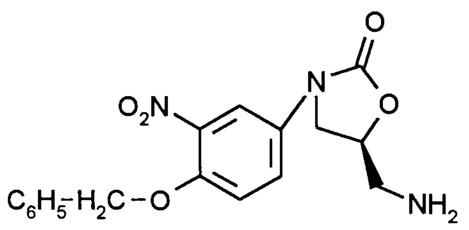

Eine Lösung von 53,1 g (144 mmol) der Verbindung aus Beispiel XXIX in 160 ml 1,2-Dimethoxyethan wird auf 50°C erwärmt. Man tropft langsam 20,4 ml (173 mmol) Trimethylphosphit zu (Gasentwicklung) und rührt nach beendeter Zugabe 2 Stunden bei 90°C. Nun tropft man 36 ml 6 N HCl zu und rührt nochmals 22 Stunden bei 90°C. Man läßt auf Raumtemperatur abkühlen, gibt 810 ml 0,1 N HCl hinzu, wäscht die wäßrige Phase mit Ether (3 x 320 ml) und stellt anschließend auf pH = 9. Die wäßrige Phase wird mit Essigester (3 x 650 ml) extrahiert (2 x 300 ml), die vereinigten organischen Phasen mit ges. NaCl-Lösung gewaschen (1 x 100 ml) und getrocknet ($Na_2SO_4$). Die Lösemittel werden im Vakuum abgezogen und im Hochvakuum getrocknet.
Ausbeute: 47,2 g (96 %)
Schmelzpunkt: 135 - 136°C
$R_f$ (VIII, 85:10:5) = 0,05
MS (EI): m/z = 344 $M^{\oplus}$
$^1$H-NMR ([$D_6$]DMSO): δ = 8,30-9,10 (bs, 3H, $NH_3$); 8,15 (d, 1H Ph); 7,3-7,8 (m, 7H, Ph); 5,30 (m, 2H, $CH_2$); 4,90-5,10 (m, 1H, 4-H); 4,20 (m, 1H 5-H); 4,00 (m, 1H, 5-H); 3,10-3,40 (m, 2H, $CH_2N$).

In Analogie zur Vorschrift des Beispiels XXXI werden die in der Tabelle VIII aufgeführten Verbindungen hergestellt:

## Tabelle VIII

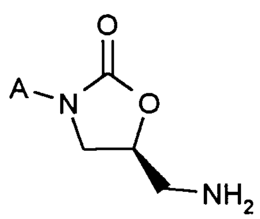

| Bsp.-Nr. | A | Ausbeute (% d.Th.) | Schmp. (°C) | $R_f$ (Laufmittel, Verhältnis | MS (FAB) m/z ($M^+$) |
|---|---|---|---|---|---|
| XXXII | 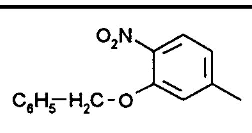 | 89 | 132-134 | 0,08 (VIII, 85:10:5) | 344 |

**Beispiel XXXIII**

(5S)-3-(4-Benzyloxy-3-nitrophenyl)-5-(acetylaminomethyl)-oxazolidin-2-on

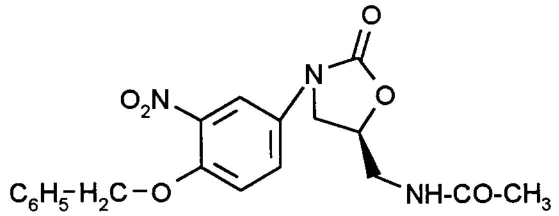

Zu einer auf 0°C gekühlten Lösung von 47,2 g (137 mmol) der Verbindung aus Beispiel XXXI und 29,4 ml (212 mmol) Triethylamin in 500 ml wasserfreiem THF tropft man langsam 14,6 ml, (205 mmol) Acetylchlorid. Man rührt 2 Stunden bei 0°C nach und gibt auf Eiswasser. Der Niederschlag wird abgesaugt, mit Wasser und Ether gewaschen, und im Hochvakuum über $P_2O_5$ getrocknet.
Ausbeute: 48,9 g (93 %)
Schmelzpunkt: 177 - 178°C
$R_f$ (VII, 1:1) = 0,51
MS (FAB): m/z = 386 $(M+H)^+$
$^1$H-NMR ([$D_6$]DMSO): δ = 8,24 (t, J = 4 Hz, 1H NH); 8,10 (d, J = 1 Hz, 1H, Ph); 7,75 (dd, J = 6 Hz, J = 1 Hz, 1H, Ph); 7,20-7,50 (m, 6H, Ph); 5,30 (s, 2H, $CH_2$); 4,70-4,80 (m, 1H, 5-H); 4,15 (t, J = 9 Hz, 1H, 4-H); 3,70 (dd, J = 9 Hz, J = 5 Hz, 1H, H-4); 3,35-3,50 (m, 5H, $CH_2N$, $NCH_3$); 1,83 (s, 3H, $COCH_3$).

In Analogie zur Vorschrift des Beispiels XXXIII werden die in der Tabelle IX aufgeführten Verbindungen hergestellt:

## Tabelle IX

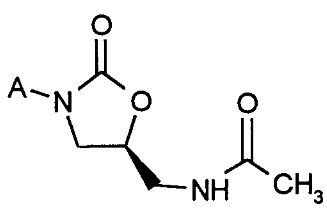

| Bsp.-Nr. | A | Ausbeute (% d.Th.) | Schmp. (°C) | $R_f$ (Laufmittel, Verhältnis | (α)20$_D$ (DMSO) | MS (FAB) m/z (M+H) |
|---|---|---|---|---|---|---|
| XXXIV | 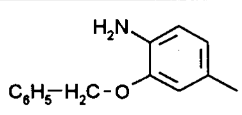 | 86 | 155-156 | 0,62 (VII, 1:1) | -23,6 (c=1,05) | 386 |

**Beispiel XXXV**

(5S)-3-(3-Amino-4-hydroxyphenyl)-5-(aminomethyl)-oxazolidin-2-on

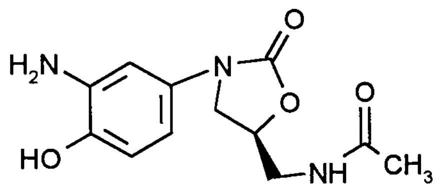

3,58 g (9,28 mmol) der Verbindung aus Beispiel XXXIII und 350 mg Pd-C (10 %) werden in 100 ml Methanol und 100 ml THF 3 Stunden unter Wasserstoff (1 atm) gerührt. Es wird vom Katalysator abfiltriert, das Lösemittel abgezogen und getrocknet.

Ausbeute: 2,5 g (quant.)
$R_f$ (VII, 1:1) = 0,42
MS (CI): m/z = 265 ($M^{+}$)
$[\alpha]_D^{20}$ = -110,45 (c=1,0, DMSO)
$^1$H-NMR ([$D_6$]DMSO): δ = 9,0-9,5 (bs, 1H, OH); 8,20 (t, J = 4 Hz, 1H, NHCO); 7,05 (bs, 1H, Ph); 6,55 (bs, 2H Ph); 4,55-4,70 (m, 1H, 5-H); 4,30-4,52 (bs, 2H, $NH_2$); 3,95 (t, J = 6 Hz, 1H 4-H); 3,60 (dd, J = 7 Hz, J = 4 Hz, 1H, 4-H); 3,40 (t, J = 4 Hz, 2H, $CH_2N$); 1,73 (s, 3H $COCH_3$).

Wie für Beispiel XXXV beschrieben erhält man aus den entsprechenden Ausgangsverbindungen die in Tabelle X aufgeführten Verbindungen:

## Tabelle X

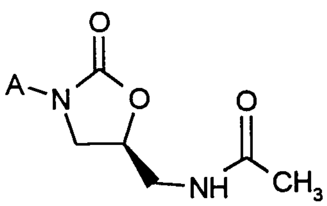

| Bsp.-Nr. | A | Ausbeute (% d.Th.) | Schmp. (°C) | $R_f$ (Laufmittel, Verhältnis | $(\alpha)20_D$ (DMSO) | MS (CDI, $NH_3$) m/z $(M+H)^+$ |
|---|---|---|---|---|---|---|
| XXXVI | 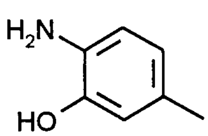 | quant. | 221-222 | 0,31 (VII, 1:1) | -19,89 (c=1,0) | 265 |

**Herstellungsbeispiele**

**Beispiel 1**

(5R)-3-(2-Methylthio-benzo[4,5-d]thiazol-6-yl)-5-methansulfonyloxy-methyloxazolidin-2-on

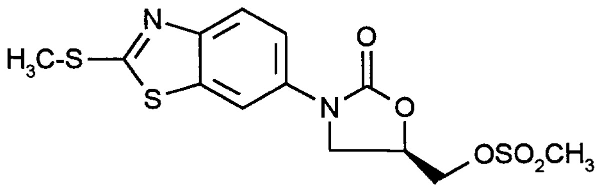

Eine auf 0°C gekühlte, gerührte Lösung von 13,63 g (45,99 mmol) der Verbindung aus Beispiel XIII und 9,00 ml (64,39 mmol) Triethylamin in 90 ml wasserfreiem Dichlormethan wird langsam mit 4,60 ml (59,78 mmol) Methansulfonsäurechlorid versetzt. Man rührt 15 min. bei 0-5°C nach und rührt das Gemisch in 100 ml Eiswasser ein. Die organische Phase wird abgetrennt, mit 20 ml gesättigter $NaHCO_3$-Lösung und 20 ml Eiswasser gewaschen und über $MgSO_4$ getrocknet. Das Lösemittel wird im Vakuum eingedampft und der Rückstand mit 50 ml Ether verrührt, abgesaugt und im Hochvakuum getrocknet. Man erhält 14,17 g (82%) der Titelverbindung als farblose Kristalle.
Schmp.: 106°C
$R_f$ = 0,41 (Dichlormethan : Methanol 95:5)
MS (DCI, $NH_3$) m/z = 375 $(M+H)^+$
$^1$H-NMR (250 MHz, $D_6$-DMSO) δ = 2,80 (s, 3H, $CH_3S$); 3,28 (s, 3H, $OSO_2CH_3$); 3,90 (dd, J = 7, 10 Hz, 1H, H-4 trans); 4,27 (dd, J = 10, 10 Hz, 1H, H-4 cis); 4,55 (m, 2H, $CH_2O$); 5,04 (m, 1H, H-5); 7,72 (dd, J = 1,5, 10 Hz, 1H, Benzothiazol H-5); 7,88 (d, J = 10 Hz, 1H, Benzothiazol H-4); 8,19 (d, J= 1,5 Hz, 1H Benzothiazol H-7).

Wie für Beispiel 1 beschrieben erhält man aus den entsprechenden Alkoholen die folgenden Methansulfonate (Tabelle 1):

**Tabelle 1:**

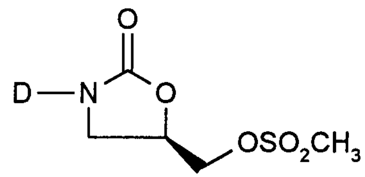

| Bsp.-Nr. | D | Ausbeute [% d.Th.] | Schmp. [°C] | $R_f$ / Laufmittel (Verhältnis) | $[\alpha]_D^{20}$ (DMSO) | MS (FAB) m/z $(M+H)^+$ |
|---|---|---|---|---|---|---|
| 2 | 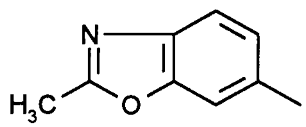 | 69 | 159 | 0,26, I (95:5) | | 327 |
| 3 | 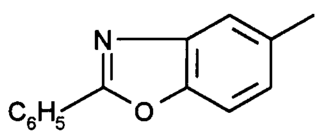 | 94 | | 0,20, II (1:1) | -55,5° (c=1) | 389 |
| 4 | 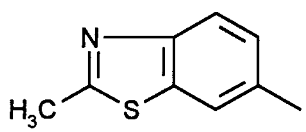 | 73 | 162 | 0,28, I (95:5) | | 343 |

| Bsp.-Nr. | D | Ausbeute [% d.Th.] | Schmp. [°C] | $R_f$ / Laufmittel (Verhältnis) | $[\alpha]_D^{20}$ (DMSO) | MS (FAB) m/z $(M+H)^+$ |
|---|---|---|---|---|---|---|
| 5 | 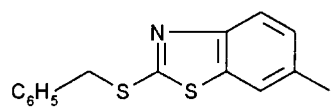 | 87 | 131 | 0,47, I (95:5) | | 451 |
| 6 | 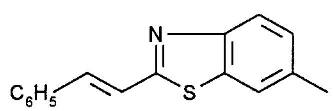 | 75 | 146 | 0,53, IV | | 431 |
| 7 | 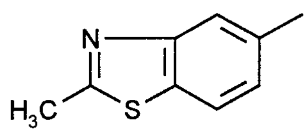 | 59 | | 0,29, II (1:5) | | |
| 8 | 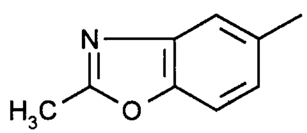 | 88 | | 0,58 b) (5:1) | -52,3° (c=1) | 327 |

b) Toluol : Ethanol

**Beispiel 9**

(5R)-3-(2-Methylthio-benzo[4,5-d]thiazol-6-yl)-5-azidomethyl-oxazolidin-2-on

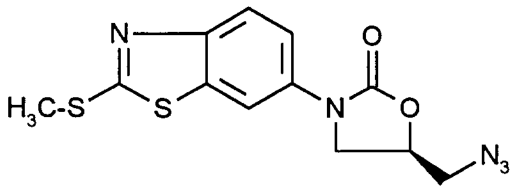

Eine gerührte Lösung von 14,16 g (37,81 mmol) der Verbindung aus Beispiel 1 in 50 ml wasserfreiem DMF wird mit 3,20 g (49,16 mmol) Natriumazid versetzt und 3 h bei 70°C gerührt. Man läßt auf Raumtemperatur abkühlen und rührt in 100 ml Eiswasser ein. Der entstandene Niederschlag wird durch Filtration abgetrennt, mit 50 ml Wasser und 20 ml Petrolether gewaschen und an der Luft getrocknet. Man erhält 11,60 g (95%) der Titelverbindung als helle Kristalle.

Schmp.: 136°C

$R_f$ = 0,59 (Dichlormethan : Methanol 95:5)

MS (DCI, $NH_3$) m/z = 322 $(M+H)^+$

$^1$H-NMR (250 MHz, $D_6$-DMSO) δ = 2,79 (s, 3H, $CH_3S$); 3,76 (m, 2H, $CH_2N_3$); 3,87 (dd, J = 6,9 Hz, 1H, H-4 trans); 4,21 (dd, J = 9,9 Hz, 1H, H-4 cis); 4,92 (m, 1H, H-5); 7,73 (dd, J = 1, 9 Hz, 1H, Benzothiazol H-5); 7,87 (d, J = 9 Hz, 1H, Benzothiazol H-4); 8,18 (d, J = 1 Hz, 1H, Benzothiazol H-7).

Wie für Beispiel 9 beschrieben erhält man aus den entsprechenden Methansulfonaten die folgenden Azide (Tabelle 2):

**Tabelle 2:**

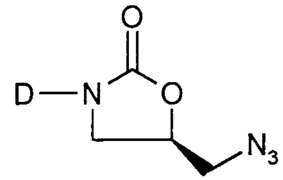

| Bsp.-Nr. | D | Ausbeute [% d.Th.] | Schmp. [°C] | $R_f$ / Laufmittel (Verhältnis) | $[\alpha]_D^{20}$ (DMSO) | MS (FAB) m/z $(M+H)^+$ |
|---|---|---|---|---|---|---|
| 10 | 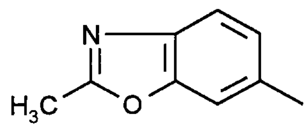 | 94 | 111 | 0,42, I (95:5) | | 274 |
| 11 | 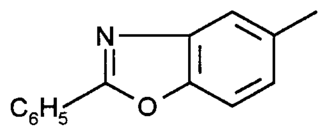 | 97 | | 0,34, II (1:1) | -128.5° (c=1) | 335 [a)] |
| 12 | 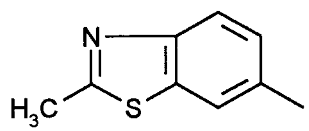 | 90 | 79 | 0,51, I (95:5) | | 280 |

| Bsp.-Nr. | D | Ausbeute [% d.Th.] | Schmp. [°C] | $R_f$ / Laufmittel (Verhältnis) | $[\alpha]_D^{20}$ (DMSO) | MS (FAB) m/z $(M+H)^+$ |
|---|---|---|---|---|---|---|
| 13 | 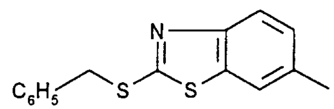 | 97 | 110 | 0,34, II (4:1) | | 398 |
| 14 | 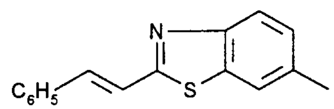 | 90 | 144 | 0,48, IV | | 377 [a)] |
| 15 | 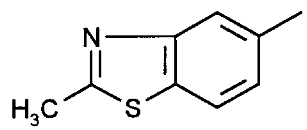 | 98 | | 0,43, II (1:1) | | |
| 16 | 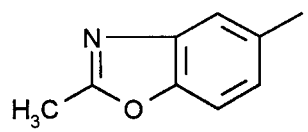 | 79 | | 0,45 | -129.7° (C=0,5) | 273 [a)] |

a) MS (EI) m/z = $(M)^+$

**Beispiel 17**

(5S)-3-(2-Methylthio-benzo[4,5-d]thiazol-6-yl)-5-aminomethyl-oxazolidin-2-on Hydrochlorid

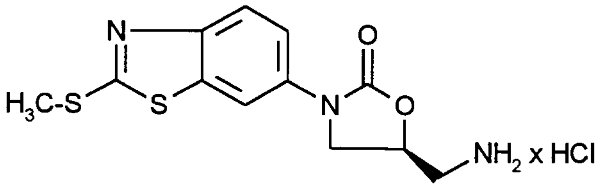

Eine gerührte Lösung von 11,58 g (36,03 mmol) der Verbindung aus Beispiel 9 in 25 ml 1,2-Dimethoxyethan wird auf 50°C erwärmt. Man tropft langsam 5,00 ml (43,24 mmol) Trimethylphosphit zu (Gasentwicklung) und rührt nach beendeter Zugabe nach 2 h bei 90°C nach. Nun tropft man 7,2 ml 6 N HCl zu und rührt nochmals 3 h bei 90°C nach. Man läßt auf Raumtemperatur abkühlen, trennt den Niederschlag durch Filtration ab, wäscht mit 2 x 10 ml 1,2-Dimethoxyethan und trocknet im Hochvakuum über NaOH. Man erhält 9,94 g (83%) der Titelverbindung als farblosen Feststoff.
Schmp.: 110°C
$R_f$ = 0,09 (Acetonitril : Wasser 9:1)
$^1$H-NMR (250 MHz, $D_6$-DMSO) δ = 2,80 (s, 3H, $CH_3S$); 3,28 (m, 2H, $\underline{CH_2}NH_2$); 3,98 (dd, J 7, 9 Hz, 1H, H-4 trans); 4,28 (dd, J = 9, 9 Hz, 1H, H-4 cis); 5,02 (m, 1H, H-5); 7,70 (dd, J = 1, 10 Hz, 1H, Benzothiazol H-5); 7,89 (d, J = 10 Hz, 1H, Benzothiazol H-4); 8,18 (d, J = 1 Hz, 1H, Benzothiazol H-7).

Wie für Beispiel 17 beschrieben erhält man durch Umsetzung der entsprechenden Azide die folgenden Produkte (Tabelle 3):

**Tabelle 3:**

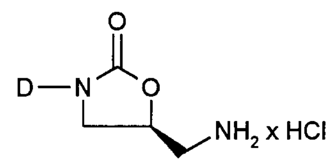

| Bsp.-Nr. | D | Ausbeute [% d.Th.] | Schmp. [°C] | $R_f$ / Laufmittel (Verhältnis) | $[\alpha]_D^{20}$ (DMSO) | MS (FAB) m/z $(M-Cl)^+$ |
|---|---|---|---|---|---|---|
| 18 | 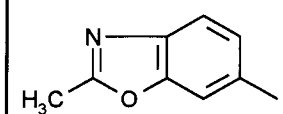 | 83 | | 0,15, III (9:1) | | 248 |
| 19 | 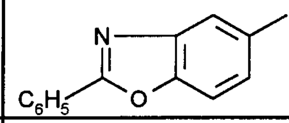 | 80 | >250 | 0,08, I (10:1) | -38,5° (c=1,0) | 309 [a)] |
| 20 | 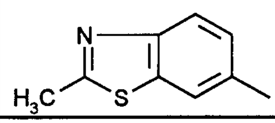 | 94 | | 0,16, III (9:1) | | 264 |
| 21 | 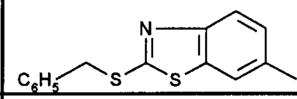 | 45 | 257 | 0,25, III (9:1) | | 372 |
| 22 | 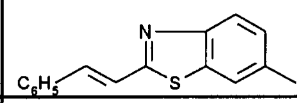 | 94 | 303 | 0,19, III (9:1) | | 351 [a)] |
| 23 | 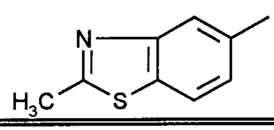 | 43 | | 0,12, I (10:1) | | |

a) MS (EI) m/z = $(M-HCl)^+$

**Beispiel 24**

Methode A

(5S)-3-(2-Methylthio-benzo[4,5-d]thiazol-6-yl))-5-acetylaminomethyl-oxazolidin-2-on

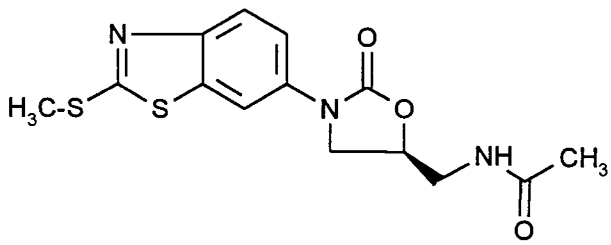

Eine gerührte Lösung von 9,35 g (28,17 mmol) der Verbindung aus Beispiel 17 in 80 ml THF wird mit einer Lösung von 1,24 g (30,99 mmol) Natriumhydroxid in 8 ml Wasser versetzt. Dazu tropft man bei 0-5°C langsam 2,90 ml (30,99 mmol) Acetanhydrid in 3 ml THF und hält pH = 9 durch gleichzeitige Zugabe einer 5 N wäßrigen NaOH-Lösung. Man rührt 1 h bei 0°C nach und dampft das Lösemittel im Vakuum ab. Der Rückstand wird mit 2 x 20 ml Wasser gut verrührt, abgetrennt und im Hochvakuum über Sicapent getrocknet. Nach Umkristallisation aus 2-Propanol erhält man 7,88 g (83%) der Titelverbindung als farblose Kristalle.
Schmp.: 136°C
$R_f$ = 0,15 (Dichlormethan : Methanol 95:5)
MS (DCI, $NH_3$) m/z = 338 $(M+H)^+$
$^1$H-NMR (200 MHz, $D_6$-DMSO) δ = 1,84 (s, 3H, $COCH_3$); 2,79 (s, 3H, $CH_3S$); 3,42 (t, J = 6,5 Hz, 2H $CH_2N$); 3,81 (dd, J = 7, 10 HZ, 1H, H-4 trans); 4,19 (dd, J = 10 Hz, 1H, H-4 cis); 4,75 (m, 1H, H-5); 7,72 (dd, J = 1, 10 Hz, 1H, Benzothiazol H-5); 7,85 (d, J = 10 Hz, 1H, Benzothiazol H-4); 8,15 (d, J = 1 Hz, 1H, Benzothiazol H-7); 8,28 (bt, J = 5 Hz, 1H, NH).

**Beispiel 25**

Methode B

(5S)-3-(2-Methyl-benzo[4,5-d]oxazol-6-yl)-5-acetamino-methyl-oxazolidin-2-on

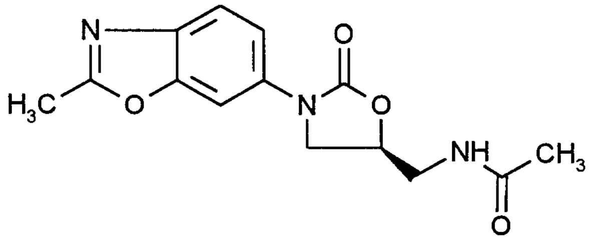

Zu einer gerührten, auf 0°C gekühlten Lösung von 13,20 g (48,51 mmol) der Verbindung aus Beispiel 18 und 16,90 ml (121,25 mmol) Triethylamin in 132 ml wasserfreiem Dichlormethan tropft man langsam 4,61 ml (67,43 mmol) Acetylchlorid. Man rührt 2 h bei 0°C nach, verdünnt mit 200 ml Wasser und 150 ml Dichlormethan, trennt die organische Phase ab, extrahiert die wässrige Phase mit 20 ml Dichlormethan und trocknet die vereinigten organischen Extrakte über $MgSO_4$. Nach Abdampfen des Lösemittels im Vakuum und Verreiben des Rückstands mit 150 ml Ether erhalt man 11,66 g (83%) der Titelverbindung als farblose Kristalle.
Schmp.: 181°C
$R_f$ = 0,40 (I 9:1)
MS (DCI, $NH_3$): m/z = 290 $(M+H)^+$

$^1H$-NMR (300 MHz, $D_6$-DMSO): $\delta$ = 1,83 (s, 3H, $COCH_3$); 2,60 (s, 2H, $CH_3$); 3,42 (t, J = 7 Hz, 2H, $CH_2N$); 3,82 (dd, J = 7,9 Hz, 1H, H-4 trans); 4,19 (dd, J = 10, 10 Hz, 1H, H-4 cis); 4,75 (m, 1H, H-5); 7,47 (dd, J = 1, 9 Hz, 1H, Benzoxazol H-5); 7,64 (d, J = 9 Hz, 1H, Benzoxazol H-6); 7,89 (d, J = 1 Hz, 1H, Benzoxazol H-7)); 8,23 (m, 1H, NH).

Wie für Beispiel 24 und 25 beschrieben erhält man durch Acylierung der entsprechenden Amine die folgenden Produkte (Tabelle 4):

**Tabelle 4:**

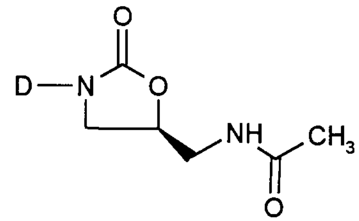

| Bsp.-Nr. | D | analog Herstellungs-methode | Ausbeute [% d.Th.] | Schmp. [°C] | $R_f$ / Laufmittel (Verhältnis) | $[\alpha]_D^{20}$ (DMSO) | MS (FAB) m/z $(M+H)^+$ |
|---|---|---|---|---|---|---|---|
| 26 | 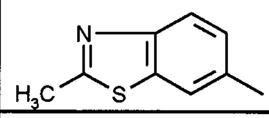 | A | 69 | 133 | 0,21, I (95:5) | | 306 |
| 27 | 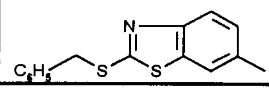 | A | 90 | 159 | 0,56, III (9:1) | | 414 |
| 28 | 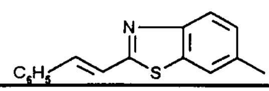 | A | 86 | 213 | 0,35, I (9:1) | | 393 [a)] |
| 29 | 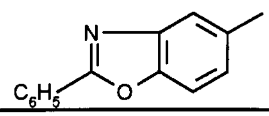 | B | 22 | 225 | 0,29, I (10:1) | -20,4° (c=1) | 351 [a)] |
| 30 | 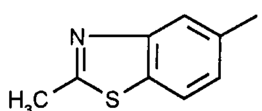 | B | 87 | 167 | 0,74, I (5:1) | | |

a) MS (EI) m/z = $(M)^+$

**Beispiel 31**

(5S)-3-(2,3-Dimethylbenzo[4,5-d]-thiazol-6-yl)-5-acetylaminomethyl-oxazolidin-2-on Jodid

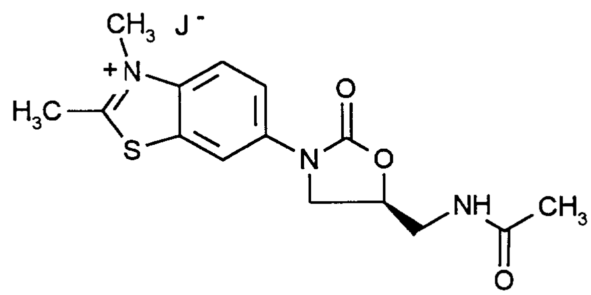

Eine gerührte Lösung von 180 mg (0,59 mmol) der Verbindung aus Beispiel 26 in 1,5 ml wasserfreiem Acetonitril wird mit 0,35 ml (5,05 mmol) Iodmethan versetzt und 5 h bei 60°C gerührt, wobei ein heller Niederschlag entsteht. Man gibt 40 ml Ether zu, rührt für 10 min gut durch, trennt den Niederschlag durch Filtration ab, wäscht mit 5 ml Ether und trocknet im Hochvakuum. Man erhält 148 mg (56%) der Titelverbindung als helle Kristalle.
Schmp.: 161°C (Zers.)
$R_f$ = 0,06 (Acetonitril / Wasser 4:1)
MS (FAB): 320 ($M^+$), freies Kation
$^1$H-NMR (250 MHz, $D_6$-DMSO): δ = 1,85 (s, 3H, $COCH_3$); 3,13 (s, 3H, $CH_3$); 3,47 (m, 2H, $CH_2N$); 3,87 (dd, J = 7, 9 Hz, 1H, H-4 trans); 4,20 (s, 3H, $CH_3N$); 4,23 (dd, J = 9, 9 Hz, 1H; H-4 cis); 4,83 (m, 1H, H-5); 8,10 (dd, J = 1,5, 10 Hz, 1H, Benzothiazol H-5); 8,30 (m, 2H, NH, Benzothiazol H-4); 8,60 (d, J = 1,5 Hz, 1H, Benzothiazol H-7).

**Beispiel 32**

(5S)-3-(2-Methylsulfinyl-benzo[4,5-d]-thiazol-6-yl)-5-acetylaminomethyl-oxazolidin-2-on

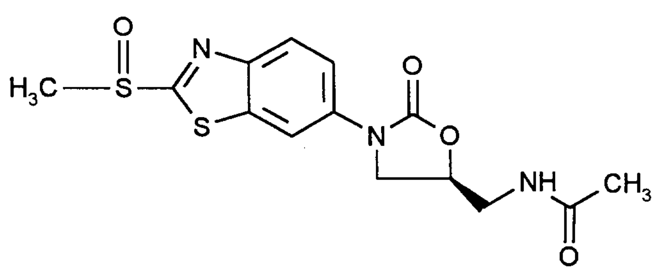

Eine gerührte, auf 0°C gekühlte Lösung von 500 mg (1,48 mmol) der Verbindung aus Beispiel 24 in einem Gemisch aus 6 ml Dichlormethan und 9 ml Chloroform wird mit 351 mg (1,63 mmol) 80%iger m-Chlorperbenzoesäure versetzt und 3 h bei 0°C gerührt. Zur Aufarbeitung wird das Gemisch in 20 ml gesättigter $NaHCO_3$-Lösung eingerührt, die organische Phase wird abgetrennt und über $MgSO_4$ getrocknet. Nach Abdampfen des Lösemittels im Vakuum und Verreiben des Rückstandes mit 5 ml Ether erhält man 224 mg (43%) der Titelverbindung als farblose Kristalle.
Schmp.: 228°C
$R_f$ = 0,16 (Dichlormethan : Methanol 95:5)
MS (FAB): m/z = 354 $(M+H)^+$
$^1$H-NMR (250 MHz, $D_6$-DMSO): δ = 1,85 (s, 3H, $COCH_3$); 3,10 (s, 3H, $CH_3SO$); 3,47 (t, J = 6 Hz, 2H, $CH_2N$); 3,87 (dd, J = 7, 10 Hz, 1H, H-4 trans); 4,23 (dd, J = 10, 10 Hz, 1H, H-4 cis); 4,80 (m, 1H, H-5); 7,94 (m, 1H, Benzothiazol H-5); 8,12 (d, J = 10 Hz, 1H, Benzothiazol H-4); 8,27 (m, 1H, NH); 8,38 (d, J = 1,5 Hz, 1H, Benzothiazol H-7).

**Beispiel 33**

(5S)-3-(2-Benzylsulfonyl-benzo[4,5-d]thiazol-6-yl)-5-acetylaminomethyl-oxazolidin-2-on

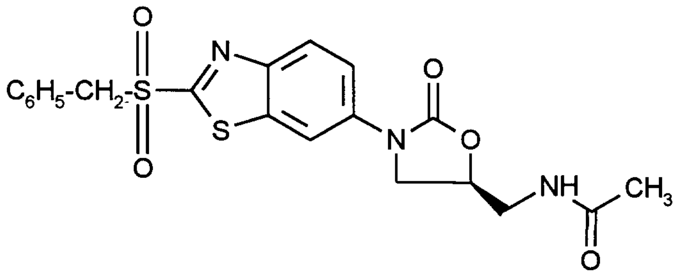

Eine gerührte Suspension von 2,50 g (6,13 mmol) der Verbindung aus Beispiel 27 in 25 ml Dichlormethan wird mit 3,17 g (14,7 mmol) 80%iger m-Chlorperbenzoesäure versetzt und 20 h bei Raumtemperatur gerührt. Danach wird das Gemisch in 50 ml 10%iger $Na_2SO_3$-Lösung eingerührt. Die organische Phase wird abgetrennt, mit 50 ml gesättiger $NaHSO_3$-Lösung gewaschen und über $MgSO_4$ getrocknet. Nach Abdampfen des Lösemittels im Vakuum und Chromatographie des Rückstands an 120 g Kieselgel (Ethylacetat : Aceton 9:1) erhält man 1,86 g (68%) der Titelverbindung als farblose Kristalle.
Schmp.: 194°C
$R_f$ = 0,14 (Ethylacetat: Aceton 9:1)
MS (FAB): m/z = 446 $(M+H)^+$
$^1$H-NMR (200 MHz, $D_6$-DMSO): δ = 1,84 (s, 3H, $COCH_3$); 3,45 (t, J = 6 Hz, 2 H, $CH_2N$); 3,85 (dd, J = 7, 10 Hz, 1H, H-4 trans); 4,22 (dd, J = 9, 10 Hz, 1H, H-4 cis); 4,45, 4,70 (AB, $J_{AB}$ = 13 Hz, 2H, $CH_2SO_2$); 4,80 (m, 1H, H-5); 7,10 - 7,35 (m, 5H, Ph); 7,93 (dd, J = 1, 10 Hz, 1H, Benzothiazol H-5); 8,13 (d, J = 10 Hz, 1H, Benzothiazol H-4); 8,30 (m, 2H, NH, Benzothiazol H-7).

**Beispiel 34**

(5S)-3-(2-Cyclopropylamino-benzo[4,5-d]thiazol-6-yl)-5-acetylaminomethyl-oxazolidin-2-on

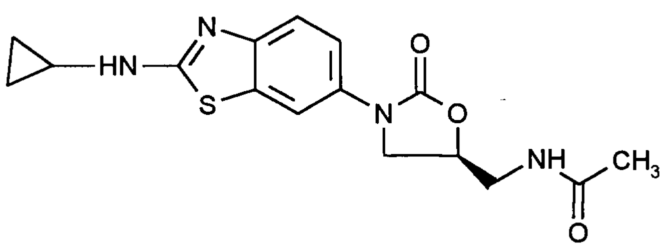

Eine Suspension von 110 mg (0,30 mmol) der Verbindung aus Beispiel 37 und 730 µl (10,5 mmol) Cyclopropylamin in 1 ml wasserfreiem Acetonitril wird 48 h zum Rückfluß erwärmt. Das Gemisch darf abkühlen, der entstandene Niederschlag wird abgetrennt und mit 0,5 ml Acetonitril gewaschen und im Hochvakuum getrocknet. Man erhält 60 mg (58%) der Titelverbindung als farblose Kristalle.
Schmp.: 226°C (Zers.)
$R_f$ = 0,26 (Dichlormethan : Methanol 92:8)
MS (DCI, $NH_3$): m/z = 347 $(M+H)^+$
$^1$H-NMR (200 MHz, $D_6$-DMSO): δ = 0,58, 0,78 (m, 4H, Cyclopropyl H); 1,82 (s, 3H, $COCH_3$); 2,70 (m, 1H, Cyclopropyl H); 3,43 (t, J= 6 Hz, 2H, $CH_2N$); 3,77 (dd, J = 7, 10 Hz, 1H, H-4 trans); 4,12 (dd, J= 10, 10 Hz, 1H, H-4 cis); 4,71 (m, 1H, H-5); 7,42 (s, 2H, Benzothiazol H); 7,89 (s, 1H, Benzothiazol H); 8,20 - 8,35 (m, 2H, NH).

**Beispiel 35**

(5S)-3-[2-(Pyridin-2-yl)thio-benzo[4,5-d]thiazol-6-yl]-5-acetylaminomethyl-oxazolidin-2-on

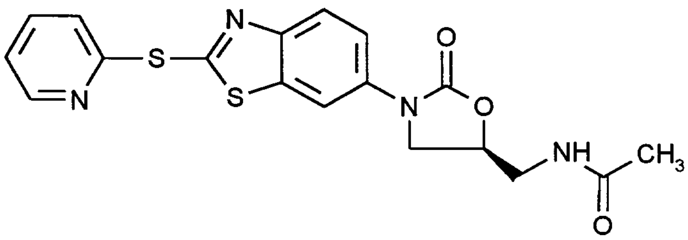

Eine gerührte Suspension von 300 mg (0,67 mmol) der Verbindung aus Beispiel 33 und 0,22 ml (1,55 mmol) Triethylamin in 3 ml Acetonitril wird mit 165 mg (1,48 mmol) 2-Mercaptopyridin versetzt und 21 h auf 60°C erwärmt. Das Gemisch darf abkühlen, das Lösemittel wird im Vakuum abgedampft und der Rückstand an 45 g Kieselgel (Dichlormethan : Methanol 95:5) gereinigt. Man erhält 119 mg (44%) der Titelverbindung als farblose Kristalle.
Schmp.: 129°C
$R_f$ = 0,06 (Dichlormethan : Methanol 95:5)
MS (DCI, $NH_3$): m/z = 401 $(M+H)^+$
$^1$H-NMR (200 MHz, $D_6$-DMSO): $\delta$ = 1,83 (s, 3H, $COCH_3$); 3,45 (m, 2H, $CH_2N$); 3,72 (dd, J = 7, 10 Hz, H-4 trans); 4,20 (dd, J = 10, 10 Hz, 1H H-4 cis); 4,78 (m, 1H, H-5); 7,5 (m, 1H, Pyridyl H-5); 7,70 (d, J = 10 Hz, 1H, Benzothiazol H-4); 7,8 - 8,0 (m, 4H); 8,20 (d, J = 1 Hz, 1H, Benzothiazol H-7); 8,28 (t, J = 6 Hz, 1H, NH); 8,62 (m, 1H, Pyridyl H-6).

Wie für die Beispiele 32,33 und 35 beschrieben, erhält man die folgenden Verbindungen (Tabelle 5):

**Tabelle 5:**

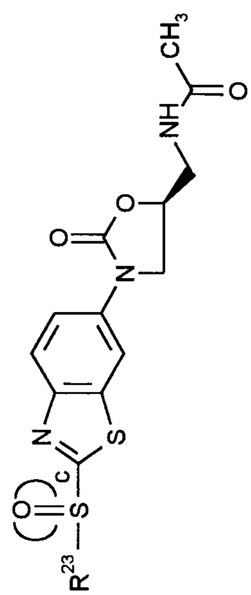

| Bsp.-Nr. | $R^{23}$ | c | analog Bsp.-Nr. | Ausbeute [% d.Th.] | Schmp. [°C] | $R_f$ / Laufmittel (Verhältnis) | $[\alpha]_D^{20}$ (DMSO) | MS (FAB) m/z $(M+H)^+$ |
|---|---|---|---|---|---|---|---|---|
| 36 | $C_6H_5$-$H_2C$- | 1 | 32 | 50 | 188 | 0,35, I (9:1) | | 430 |
| 37 | $H_3C$- | 2 | 33 | 40 | 197 | 0,14, I (95:5) | | 370 |
| 38 | 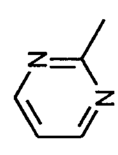 | 0 | 35 | 9 | 192 (Zers.) | 0,17, I (95:5) | | 402 |

**Beispiel 39**

(5S)-3-(2-Chlormethyl-benzo[4,5-d]thiazol-6-yl)-5-acetamidomethyl-oxazolidin-2-on

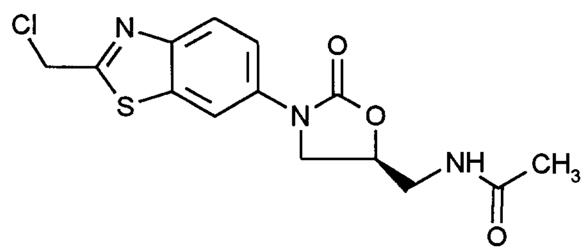

Eine Suspension von 153 mg (0,50 mmol) der Verbindung aus Beispiel 26 in 3 ml Sulfurylchlorid wird in Gegenwart von 10 mg Azoisobutyronitril 11 h auf 90°C erwärmt. Während der Reaktion werden 3 mal 10 mg Azoisobutyronitril nachgegeben. Zur Aufarbeitung wird in 50 ml Ether gegossen, der Niederschlag durch Filtration abgetrennt, mit 3 mal 5 ml Ether gewaschen und im Hochvakuum getrocknet. Man erhielt 120 mg (71%) der Titelverbindung als amorphen Feststoff.
$R_f$ = 0,16 (Dichlormethan : Methanol 95:5)
MS (FAB): m/z = 340 $(M+H)^+$

**Beispiel 40**

(5S)-3-(2-Formyl-benzo[4,5-d]thiazol-6-yl)-5-acetaminomethyl-oxazolidin-2-on

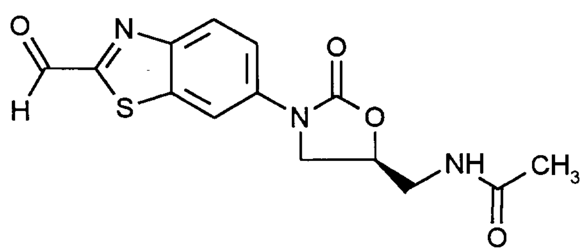

Durch eine auf -78°C gekühlte Lösung von 2,50 g (6,35 mmol) der Verbindung aus Beispiel 28 in 125 ml Dichlormethan und 12 ml Methanol wird bis zur Blaufärbung ein Ozon-Sauerstoffgemisch geleitet. Anschließend wird 10 min mit Stickstoff gespült um überschüssiges Ozon zu entfernen, dann werden 1,90 mg (26,50 mmol) Dimethylsulfid zugegeben. Es wird 30 min bei -10°C und 1 h bei Raumtemperatur gerührt, auf Abwesenheit von Ozonid geprüft und dann im Vakuum eingeengt. Der Rückstand wird in 50 ml Dichlormethan aufgenommen, mit gesättigter $NaHCO_3$-Lösung gewaschen und über $MgSO_4$ getrocknet. Nach Abdampfen des Lösemittels und Verreiben des Rückstands mit 25 ml Ether erhält man 1,70 g (79%) der Titelverbindung als Kristalle.

**Beispiel 41**

(5S)-3-[5-(2-Hydroxymethyl-benzo[4,5-d]thiazol-6-yl)]-5-acetyl-aminomethyl-oxazolidin-2-on

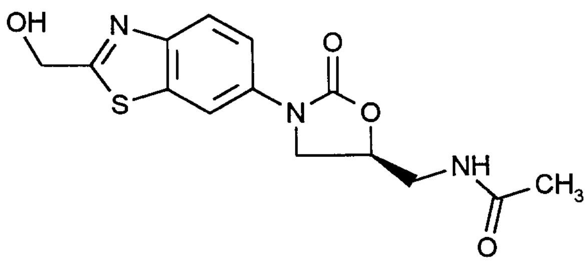

Eine gerührte, auf 0°C gekühlte Lösung von 82 mg (0,26 mmol) der Verbindung aus Beispiel 40 in 3 ml Methanol wird mit 10 mg (0,26 mmol) Natriumborhydrid versetzt und 4 h bei 0°C gerührt. Das Lösemittel wird im Vakuum abgedampft und der Rückstand wird durch Chromatographie an 2 g Kieselgel (Dichlormethan:Methanol) gereinigt. Man erhält 51 mg (61 %) der Titelverbindung als farblose Kristalle.
Schmp.: 180-182°C
$R_f$ = 0,20 (Dichlormethan:Methanol 9:1)
MS (DCI, $NH_3$) m/z = 322 $(M+H)^+$, 339 $(M+NH_4)^+$
$^1$H-NMR (250 MHz, $D_6$-DMSO) δ = 1,86 (s, 3H,$COCH_3$); 3,45 (m, 2H, $CH_2N$); 3,83 (dd, J = 8,10 Hz, 1H, H-4 trans); 4,20 (dd, J = 10, 10 Hz, 1H, H-4-cis); 4,76 (m, 1H, H-5); 4,85 (d, J = 5 Hz, 2H, $\underline{CH_2}OH$); 6,25 (bt, 1H, $CH_2O\underline{H}$); 7,76 (dd, J = 1,10 Hz, 1H, Benzothiazol H-5); 7,92 (d, J = 10 Hz, 1H, Benzothiazol H-4); 8,20 (d, J = 1 Hz, 1H, Benzothiazol H-7); 8,25 (m, 1H, NHCO).

**Beispiel 42**

(5S)-3-(2-Methylthio-3-methyl-benzo[4,5-d]-thiazol-6-yl)-5-(acetylaminomethyl)-oxazolidin-2-on Iodid

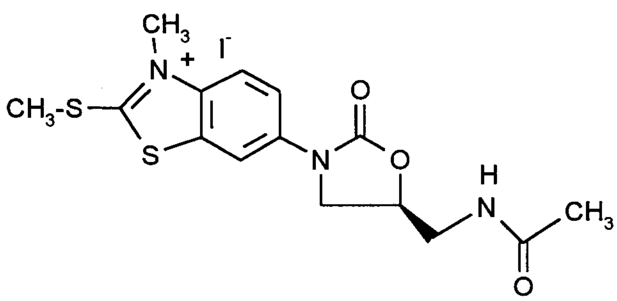

Eine gerührte Lösung von 1,35 g (4,00 mmol) der Verbindung aus Beispiel 24 in 6 ml wasserfreiem DMF wird mit 2,6 ml (40,00 mmol) Iodmethan versetzt und 23 Stunden auf 70°C erhitzt.

Danach darf die Reaktionsmischung abkühlen, man gibt 80 ml Ether zu und trennt den entstandenen Niederschlag durch Filtration ab. Nach Verrühren in 50 ml Ethanol, erneuter Filtration und Trocknen des Produkts im Hochvakuum über Sicapent erhält man 1,17 g (61 %) der Titelverbindung als farblose Kristalle.
Schmp.: 149°C (Zers.)

Die Titelverbindung kann wie oben beschrieben auch aus der Verbindung Beispiel 27 in 79 % Ausbeute erhalten werden.
MS (FAB) m/z = 352 (Kation $M^+$).
$^1$H-NMR (250 MHz, DMSO-$d_6$) δ = 8,60 (d, J = 1Hz, 1H, Benzothiazol (H-7); 8,28 (m, 1H, NHCO); 8,20 (d, J = 10 Hz, 1H, Benzothiazol H-4); 8,02 (dd, J = 1, 10 Hz, 1H, Benzothiazol H-5); 4,82 (m 1H, H-5); 4,20 (dd, J = 10, 10Hz, 1H, H-4 cis); 4,10 (s, 3H, $NCH_3$); 3,85 (dd, J = 7, 10 Hz, 1H, H-4 trans); 3,46 (m, 2H, $CH_2N$); 3,12 (s, 3H, $SCH_3$); 1,85 (s, 3H, $COCH_3$).

**Beispiel 43**

(5R)-3-(2-[4'-Chlor-styryl]-benzo[4,5-d]thiazol-6-yl)-5-methansulfonyloxy-methyl-oxazolidin-2-on

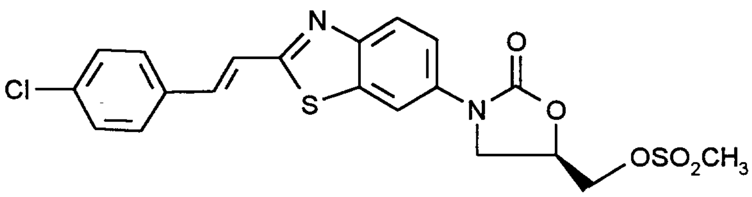

Unter Argon gibt man zu einer Suspension von 24,2 g (63 mmol) der Verbindung aus Beispiel XXII in 500 ml absolutem THF 17,4 ml (125 mmol) Triethylamin und kühlt auf 0°C ab.

Man gibt langsam 6,1 ml (78 mmol) Methansulfonsäurechlorid zu und läßt 1 Stunde bei 0°C nachrühren.

Man läßt über Nacht auf Raumtemperatur kommen und gießt den Ansatz auf 4 l Eiswasser. Es wird kurz nachgerührt und dann das ausgefallene Produkt abgesaugt. Man wäscht mit Wasser nach, bis das Waschwasser neutral reagiert. Das Produkt wird im Hochvakuum getrocknet. Man erhält 28 g (96 %) der Titelverbindung als gelbe Kristalle.

Schmp.: 231°C

$R_f$ = 0,23 (Toluol:Essigester = 1:2)

MS (FAB) m/z = 465 $(M + H)^+$

$^1$H-NMR (200 MHz, DMSO-$d_6$) $\delta$ = 8,25 (d, J = 1,5, 1H, Benzothiazol H-7); 8,00 (d, J = 9,5, 1H, Benzothiazol H-4); 7,80 (m, 3H, Benzothiazol H-5, Phenyl H-3,5); 7,64 (s, 2H, Vinyl); 7,50 (d, J = 9, 2H, Phenyl H-2,6); 5,06 (m, 1H, H-5); 4,53 (m, 2H, $CH_2OMs$); 4,30 (dd, J = 9,5, 1H, H-4); 3,94 (dd, J = 9,5, J = 6, 1H, H-4); 3,33 (s, 3H, $OSO_2CH_3$).

In Analogie zur Vorschrift des Beispiels 9 werden die in Tabelle 6 aufgeführten Verbindungen hergestellt:

**Tabelle 6**

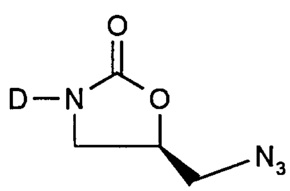

| Bsp.-Nr. | D | Ausbeute (% d.Th.) | Schmp. (°C) | $R_f$ | MS |
|---|---|---|---|---|---|
| 44 | 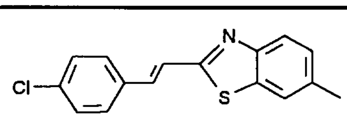 | 92 | 209 u. Zers. | 0,80 I (195:5) | 412[a)] |

a) = MS (DCI, $NH_3$) m/z = $(M + H)^+$

**Beispiel 45**

(5S)-3-(2-[4'-Chlor-styryl]-benzo[4,5-d]thiazol-6-yl)-5-aminomethyl-oxazolidin-2-on Hydrochlorid

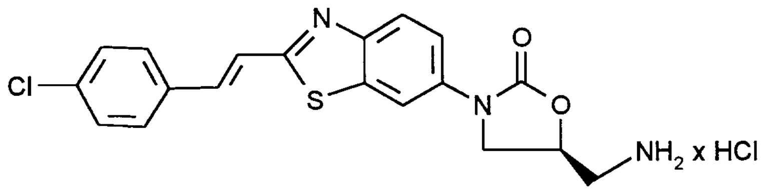

Man tropft zu einer bei 50°C gerührten Suspension 23 g (56 mmol) der Verbindung aus Beispiel 44 in 200 ml Dimethoxyethan langsam 11,5 ml (67 mmol) Triethylphosphit zu (Gasentwicklung !).

Nach beendeter Gasentwicklung wird 1 h auf 90°C erhitzt. Man gibt 23 ml 6N HCl zu und läßt 36 h bei 90°C nachrühren. Man kühlt auf Raumtemperatur ab, versetzt mit 200 ml Diethylether, läßt 15 Minuten nachrühren und saugt ab. Man wäscht mit Diethylether, und trocknet zunächst im Hochvakuum, dann im Umlufttrockenschrank bei 70°C.

Man erhält 23,0 g (98 %) der Titelverbindung als gelben Feststoff.

Schmp.: >255°C

$R_f$ = 0,12 ($CH_2Cl_2$ : MeOH : $NH_{3(ag)}$ = 100 : 5 : 2)

$^1$H-NMR (200 MHz, DMSO-$d_6$) δ: 8,57

$$(\text{s breit, 3H, } -\overset{\oplus}{N}H_3);$$

8,24 (d, J = 1,5, 1H, H-7); 8,01 (d, J = 9,5, 1H, H-4); 7,80 (m, 3H, Benzothiazol H-5, Phenyl H-3,5); 7,65 (s, 2H, Vinyl); 7,50 (d, J = 9, 2H, Phenyl H-2,6); 5,05 (m, 1H, H-5); 4,30 (dd, J = 9,5, J = 9,5, 1H, H-4); 4,02 (dd, J = 9,5, J = 6, 1H, H-4); 3,29 (m, 2H, $\underline{CH_2}$-$NH_2$)

MS (EI) m/z = 385 ($M^+$)

**Beispiel 46**

(5S)-3-(2-[4'-Chlor-styryl]-benzo[4,5-d]-6-yl)-5-acetaminomethyl-oxazolidin-2-on

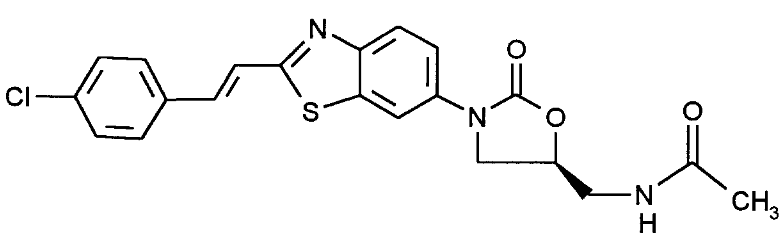

Zu einer unter Argon gerührten Suspension von 23 g (55 mmol) der Verbindung aus Beispiel 45 in 500 ml THF (abs.) gibt man 23,7 ml (136 mmol) Diisopropylethylamin und kühlt auf 0°C ab. Man setzt 5,8 ml (80 mmol) Acetylchlorid zu und rührt 30 Minuten bei 0°C nach.

Man läßt auf Raumtemperatur kommen und gibt den Ansatz auf 4,5 l Wasser. Nach 15 Minuten rühren wird abgesaugt und mit Wasser gewaschen. Man rührt den Feststoff nochmals mit Wasser aus und saugt wieder ab. Anschließend wird das Produkt im Umlufttrockenschrank bei 80°C getrocknet. Man erhält 21,1 g (90 %) der Titelverbindung als beige Kristalle.

Schmp.: 253°C unter Zersetzung
$R_f$ = 0,22 ($CH_2Cl_2$: MeOH:$NH_{3(ag)}$ = 100 : 5 : 2)
MS (EI) m/z = 427 $(M)^+$
$[\alpha]_D^{20}$ = (DMSO) = -29,3° (C = 1)
$^1$H-NMR (300 MHz, DMSO-$d_6$) δ = 8,28 (tr, J = 5,5, 1H, NH); 8,20 (d, J = 1,5, 1H, H-7); 7,99 (d, J = 9,5, 1H, H-4); 7,80 (m, 3H, Benzothiazol H-5, Phenyl H-3,5); 7,62 (s, 2H, Vinyl); 7,50 (d, J = 9, 2H, Phenyl H-2,6); 4,78 (m, 1H, H-5); 4,22 (dd, J = 9,5, J = 9,5, 1H, H-4); 3,85 (dd, J = 9,5, J = 6,5, 1H, H-4); 3,46 (dd, J = 5,5, J = 5,5, 2H, $\underline{CH_2}$-NH); 1,85 (s, 3H, $COCH_3$).

**Beispiel 47**

(5S)-3-(2-[3'-Butenyl]-benzo[4,5-d]-5-yl)-5-acetaminomethyl-oxazolidin-2-on

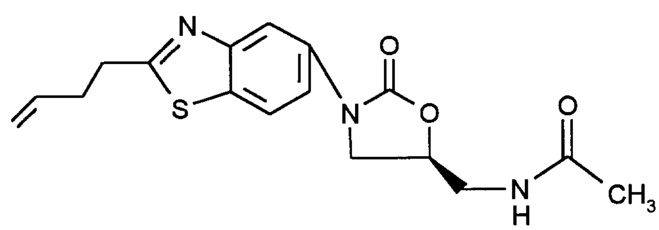

Eine Lösung von 20 mg (0,065 mmol) der Verbindung aus Beispiel 30 werden in 1 ml absolutem THF unter Argon gelöst und auf -76°C abgekühlt. Man addiert 0,235 ml einer frisch hergestellten 0,83 molaren LDA-Lösung (0,195 mmol) und rührt 1 Stunde bei -76°C. Man addiert 5,6 µl (0,065 mmol) Allylbromid und läßt 1 Stunde bei -76°C rühren. Man läßt auf Raumtemperatur erwärmen, versetzt mit 10 ml $H_2O$, extrahiert 3 x mit Essigester, trocknet über $MgSO_4$ und engt ein. Man reinigt an Kieselgel mit Essigester : MeOH = 100 : 4 und erhält 12,4 mg (55 %) der Titelverbindung.
$R_f$ = 0,20 (Essigester MeOH = 100 : 4)
MS (EI) m/z = 345 $(M)^+$
$^1$H-NMR (200 MHz, DMSO-$d_6$) δ = 8,15 (tr, J = 6, 1H, NH); 7,9 (m, 2H, Benzothiazol H-4,7); 7,55 (dd, J = 9,5, J = 2, 1H, H-6); 5,77 (m, 1H -$\underline{CH}$=$CH_2$); 4,98 (m, 1H, CH=$\underline{CH_2}$); 4,89 (m, 1H, CH=$\underline{CH_2}$); 4,62 (m, 1H, H-5); 4,09 (dd, J = 9,5, J = 9,5, 1H, H-4); 3,70 (dd, J = 9,5, J = 6, 1H, H-4); 3,30 (dd, J = 6, J = 6, 2H, $\underline{CH_2}$-NH); 3,06 (tr, J = 7,5, 2H, $\underline{CH_2}$-$CH_2$-CH=$CH_2$); 2,45 (m, 2H, $\underline{CH_2}$-CH=$CH_2$); 1,70 (s, 3H, $COCH_3$).

**Beispiel 48**

(5S)-3-[2-(Ethoxycarbonylmethylthio)benzoxazol-6-yl]-5-acetylamimo-methyloxazolidin-2-on

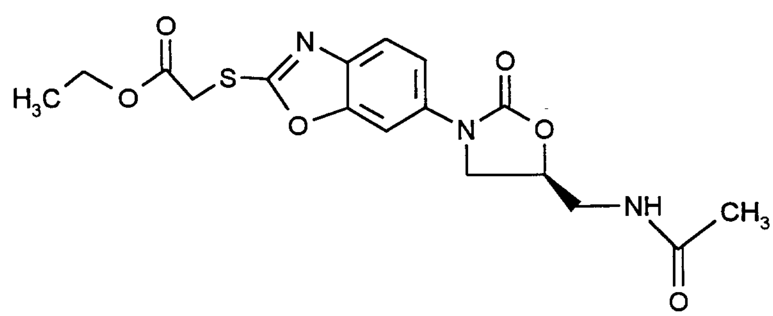

290 mg (1,1 mmol) der Verbindung aus Beispiel XXXVI und 193 mg (1,2 mmol) Kalium-O-ethyldithiocarbonat in 6 ml Ethanol werden 8 Stunden bei 70°C gerührt. Das Lösungsmittel wird abgezogen, der Rückstand in 20 ml Methanol suspendiert mit 265 mg (1,6 mmol) Bromessigsäureethylester versetzt. Nach 1 Stunde bei 0°C werden 40 ml Wasser

hinzugegeben, die wäßrige Phase mit Essigester extrahiert, die vereinigten organischen Phasen getrocknet ($Na_2SO_4$) und die Lösungsmittel abgezogen. Der Rückstand wird aus Methanol umkristallisiert.

Ausbeute 155 mg (36 %)

$R_f$ = 0,67 (VII, 1:1)

Schmp.: 146-147°C

$[\alpha]_D^{20}$ = -19,05 (c = 0,5, DMSO)

MS (FAB): m/z = 394 ($M^+$ + H)

$^1$H-NMR (250 MHz, $D_6$-DMSO): 1,20 (t, 3H, $CH_3$); 1,80 (s, 3H, $NHCOCH_3$); 3,35-3,45 (m, 2H, $CH_2N$), 3,80 (dd, 1H, H-4), 4,10-4,25 (m, 3H, $CO_2CH_2$, H-4), 4,70-4,85 (m, 1H, H-5), 7,48 (dd, 1H; Benzoxazol H-5), 7,60 (d, 1H, Benzoxazol H-4), 7,90 (d, 1H, Benzoxazol H-7), 8,23 (bs, 1H, NH).

Die in der Tabelle 7 aufgeführten Verbindungen werden in Analogie zur Vorschrift des Beispiels 48 hergestellt.

**Tabelle 7**

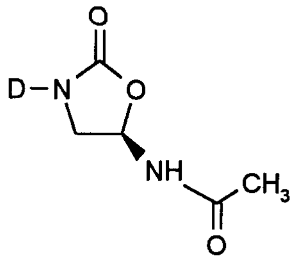

| Bsp.-Nr. | D | Ausbeute [%d.Th.] | Smp. [°C] | $R_f$ Laufmittel (Verhältnis) | $[\alpha]_D^{20}$ (DMSO) | MS(FAB) m/z $(M+H)^+$ |
|---|---|---|---|---|---|---|
| 49 | 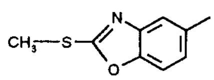 | 92 | 205-206 | 0,65 VII (1:1) | -21,9 (c = 1,0) | 321[a)] |
| 50 | 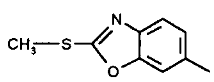 | 83 | 163-165 | 0,62 VII (1:1) | - | 321[a)] |
| 51 | 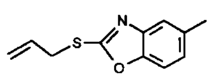 | 40 | 181-182 | 0,59 VII (1:1) | -19,0 (c = 1,0) | 347[a)] |
| 52 | 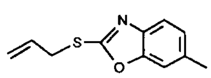 | 47 | 145-147 | 0,60 VII (1:1) | -19,0 (c = 1,0) | 347[a)] |
| 53 | 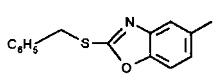 | 60 | 169-170 | 0,65 VII (1:1) | -15,9 (c = 0,5) | 397[a)] |
| 54 | 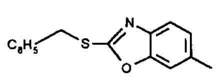 | 30 | 150-151 | 0,77 VII (1:1) | - | 397[a)] |
| 55 | 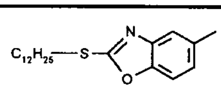 | 56 | 158-159 | 0,73 VII (1:1) | -15,1 (c = 0,5) | 476 |
| 56 | 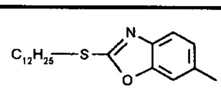 | 30 | 147-148 | 0,65 VII (1:1) | - | 476 |

a) MS (EI) m/Z = $(M)^+$

**Beispiel 57**

(5S)-3-(Benzoxazo-6-yl)-5-acetylaminomethyloxazolidin-2-on

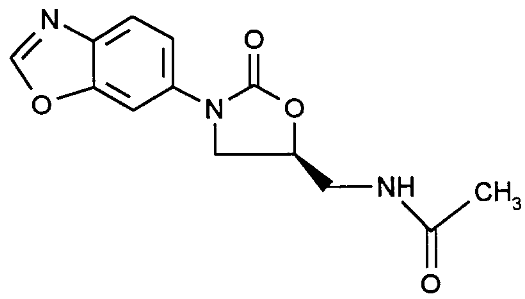

Eine Mischung aus 500 mg (1,88 mmol) der Verbindung aus Beispiel XXXVI 50 µl konz. Salzsäure und 13 ml Triethylorthoformiat werden 2 Tage bei Raumtemperatur gerührt. Der überschüssige Orthoester wird im Vakuum abgezogen und der Rückstand durch Chromatographie gereinigt.
Ausbeute: 363 mg (70 %)
$R_f$: 0,18 (VII, 5:1)
Schmp.: 186,5°C
$[\alpha]_D^{20}$ = 26,7 (c = 1,0, DMSO)
MS (EI): m/z, 272
$^1$H-NMR (250 MHz, $D_6$-DMSO): δ = 1,85 (s, 3H, $COCH_3$); 3,47 (t, J = 6 Hz, 2H, $CH_2N$); 3,87 (dd, J = 7, 10 Hz, 1H, H-4); 4,23 (dd, J = 10, 10 Hz, 1H, H-4); 4,80 (m, 1H, H-5); 7,55 (dd, 1H, Benzoxazol H-5); 7,80 (d, J = 10 Hz, 1H, Benzoxazol H-4); 7,95 (d, J = 1,5 Hz, 1H, Benzoxazol H-7); 8,25 (bt, J = 1,5 Hz, NH); 8,70 (s, 1H, Benzoxazol H-2).

Die in Tabelle 8 aufgeführten Verbindungen werden in Analogie zur Vorschrift des Beispiels 57 hergestellt.

**Tabelle 8**

| Bsp. -Nr. | | Ausbeute (% d.Th.) | $R_f$ | Schmp.: (°C') | $\alpha_D$ | Ms (EI) m/z ($M^+$) |
|---|---|---|---|---|---|---|
| 58 | 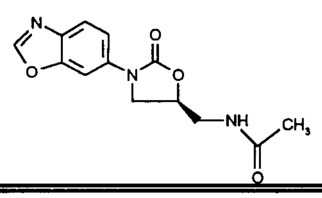 | 50 | 0,47 VII (1:1) | 170 | -26,7 (c = 1,0) | 275 |

Die in der Tabelle 9 aufgeführten Verbindungen werden nach den folgenden Methoden hergestellt:

**Tabelle 9**

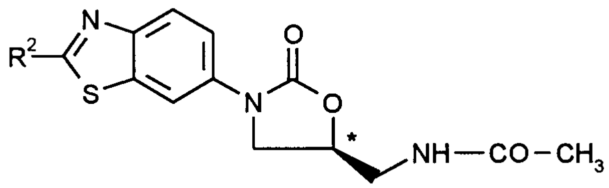

| Bsp.-Nr. | $R^2$ | Methode | Ausbeute (% d. Th.) | Fp. °C | $R_f$-Wert Laufmittel | MS m/z $(M+H)^+$ |
|---|---|---|---|---|---|---|
| 59 | 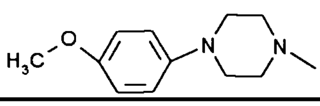 | 1) | 85 | >250 | 0,5 (I) 10:1 | 482 |
| 60 | 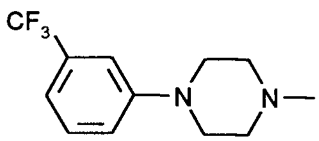 | 1) | 26,4 | 225 | 0,35 (I) 10:1 | 520 |
| 61 | 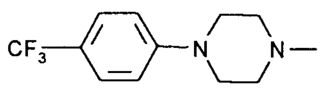 | 1) | 85 | >250 | 0,35 (I) 10:1 | 520 |
| 62 | 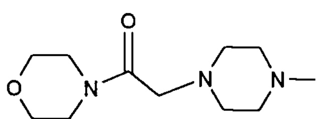 a) | 1) | 61,8 | 219 | 0,39 (I) 10:1 | 503 |
| 63 | 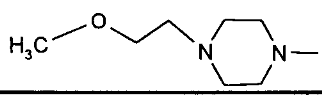 | 2) | 55,2 | 147 | 0,35 (I) 10:1 | 434 $M^{+N}$ |

| Bsp.-Nr. | $R^2$ | Methode | Ausbeute (% d. Th.) | Fp. °C | $R_f$-Wert Laufmittel | MS m/z $(M+H)^+$ |
|---|---|---|---|---|---|---|
| 64 | 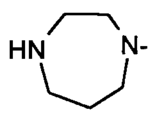 | 2) | 96 | amorph | 0,12 (I) 10:1 | 390 |
| 65 | 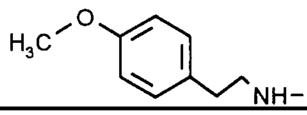 | 1) | 95,8 | 168 | 0,45 (I) 10:1 | 441 |
| 66 | 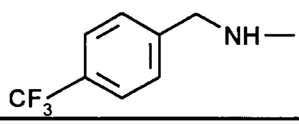 | 1) | 35,6 | 203 | 0,54 (I) 10:1 | 465 |
| 67 | 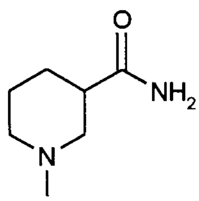 | 1) | 85,9 | 214 | 0,39 (I) 10:1 | 418 |
| 68 | 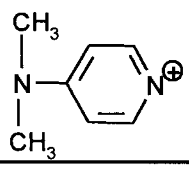 | 1) | 48,1 | 221 Zers. | - | 412 7) |
| 69 | 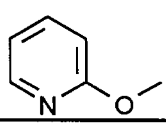 | 1) | 90 | >250 | 0,53 (I) 10:1 | 385 |
| 70 | 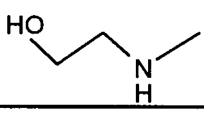 | 1) | 47 | 183 | 0,14, I (9:1) | 351 |
| 71 | 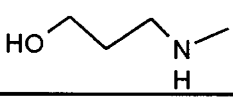 | 1) | 42 | 182 | 0,20, I (9:1) | 365 |
| 72 | 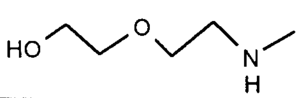 | 1) | 53 | 73 | 0,40, I (9:1) | 395 |
| 73 | 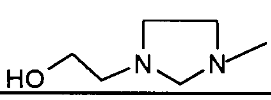 | 1) | 68 | 149 | 0,37, III (4:1) | 406 |
| 74 | 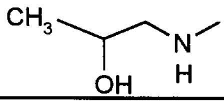 | 1) | 59 | 93 | 0,19, I (9:1) | 365 |

| Bsp.-Nr. | $R^2$ | Methode | Ausbeute (% d. Th.) | Fp. °C | $R_f$-Wert Laufmittel | MS m/z $(M+H)^+$ |
|---|---|---|---|---|---|---|
| 75 | 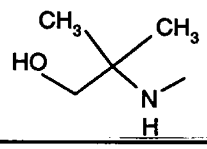 | 1) | 43 | - | 0,12, I (9:1) | 379 |
| 76 | 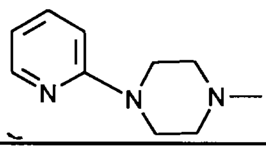 | 1) | 85,7 | 223-224 | 0,46 | 453 |
| 77 | 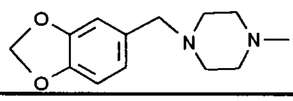 | 1) | 28 | 206-207 | 0,4 | 510 |
| 78 | 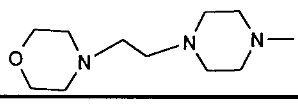 | 1) | 83,8 | 181-182 | 0,11 | 489 |
| 79 | 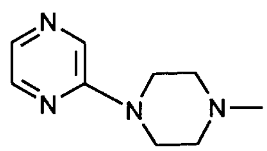 | 1) | 80,7 | >250 | 0,36 | 454 |
| 80 | 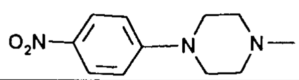 | 1) | 30,5 | >250 | 0,44 | 497 |
| 81 | 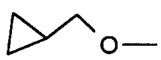 | 3) | 30 | 165-167 | 0,46 | 362 |
| 82 | 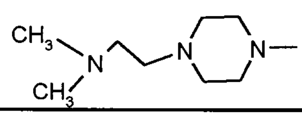 | 1) | 42 | 183 | 0,2/I (10:1) | 447 |
| 83 | 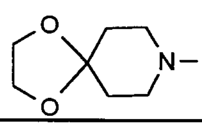 | 1) | 47 | 188 | 0,45/I (10:1) | 432 |
| 84 | 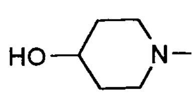 | 1) | 65 | 211 | 0,2/I (10:1) | 391 |
| 85 | 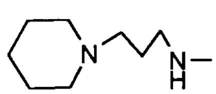 | 1) | 19 | 131 | 0,05/I (10:1) | 432 |
| 86 | 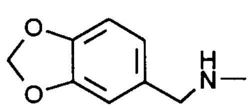 | 1) | 31 | 227 | 0,35/I (10:1) | 441 |

| Bsp.-Nr. | $R^2$ | Methode | Ausbeute (% d. Th.) | Fp. °C | $R_f$-Wert Laufmittel | MS m/z $(M+H)^+$ |
|---|---|---|---|---|---|---|
| 87 | 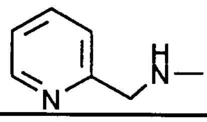 | 1) | 52 | 214 | 0,2/I (10:1) | 398 |
| 88 | 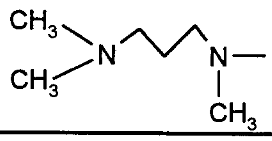 | 1) | 43 | 114 | 0,2/I (10:1) | 406 |
| 89 | 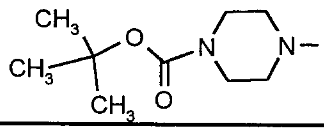 | 1) | 38 | 208 | 0,45/I (10:1) | 476 |
| 90 | x HCl 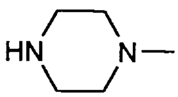 | 4) | 99 | 201 | - | 376 |
| 91 | 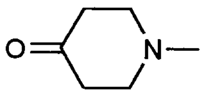 | 5) | 90 | - | 0,49/I (10:1) | 389 |
| 92 | 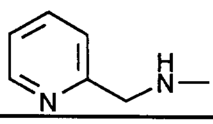 xHCl | 6) | 99 | - | - | - |
| 93 | 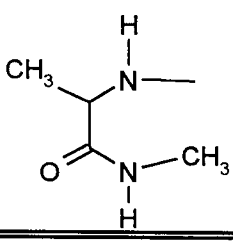 | 1) | 43 | 207 u.Zersetzung | - | - |

1) 1 mmol Sulfon 37 mit 10 mmol des Nukleophils in 4 ml Acetonitril 2 Tage zum Rückfluß erhitzen. Aufarbeitung wie Beispiel 34 oder 35.

2) 1 mmol Sulfon 37 mit 35 mmol des Nukleophils in 4 ml Acetonitril 2 Tage zum Rückfluß erhitzen. Aufarbeitung wie Beispiel 34 oder 35.

3) 1,05 mmol KH wird mit 1,05 mmol des Nukleophils in 4 ml DMF versetzt und anschließend bei 0°C mit 1 mmol des Sulfons 37 versetzt. Man läßt auf Raumtemperatur kommen und läßt 1 Stunde rühren. Man versetzt mit Diethylether und saugt ab.

4) Umsetzung von Beispiel 89 mit 6 N HCl/Dioxan

5) Umsetzung von Beispiel 83 mit 4 N HCl/Dioxan

6) Umsetzung von Beispiel 87 mit HCl/Ether

7) $M^+$ des Kations, Gegenion $CH_3SO_2^{\ominus}$

## Patentansprüche

1. Verbindungen der allgemeinen Formel (I)

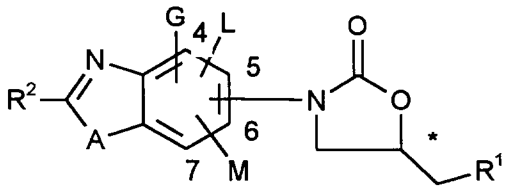

(I)

in welcher

A für ein Sauerstoffatom oder für einen Rest der Formel - $S(O)_a$ steht, worin

a eine Zahl 0 oder 2 bedeutet,

$R^1$ für Azido oder für eine Gruppe der Formel $O-SO_2R^3$ oder $-NR^4R^5$ steht, worin

$R^3$ geradkettiges oder verzweigtes Alkyl mit bis zu 4 Kohlenstoffatomen oder Phenyl bedeutet, das gegebenenfalls durch geradkettiges oder verzweigtes Alkyl mit bis zu 4 Kohlenstoffatomen substituiert ist,

$R^4$ und $R^5$ gleich oder verschieden sind und Cycloalkyl mit 3 bis 6 Kohlenstoffatomen, Wasserstoff, Phenyl oder geradkettiges oder verzweigtes Alkyl mit bis zu 8 Kohlenstoffatomen oder eine Aminoschutzgruppe bedeuten, oder

$R^4$ oder $R^5$ eine Gruppe der Formel $-CO-R^6$ bedeutet, worin

$R^6$ Cycloalkyl mit 3 bis 6 Kohlenstoffatomen, geradkettiges oder verzweigtes Alkyl oder Alkoxy mit jeweils bis zu 8 Kohlenstoffatomen , Phenyl oder Wasserstoff bedeutet,

G, L und M gleich oder verschieden sind und für Wasserstoff, Carboxy, Halogen, Cyano, Formyl, Trifluormethyl, Nitro, geradkettiges oder verzweigtes Alkoxy, Alkoxycarbonyl, Alkylthio oder Acyl mit jeweils bis zu 6 Kohlenstoffatomen oder für geradkettiges oder verzweigtes Alkyl mit bis zu 6 Kohlenstoffatomen stehen, das seinerseits durch Hydroxy, durch geradkettiges oder verzweigtes Alkoxy oder Acyl mit bis zu 5 Kohlenstoffatomen oder durch eine Gruppe der Formel $-NR^7R^8$ substituiert sein kann, worin

$R^7$ und $R^8$ gleich oder verschieden sind und Wasserstoff, geradkettiges oder verzweigtes Alkyl mit bis zu 4 Kohlenstoffatomen oder Phenyl bedeuten, oder gemeinsam mit dem Stickstoffatom einen 5- bis 6-gliedrigen, gesättigten Heterocyclus mit gegebenenfalls einem weiteren Heteroatom aus der Serie N, S und/oder O bilden, der seinerseits gegebenenfalls, auch an einem weiteren Stickstoffatom, durch geradkettiges oder verzweigtes Alkyl oder Acyl mit bis zu 3 Kohlenstoffatomen substituiert sein kann,

und/oder
gegebenenfalls für eine Gruppe der Formel $-NR^{7'}R^{8'}$ stehen,
worin

$R^{7'}$ und $R^{8'}$ gleich oder verschieden sind und die oben angegebene Bedeutung von $R^7$ und $R^8$ haben und mit dieser gleich oder verschieden sind, und/oder gegebenenfalls für $(C_2$-$C_8)$-Alkenylphenyl, Phenyl oder durch einen 5- oder 6-gliedrigen gesättigten oder ungesättigten Heterocyclus mit bis zu 3 Heteroatomen aus der Reihe S, N und/oder O stehen, die ihrerseits gegebenenfalls durch eine Gruppe der Formel $-CO-NR^9R^{10}$, $NR^{11}R^{12}$, $NR^{13}-SO_2-R^{14}$, $R^{15}R^{16}N-SO_2-$ oder $R^{17}-S(O)_b$-substituiert sind, worin

b eine Zahl 0, 1 oder 2 bedeutet,

$R^9$, $R^{10}$, $R^{13}$, $R^{15}$ und $R^{16}$ gleich oder verschieden sind und Wasserstoff, geradkettiges oder verzweigtes Alkyl mit bis zu 6 Kohlenstoffatomen oder Phenyl bedeuten,

$R^{11}$ und $R^{12}$ gleich oder verschieden sind und die oben angegebene Bedeutung von $R^7$ und $R^8$ haben und mit dieser gleich oder verschieden sind,

$R^{14}$ und $R^{17}$ gleich oder verschieden sind und die oben angegebene Bedeutung von $R^3$ haben und mit dieser gleich oder verschieden sind, und/oder ihrerseits gegebenenfalls bis zu 2-fach gleich oder verschieden durch Carboxy, Halogen, Cyano, Formyl, Trifluormethyl, Nitro, Phenyl, geradkettiges oder verzweigtes Alkoxy, Alkoxycarbonyl, Alkylthio oder Acyl mit jeweils bis zu 6 Kohlenstoffatomen oder durch geradkettiges oder verzweigtes Alkyl mit bis zu 6 Kohlenstoffatomen substituiert sind, das seinerseits durch Hydroxy, durch geradkettiges oder verzweigtes Alkoxy oder Acyl mit bis zu 5 Kohlenstoffatomen oder durch eine Gruppe der Formel $-NR^{18}R^{19}$ substituiert sein kann, worin

$R^{18}$ und $R^{19}$ die oben angegebene Bedeutung von $R^7$ und $R^8$ haben und mit dieser gleich oder verschieden sind,

$R^2$ für Wasserstoff, Formyl, Carboxy, für geradkettiges oder verzweigtes Alkoxycarbonyl mit bis zu 6 Kohlenstoffatomen steht, oder

für geradkettiges oder verzweigtes Alkyl oder Alkenyl mit jeweils bis zu 8 Kohlenstoffatomen steht, die gegebenenfalls durch Hydroxy, Halogen oder durch geradkettiges oder verzweigtes Alkoxy, Acyl, Alkylthio oder Alkoxycarbonyl mit jeweils bis zu 6 Kohlenstoffatomen oder Phenyl substituiert sind, das seinerseits durch Halogen substituiert sein kann, oder

für Aryl mit 6 bis 10 Kohlenstoffatomen steht, das gegebenenfalls durch Carboxy, Halogen, Cyano, Formyl, Trifluormethyl, Nitro, geradkettiges oder verzweigtes Alkoxy, Alkoxycarbonyl, Alkylthio oder Acyl mit jeweils bis zu 6 Kohlenstoffatomen oder durch geradkettiges oder verzweigtes Alkyl mit bis zu 6 Kohlenstoffatomen substituiert ist,

oder

für einen Rest der Formel - $NR^{20}R^{21}$, $-OR^{22}$ oder $-S(O)_c-R^{23}$ steht,

worin

$R^{20}$ Cycloalkyl mit 3 bis 6 Kohlenstoffatomen, Phenyl, geradkettiges oder verzweigtes Acyl mit bis zu 6 Kohlenstoffatomen oder geradkettiges oder verzweigtes Alkyl mit bis zu 8 Kohlenstoffatomen bedeutet, das gegebenenfalls durch Hydroxy, geradkettiges oder verzweigtes Alkoxy oder Hydroxy substituiertes Alkoxy mit jeweils bis zu 6 Kohlenstoffatomen, durch einen 5-bis 6-gliedrigen aromatischen Heterocyclus mit bis zu 3 Heteroatomen aus der Reihe S, N und/oder O, oder durch Phenyl substituiert ist, das seinerseits durch Hydroxy, Trifluormethyl, Halogen, Nitro oder durch geradkettiges oder verzweigtes Alkoxy mit bis zu 4 Kohlenstoffatomen substituiert sein kann, oder

Alkyl gegebenenfalls durch einen Rest der Formel - $NR^{24}R^{25}$ oder

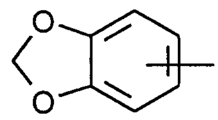

substituiert ist,

worin

$R^{24}$ und $R^{25}$ gleich oder verschieden sind und Wasserstoff oder geradkettiges oder verzweigtes Alkyl mit bis zu 4 Kohlenstoffatomen bedeuten, oder

$R^{20}$ einen Rest der Formel

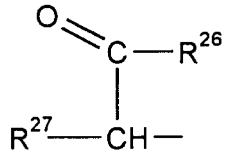

bedeutet

worin

$R^{26}$ Hydroxy oder geradkettiges oder verzweigtes Alkoxy mit bis zu 4 Kohlenstoffatomen oder einen Rest der Formel $-NR^{28}R^{29}$ bedeutet, worin $R^{28}$ und $R^{29}$ gleich oder verschieden sind und Wasserstoff, geradkettiges oder verzweigtes Alkyl mit bis zu 5 Kohlenstoffatomen, Cycloalkyl mit 3 bis 6 Kohlenstoffatomen oder Phenyl bedeuten

$R^{27}$ Wasserstoff oder geradkettiges oder verzweigtes Alkyl mit bis zu 7 Kohlenstoffatomen bedeutet, das gegebenenfalls durch Indolyl, Hydroxy, Mercaptyl, Imidazolyl, Methylthio, Amino, Phenyl, Hydroxy substituiertes Phenyl oder durch einen Rest der Formel $-CO-NH_2$, $-CO_2H$ oder

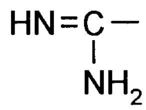

substituiert ist, oder

$R^{20}$ einen Rest der Formel

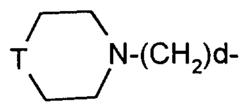

bedeutet
worin

d eine Zahl 0, 1, 2, 3, 4, 5 oder 6 bedeutet

T ein Sauerstoffatom oder eine Gruppe der Formel $CH_2$ oder $-NR^{30}$ bedeutet,
worin

$R^{30}$ Wasserstoff, Phenyl oder geradkettiges oder verzweigtes Alkyl mit bis zu 6 Kohlenstoffatomen bedeutet, das gegebenenfalls durch Hydroxy substituiert ist,

und

$R^{21}$ die oben angegebene Bedeutung von $R^{20}$ hat und mit dieser gleich oder verschieden ist, oder Wasserstoff bedeutet,

$R^{22}$ geradkettiges oder verzweigtes Alkyl mit bis zu 8 Kohlenstoffatomen bedeutet, das gegebenenfalls durch geradkettiges oder verzweigtes Alkoxy oder Hydroxy oder Alkoxy substituiertes Alkoxy mit jeweils bis zu 6 Kohlenstoffatomen, Cycloalkyl mit 3 bis 6 Kohlenstoffatomen oder einen 6-gliedrigen aromatischen, gegebenenfalls benzokondensierten Heterocyclus mit bis zu 4 Stickstoffatomen substituiert ist,
der seinerseits bis zu 2fach gleich oder verschieden durch Nitro, Trifluormethyl, Halogen, Cyano, Hydroxy oder durch geradkettiges oder verzweigtes Alkyl, Alkoxy oder Acyl mit jeweils bis zu 5 Kohlenstoffatomen substituiert sein kann,

oder

$R^{22}$ einen Rest der Formel

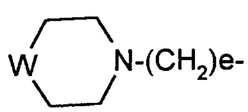

bedeutet,
worin

e die oben angegebene Bedeutung von d hat und mit dieser gleich oder verschieden ist,

W die oben angegebene Bedeutung von T hat und mit dieser gleich oder verschieden ist,

oder

$R^{22}$ Phenyl oder Pyridyl bedeutet,

c eine Zahl 0, 1 oder 2 bedeutet,

$R^{23}$ geradkettiges oder verzweigtes Alkyl oder Alkenyl mit bis zu 16 Kohlenstoffatomen bedeutet, das gegebenenfalls durch geradkettiges oder verzweigtes Alkoxycarbonyl mit bis zu 6 Kohlenstoffatomen oder Phenyl oder durch einen 5- bis 7-gliedrigen, aromatischen Heterocyclus mit bis zu 3 Heteroatomen aus der Reihe

S, N oder O substituiert ist, oder
Aryl mit 6 bis 10 Kohlenstoffatomen oder einen 5- bis 7-gliedrigen aromatischen Heterocyclus mit bis zu 3 Heteroatomen aus der Reihe S, N oder O bedeutet,
und wobei die oben aufgeführten Cyclen gegebenenfalls bis zu 2fach gleich oder verschieden durch Carboxy, Halogen, Cyano, Formyl, Trifluormethyl, Nitro, geradkettiges oder verzweigtes Alkoxy, Alkoxycarbonyl, Alkylthio oder Acyl mit jeweils bis zu 6 Kohlenstoffatomen oder durch geradkettiges oder verzweigtes Alkyl mit bis zu 6 Kohlenstoffatomen substituiert sind

oder

$R^2$ für Morpholinyl oder für einen Rest der Formel

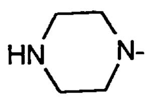 ,

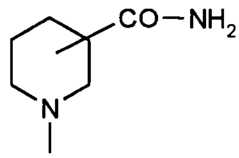 ,

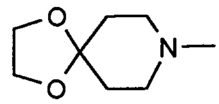

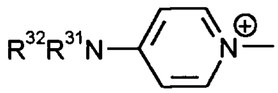 ,

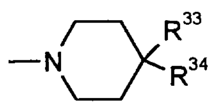 ,

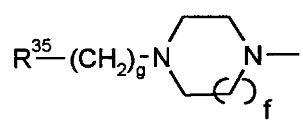

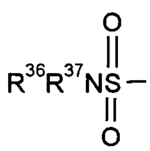 oder

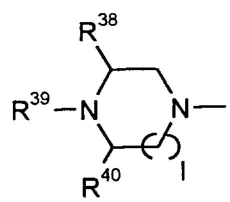

steht
worin

$R^{31}$ und $R^{32}$ die oben angegebene Bedeutung von $R^{24}$ und $R^{25}$ haben und mit dieser gleich oder verschieden sind,

$R^{33}$ und $R^{34}$ gemeinsam einen Rest der Formel =O bilden
oder

$R^{33}$ und $R^{34}$ gleich oder verschieden sind und Wasserstoff, Hydroxy oder geradkettiges oder verzweigtes Alkyl mit bis zu 6 Kohlenstoffatomen bedeuten, das gegebenenfalls durch eine Gruppe der Formel $-NR^{41}R^{42}$ substituiert ist,
worin

$R^{41}$ und $R^{42}$ die oben angegebene Bedeutung von $R^{24}$ und $R^{25}$ haben und mit dieser gleich oder verschieden sind

f eine Zahl 0 oder 1 bedeutet,

g eine Zahl 0, 1, 2, 3, 4, 5 oder 6 bedeutet,

$R^{35}$ Aryl mit 6 bis 10 Kohlenstoffatomen oder einen 5-bis 6-gliedrigen aromatischen, gegebenenfalls auch benzokondensierten Heterocyckus mit bis zu 3 Heteroatomen aus der Reihe S, N und oder O bedeuten, wobei alle Ringsysteme bis zu 3fach, gleich oder verschieden durch Nitro, Cyano, Hydroxy, Phenyl, Halogen, Trifluormethyl oder durch geradkettiges oder verzweigtes Alkyl, Alkoxy oder Acyl mit jeweils bis zu 5 Kohlenstoffatomen substituiert sein können,
oder

$R^{35}$ Morpholinyl, Hydroxy, geradkettiges oder verzweigtes Alkoxy mit bis zu 6 Kohlenstoffatomen oder einen Rest der Formel

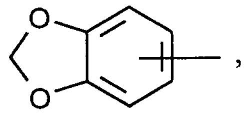

$-NR^{43}R^{44}$ oder $-CO-R^{45}$ bedeutet,
worin

$R^{43}$ und $R^{44}$ gleich oder verschieden sind und die oben angegebene Bedeutung von $R^{24}$ und $R^{25}$ haben,

$R^{45}$ Morpholinyl, Hydroxy oder geradkettiges oder verzweigtes Alkoxy mit bis zu 6 Kohlenstoffatomen bedeutet,

$R^{36}$ und $R^{37}$ gleich oder verschieden sind und Wasserstoff, Phenyl oder geradkettiges oder verzweigtes Alkyl mit bis zu 6 Kohlenstoffatomen oder Benzyl bedeutet,

$R^{38}$, $R^{39}$ und $R^{40}$ gleich oder verschieden sind und die oben angegebene Bedeutung von $R^{30}$ haben und mit dieser gleich oder verschieden sind,

l eine Zahl 1 oder 2 bedeutet,

und deren Salze.

2. Verbindungen nach Anspruch 1, in denen

A für ein Sauerstoffatom oder für einen Rest der Formel $-S(O)_a$ steht,
worin

a eine Zahl 0 oder 2 bedeutet,

$R^1$ für Azido oder für eine Gruppe der Formel $-OSO_2R^3$ oder $-NR^4R^5$ steht,
worin

$R^3$ geradkettiges oder verzweigtes Alkyl mit bis zu 3 Kohlenstoffatomen, Phenyl oder Toluolyl bedeutet,

$R^4$ und $R^5$ gleich oder verschieden sind und
Cyclopropyl, Cycylopentyl, Cyclohexyl, Wasserstoff, Phenyl oder geradkettiges oder verzweigtes Alkyl oder Alkoxy mit jeweils bis zu 6 Kohlenstoffatomen, tert. Butoxycarbonyl oder Benzyloxycarbonyl bedeuten,
oder

$R^4$ oder $R^5$ eine Gruppe der Formel $-CO-R^6$ bedeutet,
worin

$R^6$ Cyclopropyl, Cyclobutyl, Cyclopentyl, Cyclohexyl oder geradkettiges oder verzweigtes Alkyl oder Alkoxy mit jeweils bis zu 6 Kohlenstoffatomen, Phenyl oder Wasserstoff bedeutet,

G, L und M gleich oder verschieden sind und
für Wasserstoff, Carboxy, Fluor, Chlor, Brom, Jod, Cyano, Trifluormethyl, Formyl, Nitro, geradkettiges oder verzweigtes Alkoxy, Alkoxycarbonyl, Alkylthio oder Acyl mit jeweils bis zu 3 Kohlenstoffatomen oder für geradkettiges oder verzweigtes Alkyl mit bis zu 4 Kohlenstoffatomen stehen, das seinerseits durch Hydroxy, durch geradkettiges oder verzweigtes Alkoxy oder Acyl mit bis zu 4 Kohlenstoffatomen oder durch eine Gruppe der Formel $-NR^7R^8$ substituiert sein kann,
worin

$R^7$ und $R^8$ gleich oder verschieden sind und
Wasserstoff geradkettiges oder verzweigtes Alkyl mit bis zu 3 Kohlenstoffatomen oder Phenyl bedeuten,
oder gemeinsam mit dem Stickstoffatom einen Morpholinyl-, Pyrrolidinyl-, Piperazinyl-oder Piperidylring bilden, die gegebenenfalls, auch über die freie N-Funktion, durch Methyl, Ethyl oder Acetyl substituiert

sind,
und/oder
gegebenenfalls für eine Gruppe der Formel - $NR^{7'}R^{8'}$ stehen,
worin

| | |
|---|---|
| $R^{7'}$ und $R^{8'}$ | die oben angegebene Bedeutung von $R^7$ und $R^8$ haben und mit dieser gleich oder verschieden sind, und/oder gegebenenfalls für $(C_2-C_4)$-Alkenylphenyl, Phenyl, Pyridyl oder Thienyl stehen, die ihrerseits gegebenenfalls durch eine Gruppe der Formel -CO-$NR^9R^{10}$, -$NR^{11}R^{12}$, - $NR^{13}$-$SO_2$-$R^{14}$, $R^{15}R^{16}N$-$SO_2$- oder -$R^{17}$-$S(O)_b$- substituiert sind, worin |
| b | eine Zahl 0, 1 oder 2 bedeutet, |
| $R^9$, $R^{10}$, $R^{13}$, $R^{15}$ und $R^{16}$ | gleich oder verschieden sind und Wasserstoff, geradkettiges oder verzweigtes Alkyl mit bis zu 4 Kohlenstoffatomen oder Phenyl bedeuten, |
| $R^{11}$ und $R^{12}$ | gleich oder verschieden sind und die oben angegebene Bedeutung von $R^7$ und $R^8$ haben und mit dieser gleich oder verschieden sind, |
| $R^{14}$ und $R^{17}$ | gleich oder verschieden sind und die oben angegebene Bedeutung von $R^3$ haben und mit dieser gleich oder verschieden sind, und/oder ihrerseits gegebenenfalls bis zu 2-fach gleich oder verschieden durch Carboxy, Fluor, Chlor, Brom, Jod, Cyano, Trifluormethyl, Formyl, Nitro, Phenyl geradkettiges oder verzweigtes Alkoxy, Alkoxycarbonyl, Alkylthio oder Acyl mit jeweils bis zu 4 Kohlenstoffatomen oder durch geradkettiges oder verzweigtes Alkyl mit bis zu 4 Kohlenstoffatomen substituiert sind, das seinerseits durch Hydroxy, durch geradkettiges oder verzweigtes Alkoxy oder Acyl mit bis zu 4 Kohlenstoffatomen oder durch eine Gruppe der Formel -$NR^{18}R^{19}$ gegebenenfalls substituiert sein kann, worin |
| $R^{18}$ und $R^{19}$ | die oben angegebene Bedeutung von $R^7$ und $R^8$ haben und mit dieser gleich oder verschieden sind, |
| $R^2$ | für Wasserstoff, Formyl, Carboxy, für geradkettiges oder verzweigtes Alkoxycarbonyl mit bis zu 5 Kohlenstoffatomen steht, oder für geradkettiges oder verzweigtes Alkyl oder Alkenyl mit jeweils bis zu 6 Kohlenstoffatomen steht, die gegebenenfalls durch Hydroxy, Fluor, Chlor, Brom oder durch geradkettiges oder verzweigtes Alkoxy, Acyl, Alkylthio oder Alkoxycarbonyl mit jeweils bis zu 6 Kohlenstoffatomen oder durch Phenyl substituiert sind, das seinerseits durch Fluor, Chlor oder Brom substituiert sein kann, für Phenyl steht, das gegebenenfalls durch Carboxy, Fluor, Chlor, Brom, Jod, Cyano, Trifluormethyl, Formyl, Nitro, geradkettiges oder verzweigtes Alkoxy, Alkoxycarbonyl, Alkylthio oder Acyl mit jeweils bis zu 4 Kohlenstoffatomen oder durch geradkettiges oder verzweigtes Alkyl mit bis zu 4 Kohlenstoffatomen substituiert ist, oder für einen Rest der Formel -$NR^{20}R^{21}$, -$OR^{22}$ oder $S(O)_c$-$R^{23}$ steht, worin |
| $R^{20}$ | Cyclopropyl, Cyclopentyl, Cyclohexyl, Phenyl, geradkettiges oder verzweigtes Acyl mit bis zu 4 Kohlenstoffatomen oder geradkettiges oder verzweigtes Alkyl mit bis zu 6 Kohlenstoffatomen bedeutet, das gegebenenfalls durch Hydroxy, geradkettiges oder verzweigtes Alkoxy oder Hydroxy substituiertes Alkoxy mit jeweils bis zu 5 Kohlenstoffatomen, Pyridyl, Pyrazinyl oder Pyrimidyl, oder durch Phenyl substituiert ist, das seinerseits durch Hydroxy, Trifluormethyl, Fluor, Chlor, Brom, Nitro oder durch geradkettiges oder verzweigtes Alkoxy mit bis zu 3 Kohlenstoffatomen substituiert sein kann, oder |

Alkyl gegebenenfalls durch einen Rest der Formel - $NR^{24}R^{25}$ oder

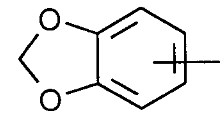

substituiert ist,
worin

$R^{24}$ und $R^{25}$ gleich oder verschieden sind und Wasserstoff oder geradkettiges oder verzweigtes Alkyl mit bis zu 3 Kohlenstoffatomen bedeuten, oder

$R^{20}$ einen Rest der Formel

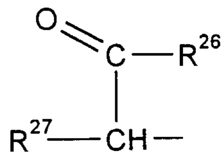

bedeutet
worin

$R^{26}$ Hydroxy, geradkettiges oder verzweigtes Alkoxy mit bis zu 3 Kohlenstoffatomen, oder einen Rest der Formel $-NR^{28}R^{29}$ bedeutet, worin $R^{28}$ und $R^{29}$ gleich oder verschieden sind und Wasserstoff oder geradkettiges oder verzweigtes Alkyl mit bis zu 3 Kohlenstoffatomen, Cyclopropyl, Cyclopentyl, Cyclohexyl oder Phenyl bedeuten,

$R^{27}$ Wasserstoff oder geradkettiges oder verzweigtes Alkyl mit bis zu 5 Kohlenstoffatomen bedeutet, das gegebenenfalls durch Phenyl substituiert oder

$R^{20}$ einen Rest der Formel

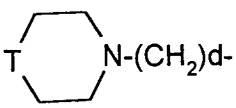

bedeutet
worin

d eine Zahl 0, 1, 2 oder 3 bedeutet

T ein Sauerstoffatom oder eine Gruppe der Formel $-CH_2$ oder $-NR^{30}$ bedeutet,
worin

$R^{30}$ Wasserstoff Phenyl oder geradkettiges oder verzweigtes Alkyl mit bis zu 4 Kohlenstoffatomen bedeutet, das gegebenenfalls durch Hydroxy substituiert ist,
und

$R^{21}$ die oben angegebene Bedeutung von $R^{20}$ hat und mit dieser gleich oder verschieden ist, oder
Wasserstoff bedeutet,

$R^{22}$ geradkettiges oder verzweigtes Alkyl mit bis zu 6 Kohlenstoffatomen bedeutet, das gegebenenfalls durch geradkettiges oder verzweigtes Alkoxy oder Hydroxy oder Alkoxy substituiertes Alkoxy mit jeweils bis zu 5 Kohlenstoffatomen, Cyclopropyl, Cyclopentyl, Cyclohexyl, Pyridyl, Pyrimidyl, Pyrazinyl oder Chinolyl substituiert ist,
die ihrerseits durch Nitro, Trifluormethyl, Fluor, Chlor, Brom, Cyano, Hydroxy oder durch geradkettiges oder verzweigtes Alkyl, Alkoxy oder

Acyl mit jeweils bis zu 4 Kohlenstoffatomen substituiert sein können, oder

$R^{22}$ einen Rest der Formel

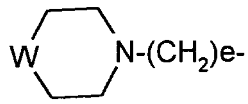

bedeutet,

worin

e die oben angegebene Bedeutung von d hat und mit dieser gleich oder verschieden ist,

W die oben angegebene Bedeutung von T hat und mit dieser gleich oder verschieden ist,

oder

$R^{22}$ Phenyl oder Pyridyl bedeutet,

c eine Zahl 0, 1 oder 2 bedeutet,

$R^{23}$ geradkettiges oder verzweigtes Alkyl oder Alkenyl mit jeweils bis zu 14 Kohlenstoffatomen bedeutet, das gegebenenfalls durch geradkettiges oder verzweigtes Alkoxycarbonyl mit bis zu 4 Kohlenstoffatomen oder durch Phenyl, Thienyl, Furyl, Pyrrolyl, Imidazolyl, Pyridyl, Pyrimidyl, Pyrazinyl oder Pyridazinyl substituiert ist, oder

Phenyl, Thienyl, Furyl, Pyrrolyl, Imidazolyl, Pyridin, Pyrazinyl, Pyrimidyl oder Pyridazinyl bedeutet,

und wobei die oben aufgeführten Cyclen gegebenenfalls bis zu 2-fach gleich oder verschieden durch Carboxy, Fluor, Chlor, Brom, Jod, Cyano, Trifluormethyl, Formyl, Nitro, geradkettiges oder verzweigtes Alkoxy, Alkoxycarbonyl, Alkylthio oder Acyl mit jeweils bis zu 4 Kohlenstoffatomen oder durch geradkettiges oder verzweigtes Alkyl mit bis zu 4 Kohlenstoffatomen substituiert sind

oder

$R^2$ für Morpholinyl oder für einen Rest der Formel

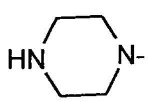 , 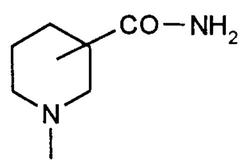 , 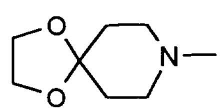

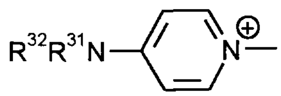 , 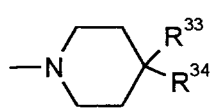 , 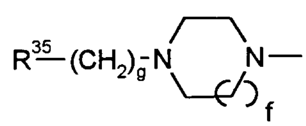

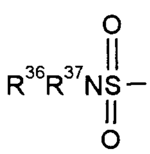 oder 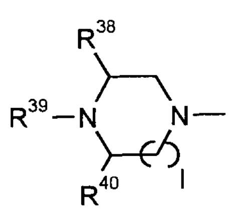

steht

worin

| | |
|---|---|
| $R^{31}$ und $R^{32}$ | die oben angegebene Bedeutung von $R^{24}$ und $R^{25}$ haben und mit dieser gleich oder verschieden sind, |
| $R^{33}$ und $R^{34}$ | gemeinsam einen Rest der Formel =O bilden<br>oder |
| $R^{33}$ und $R^{34}$ | gleich oder verschieden sind und Wasserstoff, Hydroxy oder geradkettiges oder verzweigtes Alkyl mit bis zu 4 Kohlenstoffatomen bedeuten, das gegebenenfalls durch eine Gruppe der Formel - $NR^{41}R^{42}$ substituiert ist,<br>worin |
| $R^{41}$ und $R^{42}$ | die oben angegebene Bedeutung von $R^{24}$ und $R^{25}$ haben und mit dieser gleich oder verschieden sind |
| f | eine Zahl 0 oder 1 bedeutet, |
| g | eine Zahl 0, 1, 2, 3 oder 4 bedeutet, |
| $R^{35}$ | Phenyl, Pyridyl, Pyrimidyl, Pyrazinyl oder Chinolyl bedeutet, die ihrerseits bis zu 2fach, gleich oder verschieden durch Nitro, Cyano, Hydroxy, Phenyl, Fluor, Chlor, Brom, Trifluormethyl oder durch geradkettiges oder verzweigtes Alkyl, Alkoxy oder Acyl mit jeweils bis zu 4 Kohlenstoffatomen substituiert sein können, oder |
| $R^{35}$ | Morpholinyl, Hydroxy, geradkettiges oder verzweigtes Alkoxy mit bis zu 4 Kohlenstoffatomen oder einen Rest der Formel<br>$-NR^{43}R^{44}$ oder $-CO-R^{45}$ bedeutet,<br>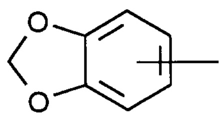 ,<br>worin |
| $R^{43}$ und $R^{44}$ | gleich oder verschieden sind und die oben angegebene Bedeutung von $R^{24}$ und $R^{25}$ haben, |
| $R^{45}$ | Morpholinyl, Hydroxy oder geradkettiges oder verzweigtes Alkoxy mit bis zu 5 Kohlenstoffatomen bedeutet, |
| $R^{36}$ und $R^{37}$ | gleich oder verschieden sind und Wasserstoff, Phenyl oder geradkettiges oder verzweigtes Alkyl mit bis zu 4 Kohlenstoffatomen oder Benzyl bedeutet, |
| $R^{38}$, $R^{39}$ und $R^{40}$ | gleich oder verschieden sind und die oben angegebene Bedeutung von $R^{30}$ haben und mit dieser gleich oder verschieden sind, |
| l | eine Zahl 1 oder 2 bedeutet, |

und deren Salze.

**3.** Verbindungen nach Anspruch 1, in denen

| | |
|---|---|
| A | für ein Sauerstoffatom oder für einen Rest der Formel - $S(O)_a$ steht,<br>worin |
| a | eine Zahl 0 oder 2 bedeutet, |
| $R^1$ | für Azido oder für eine Gruppe der Formel - $OSO_2R^3$ oder $-NR^4R^5$ steht,<br>worin |
| $R^3$ | Methyl, Ethyl, Phenyl oder Toluolyl bedeutet, |
| $R^4$ und $R^5$ | gleich oder verschieden sind und<br>Cyclopropyl, Cyclopentyl, Cyclohexyl, Wasserstoff, Phenyl oder geradkettiges oder verzweigtes Alkyl mit bis zu 5 Kohlenstoffatomen bedeuten,<br>oder |
| $R^4$ oder $R^5$ | eine Gruppe der Formel $-CO-R^6$ bedeutet,<br>worin |

$R^6$ Cyclopropyl, Cyclopentyl, Cyclohexyl oder geradkettiges oder verzweigtes Alkyl oder Alkoxy mit jeweils bis zu 4 Kohlenstoffatomen, Wasserstoff oder Phenyl bedeutet,

G, L und M gleich oder verschieden sind und für Wasserstoff, Carboxy, Fluor, Chlor, Brom, Jod, Cyano, Formyl, Nitro, oder für geradkettiges oder verzweigtes Alkyl mit bis zu 3 Kohlenstoffatomen stehen, und/oder für eine Gruppe der Formel $-NR^{7'}R^{8'}$ stehen, worin

$R^{7'}$ und $R^{8'}$ gleich oder verschieden sind und Wasserstoff oder Methyl bedeuten,

$R^2$ für Wasserstoff, Formyl, Carboxy, geradkettiges oder verzweigtes Alkoxycarbonyl mit bis zu 3 Kohlenstoffatomen oder für geradkettiges oder verzweigtes Alkyl oder Alkenyl mit jeweils bis zu 4 Kohlenstoffatomen steht, die gegebenenfalls durch Hydroxy, Fluor, Chlor, Brom oder durch geradkettiges oder verzweigtes Alkoxy, Acyl, Alkylthio oder Alkoxycarbonyl mit jeweils bis zu 3 Kohlenstoffatomen oder durch Phenyl substituiert sind, das seinerseits durch Chlor substituiert sein kann, für Phenyl steht, das gegebenenfalls durch Carboxy, Fluor, Chlor, Brom, Jod, Cyano, Formyl, Trifluormethyl, Nitro, geradkettiges oder verzweigtes Alkoxy, Alkoxycarbonyl oder Acyl mit jeweils bis zu 3 Kohlenstoffatomen oder durch geradkettiges oder verzweigtes Alkyl mit bis zu 3 Kohlenstoffatomen substituiert ist, oder für einen Rest der Formel $-NR^{20}R^{21}$, $-OR^{22}$ oder $-S(O)_c-R^{23}$ steht, worin

$R^{20}$ Cyclopropyl, Cyclopentyl, Cyclohexyl, Phenyl, geradkettiges oder verzweigtes Acyl mit bis zu 3 Kohlenstoffatomen, oder geradkettiges oder verzweigtes Alkyl mit bis zu 4 Kohlenstoffatomen bedeutet, das gegebenenfalls durch Hydroxy, geradkettiges oder verzweigtes Alkoxy oder Hydroxy substituiertes Alkoxy mit jeweils bis zu 3 Kohlenstoffatomen, Pyridyl, Pyrimidyl oder Pyrazinyl, oder durch Phenyl substituiert ist, das seinerseits durch Hydroxy, Trifluormethyl, Fluor, Chlor, Brom, Nitro, Methoxy oder Ethoxy substituiert sein kann, oder Alkyl gegebenenfalls durch einen Rest der Formel $-NR^{24}R^{25}$ oder

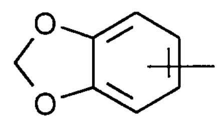

substituiert ist, worin

$R^{24}$ und $R^{25}$ gleich oder verschieden sind und Wasserstoff, Methyl oder Ethyl bedeuten, oder

$R^{20}$ einen Rest der Formel

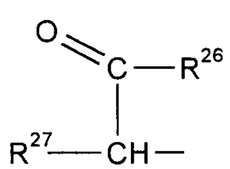

bedeutet worin

$R^{26}$ Hydroxy, Methoxy, Ethoxy oder einen Rest der Formel $-NR^{28}R^{29}$ bedeutet, worin $R^{28}$ und $R^{29}$ gleich oder verschieden sind und Wasserstoff Methyl, Ethyl, Cyclopropyl oder Phenyl bedeuten,

$R^{27}$ Wasserstoff oder geradkettiges oder verzweigtes Alkyl mit bis zu 3 Kohlenstoffatomen bedeutet, das gegebenenfalls durch Phenyl substituiert ist, oder

$R^{20}$ einen Rest der Formel

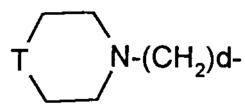

bedeutet

worin

d eine Zahl 0, 1 oder 2 bedeutet

T ein Sauerstoffatom oder eine Gruppe der Formel $-CH_2$ oder $-NR^{30}$ bedeutet,

worin

$R^{30}$ Wasserstoff oder geradkettiges oder verzweigtes Alkyl mit bis zu 3 Kohlenstoffatomen bedeutet, das gegebenenfalls durch Hydroxy substituiert ist,

und

$R^{21}$ die oben angegebene Bedeutung von $R^{20}$ hat und mit dieser gleich oder verschieden ist, oder

Wasserstoff bedeutet,

$R^{22}$ geradkettiges oder verzweigtes Alkyl mit bis zu 4 Kohlenstoffatomen bedeutet, das gegebenenfalls durch geradkettiges oder verzweigtes Alkoxy oder Hydroxy oder Alkoxy substituiertes Alkoxy mit jeweils bis zu 4 Kohlenstoffatomen, Cyclopropyl, Cyclopentyl, Cyclohexyl, Pyridyl, Pyrazinyl oder Pyrimidyl substituiert ist,

die ihrerseits durch Nitro, Trifluormethyl, Fluor, Chlor, Brom, Cyano, Hydroxy oder durch geradkettiges oder verzweigtes Alkyl, Alkoxy oder Acyl mit jeweils bis zu 3 Kohlenstoffatomen substituiert sein können,

oder

$R^{22}$ einen Rest der Formel

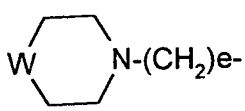

bedeutet,

worin

e die oben angegebene Bedeutung von d hat und mit dieser gleich oder verschieden ist,

W die oben angegebene Bedeutung von T hat und mit dieser gleich oder verschieden ist,

oder

$R^{22}$ Phenyl oder Pyridyl bedeutet,

c eine Zahl 0, 1 oder 2 bedeutet,

$R^{23}$ geradkettiges oder verzweigtes Alkyl oder Alkenyl mit bis zu 13 Kohlenstoffatomen bedeutet, das gegebenenfalls durch geradkettiges oder verzweigtes Alkoxycarbonyl mit bis zu 3 Kohlenstoffatomen oder Phenyl, durch Thienyl, Pyridyl, Pyrazinyl oder Pyrimidyl substituiert ist, oder

Phenyl, Thienyl, Pyridyl, Pyrazinyl oder Pyrimidyl bedeutet, und wobei die oben aufgeführten Cyclen gegebenenfalls durch Carboxy, Fluor, Chlor, Brom, Jod, Cyano, Formyl, Trifluormethyl, Nitro, geradkettiges oder verzweigtes Alkoxy, Alkoxycarbonyl oder Acyl mit jeweils bis zu 4 Kohlenstoffatomen oder durch geradkettiges oder verzweigtes Alkyl mit bis zu 4 Kohlenstoffatomen substituiert sind

oder

$R^2$ für Morpholinyl oder für einen Rest der Formel

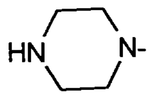 , 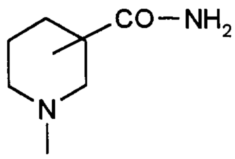 , 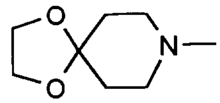

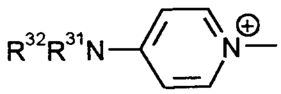 , 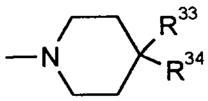 , 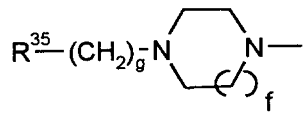

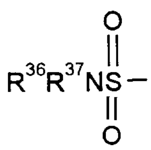 oder 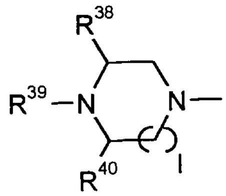

steht

worin

$R^{31}$ und $R^{32}$ die oben angegebene Bedeutung von $R^{24}$ und $R^{25}$ haben und mit dieser gleich oder verschieden sind,

$R^{33}$ und $R^{34}$ gemeinsam einen Rest der Formel =O bilden

oder

$R^{33}$ und $R^{34}$ gleich oder verschieden sind und Wasserstoff, Hydroxy oder geradkettiges oder verzweigtes Alkyl mit bis zu 3 Kohlenstoffatomen bedeuten, das gegebenenfalls durch eine Gruppe der Formel $-NR^{41}R^{42}$ substituiert ist,

worin

$R^{41}$ und $R^{42}$ die oben angegebene Bedeutung von $R^{24}$ und $R^{25}$ haben und mit dieser gleich oder verschieden sind

f eine Zahl 0 oder 1 bedeutet,

g eine Zahl 0, 1, 2 oder 3 bedeutet,

$R^{35}$ Phenyl, Pyridyl, Pyrazinyl oder Pyrimidyl bedeutet, die ihrerseits durch Nitro, Cyano, Hydroxy, Phenyl, Fluor, Chlor, Brom, Trifluormethyl oder durch geradkettiges oder verzweigtes Alkyl, Alkoxy oder Acyl mit jeweils bis zu 3 Kohlenstoffatomen substituiert sein können, oder

$R^{35}$ Morpholinyl, Hydroxy, geradkettiges oder verzweigtes Alkoxy mit bis zu 3 Kohlenstoffatomen oder einen Rest der Formel

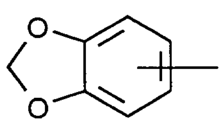 ,

$-NR^{43}R^{44}$ oder $-CO-R^{45}$ bedeutet,

worin

$R^{43}$ und $R^{44}$ gleich oder verschieden sind und die oben angegebene Bedeutung von $R^{24}$ und $R^{25}$ haben,

$R^{45}$ Morpholinyl, Hydroxy oder geradkettiges oder verzweigtes Alkoxy mit bis zu 4 Kohlenstoffatomen bedeutet,

$R^{36}$ und $R^{37}$ gleich oder verschieden sind und Wasserstoff, Phenyl oder geradkettiges oder verzweigtes Alkyl mit bis zu 3 Kohlenstoffatomen oder Benzyl bedeutet,

| | |
|---|---|
| $R^{38}$, $R^{39}$ und $R^{40}$ | gleich oder verschieden sind und die oben angegebene Bedeutung von $R^{28}$ haben und mit dieser gleich oder verschieden sind, |
| l | eine Zahl 1 oder 2 bedeutet, |

und deren Salze.

**4.** Verbindungen nach Anspruch 1, in denen G, L und M für Wasserstoff stehen und der Oxazolidinonrest in den Positionen 5 oder 6 an den Phenylring angebunden ist.

**5.** Verfahren zur Herstellung von Verbindungen nach Anspruch 1 bis 4, dadurch gekennzeichnet, daß man [A] zunächst Verbindungen der allgemeinen Formel (II)

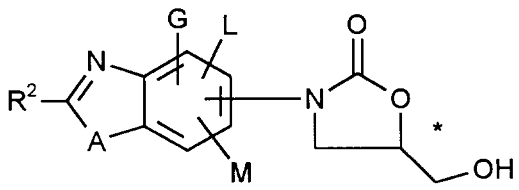

(II)

in welcher
A, G, L, M und $R^2$ die oben angegebene Bedeutung haben,
durch Umsetzung mit $(C_1\text{-}C_4)$-Alkyl- oder Phenylsulfonsäurechloriden in inerten Lösemitteln und in Anwesenheit einer Base in die entsprechenden Verbindungen der allgemeinen Formel (Ia)

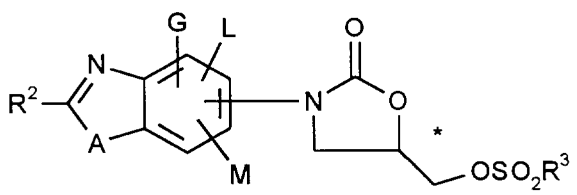

(Ia)

in welcher
A, G, L, M, $R^2$ und $R^3$ die oben angegebene Bedeutung haben,
überführt,
anschließend mit Natriumazid in inerten Lösemitteln die Azide der allgemeinen Formel (Ib)

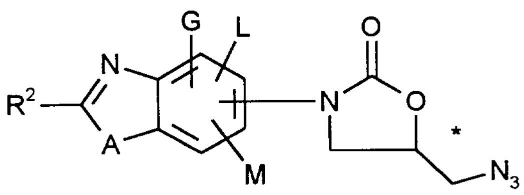

(Ib)

in welcher
A, G, L, M und $R^2$ die oben angegebene Bedeutung haben,
herstellt,
in einem weiteren Schritt durch Umsetzung mit $(C_1\text{-}C_4\text{-}O)_3\text{-}P$ oder $Ph_3P$, vorzugsweise $(CH_3O)_3P$ in inerten Löse-

mitteln und mit Säuren in die Amine der allgemeinen Formel (Ic)

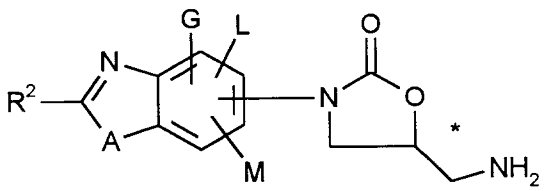

(Ic)

in welcher
A, G, L, M und $R^2$ die oben angegebene Bedeutung haben,
überführt,
und durch Umsetzung mit Acetanhydrid, Acetylchlorid oder anderen Acylierungsmitteln der allgemeinen Formel (III)

$$Y\text{-}CO\text{-}R^6 \qquad (III)$$

in welcher
$R^6$ die oben angegebene Bedeutung hat
und

Y für Halogen, vorzugsweise für Chlor oder für den Rest $-OCOR^6$ steht,

in inerten Lösemitteln die Verbindungen der allgemeinen Formel (Id)

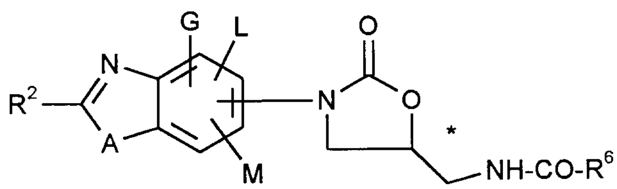

(Id)

in welcher
A, G, L, M, $R^2$ und $R^6$ die oben angegebene Bedeutung haben
herstellt,
und im Fall der S-Oxide, ausgehend von den entsprechenden S-Alkyl-Verbindungen mit m-Chlorperbenzoesäure oxidiert [B],
und im Fall von Substitutionsreaktionen, ausgehend von den Sulfonylverbindungen unter Substitution Hucleophile einführt [C],
und gegebenenfalls eine Alkylhalogenierung nach üblichen Methoden durchführt [D],
und gegebenenfalls ausgehend von den Verbindungen mit $R^2$ = 2-Phenylvinyl zunächst zu den entsprechenden Formylderivaten oxidiert und in einem zweiten Schritt nach bekannten Methoden reduziert [E],
und im Fall $R^4$, $R^5$, $R^7$, $R^{7'}$, $R^8$, $R^{8'}$, $R^9$, $R^{10}$, $R^{11}$, $R^{12}$, $R^{13}$, $R^{15}$, $R^{16}$, $R^{18}$, $R^{19}$, $R^{21}$, $R^{24}$, $R^{25}$, $R^{31}$, $R^{32}$, $R^{41}$, $R^{42}$, $R^{43}$ und/oder $R^{44} \neq H$ gegebenenfalls eine Alkylierung nach üblichen Methoden durchführt,
und gegebenenfalls weitere Substituenten oder bereits vorhandene funktionelle Gruppen nach üblichen Methoden, wie beispielsweise Redoxreaktionen, Substitutionsreaktionen und/oder Verseifungen oder Ein- und Abbau von Schutzgruppen, einführt bzw. derivatisiert.

**6.** Verwendung der Verbindungen nach den Ansprüchen 1 bis 4 zur Herstellung von Arzneimitteln.

**7.** Arzneimittel, enthaltend Verbindungen gemäß den Ansprüchen 1 bis 4.

Europäisches Patentamt

# EUROPÄISCHER RECHERCHENBERICHT

Nummer der Anmeldung
EP 95 11 1477

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
| A,D<br><br>P,A | WO-A-93 08179 (EISAI CO LTD)<br>* Zusammenfassung *<br>& EP-A-0 609 441 (EISAI CO LTD)<br>* Beispiele 2, 30, 44, 48, 49; Ansprüche 1-3, 8-17 *<br>--- | 1,4,6,7<br><br>1,4,6,7 | C07D417/04<br>C07D263/56<br>C07D417/14<br>C07D263/58<br>C07D491/10<br>A61K31/42<br>A61K31/425<br>A61K31/44<br>A61K31/445<br>A61K31/495 |
| A | WO-A-93 09103 (THE UPJOHN CO)<br>* Seite 8, Zeile 26 - Seite 9,Zeile 15; Beispiele 39, 40, 51, 52; Seite 39; Ansprüche 1, 2, 11, 12, 15, 24, 25 *<br>--- | 1,5-7 | |
| A | EP-A-0 359 418 (THE UPJOHN CO)<br>* Seite 14, Zeilen 22-46; Ansprüche *<br>--- | 1,4-7 | |
| A,D<br><br><br>D<br>D<br>D | EP-A-0 311 090 (E.I. DU PONT DE NEMOURS AND CO)<br>* Seite 10; Ansprüche 1, 11 *<br>& US-A-4 801 600<br>& US-A-4 921 869<br>& US-A-4 965 268<br>----- | 1,4-7 | RECHERCHIERTE SACHGEBIETE (Int.Cl.6)<br>C07D<br>A61K |

Der vorliegende Recherchenbericht wurde für alle Patentansprüche erstellt

| Recherchenort | Abschlußdatum der Recherche | Prüfer |
|---|---|---|
| BERLIN | 20.November 1995 | Van Amsterdam, L |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04C03)